(19) Europäisches Patentamt
European Patent Office
Office européen des brevets

(11) EP 4 201 408 B1

(12) EUROPEAN PATENT SPECIFICATION

(45) Date of publication and mention of the grant of the patent:
**25.06.2025 Bulletin 2025/26**

(21) Application number: **22211737.6**

(22) Date of filing: **22.08.2009**

(51) International Patent Classification (IPC):
***A61K 31/7004*** *(2006.01)* ***A61K 31/78*** *(2006.01)*
***A61P 7/08*** *(2006.01)*

(52) Cooperative Patent Classification (CPC):
**A61K 31/78; A61K 31/7004; A61P 1/00; A61P 1/04; A61P 3/00; A61P 3/02; A61P 3/12; A61P 7/00; A61P 7/08; A61P 9/04; A61P 9/10; A61P 13/12; B01J 39/20; C08F 220/22;**
C08F 212/36

# (54) CROSSLINKED CATION EXCHANGE POLYMERS, COMPOSITIONS AND USE IN TREATING HYPERKALEMIA

VERNETZTE KATIONENAUSTAUSCHPOLYMERE, ZUSAMMENSETZUNGEN UND VERWENDUNG BEI DER BEHANDLUNG VON HYPERKALÄMIE

POLYMÈRES RÉTICULÉS ÉCHANGEURS DE CATIONS, COMPOSITIONS ET UTILISATION DANS LE TRAITEMENT DE L’HYPERKALIÉMIE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.08.2008 US 91110 P**
**22.08.2008 US 9112508 P**
**22.08.2008 US 9109708 P**
**01.04.2009 US 165894 P**
**01.04.2009 US 16589909 P**
**02.04.2009 US 165905 P**

(43) Date of publication of application:
**28.06.2023 Bulletin 2023/26**

(62) Document number(s) of the earlier application(s) in accordance with Art. 76 EPC:
**18188764.7 / 3 431 094**
**15171701.4 / 2 957 286**
**09748557.7 / 2 365 988**

(73) Proprietor: **Vifor (International) Ltd.**
**9014 St. Gallen (CH)**

(72) Inventors:
- **CHANG, Han-Ting**
  **Livermore, CA 94550 (US)**
- **CHARMOT, Dominique**
  **Campbell, CA 95008 (US)**
- **LIU, Mingjun**
  **Sewickley, PA 15143 (US)**
- **STRUEVER, Werner**
  **51375 Leverkusen (DE)**
- **MANSKY, Paul**
  **San Francisco, CA 94110 (US)**
- **ALBRECHT, Detlef**
  **Saratoga, CA 95070 (US)**
- **BURDICK, Michael**
  **Los Altos, CA 94022 (US)**
- **CONNOR, Eric**
  **Los Gatos, CA 94560 (US)**
- **HALFON, Sherin**
  **Palo Alto, CA 94306 (US)**
- **HUANG, I-Zu**
  **Mountain View, CA 94040 (US)**
- **CHIDAMBARAM, Ramakrishnan**
  **Pleasanton, CA 94566 (US)**
- **MILLS, Jonathan**
  **San Jose, CA 95120 (US)**

(74) Representative: **Gille Hrabal Partnerschaftsgesellschaft mbB**
**Patentanwälte**
**Brucknerstraße 20**
**40593 Düsseldorf (DE)**

(56) References cited:
**WO-A2-2007/038802** **US-A1- 2005 220 752**
**US-A1- 2006 024 396**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

FIELD OF THE INVENTION

The present invention is directed to methods of removing potassium in the gastrointestinal tract, including methods of treating hyperkalemia, by administration of crosslinked cation exchange polymers having beneficial physical properties, including combinations of particle size, particle shape, particle size distribution, viscosity, yield stress, compressibility, surface morphology, and/or swelling ratio; processes for preparing crosslinked cation exchange polymers comprising a fluoro group and an acid group and being the product of the polymerization of at least three monomer units; and compositions of a stabilizing linear polyol and a salt of a crosslinked cation exchange polymer comprising a fluoro group and an acid group useful to bind potassium in the gastrointestinal tract.

BACKGROUND OF THE INVENTION

Potassium ($K^+$) is one of the most abundant intracellular cations. Potassium homeostasis is maintained predominantly through the regulation of renal excretion. Various medical conditions, such as decreased renal function, genitourinary disease, cancer, severe diabetes mellitus, congestive heart failure and/or the treatment of these conditions can lead to or predispose patients to hyperkalemia. Hyperkalemia can be treated with various cation exchange polymers including polyfluoroacrylic acid (polyFAA) as disclosed in WO 2005/097081.

Various polystyrene sulfonate cation exchange polymers (e.g., Kayexalate®, Argamate®, Kionex®) have been used to treat hyperkalemia in patients. These polymers and polymer compositions are known to have patient compliance issues, including dosing size and frequency, taste and/or texture, and gastric irritation. For example, in some patients, constipation develops, and sorbitol is thus commonly co-administered to avoid constipation, but this leads to diarrhea and other gastrointestinal side effects. It is also known that a wide variety of sugars can be used in pharmaceutical compositions. See, for example, EP 1785141.

Methods of reducing potassium and/or treatment of hyperkalemia have been found to raise patient compliance problems, in particular in chronic settings, which are solved by the present invention. Such problems include lack of tolerance of the therapeutically effective dose of polymeric binder (e.g., anorexia, nausea, gastric pain, vomiting and fecal impaction), dosing form (e.g., taste, mouth feel, etc.) and dose frequency (e.g., three times per day). The present invention solves these problems by providing a polymeric binder or a composition containing a polymeric binder that can be given once a day or twice a day without significant gastrointestinal side effects while retaining substantially similar efficacy. The methods of the present invention reduce the frequency and form of administration of potassium binder and increase tolerance, which will improve patient compliance, and potassium binding effectiveness.

Also, it has been found that linear polyols in particular have a stabilizing effect during storage on crosslinked poly alpha-fluoroacrylic acid in its salt form. It has also now been found that the production of cross-linked fluoroacrylic acid polymers is improved by the addition of a second cross linker having a slower reactivity rate that DVB. US-2005220752 discloses a crosslinked cation exchange polymer consisting of 90 weight % of 2-fluoroacrylic methyl ester and 9 weight % of divinylbenzene.US 2006/0024336 is entitled "Ion binding compositions" and exemplifies poly(alpha-fluoroacrylic acid) crosslinked with divinylbenzene. WO 2007/038802 is entitled "Methods for preparing core-shell composites having cross-linked shells and core-shell composites resulting therefrom", and relates to particles having a crosslinked poly(vinylamine) shell.

SUMMARY OF THE INVENTION

The present invention provides a pharmaceutical composition that comprises a salt of a crosslinked cation exchange polymer and a linear polyol stabilizer. Optionally, moisture is added to the composition. The salt of a preferred crosslinked cation exchange polymer is the product of the polymerization of at least two, and optionally three, different monomer units and is stabilized with respect to fluoride release. Among the various aspects of the invention is a composition comprising a linear polyol and a salt of a crosslinked cation exchange polymer comprising a fluoro group and an acid group that is the product of the polymerization of at least two, and optionally three, different monomer units. Typically, one monomer comprises a fluoro group and an acid group and the other monomer is a difunctional arylene monomer or a difunctional alkylene, ether- or amide-containing monomer, or a combination thereof.

A further aspect of the invention is a pharmaceutical composition comprising a crosslinked cation exchange polymer salt and from about 10 wt.% to about 40 wt.% of a linear polyol based on the total weight of the composition. The crosslinked cation exchange polymer comprises structural units corresponding to Formulae 1 and 2, Formulae 1 and 3, or Formulae 1, 2, and 3, wherein Formula 1, Formula 2, and Formula 3 are represented by the following structures:

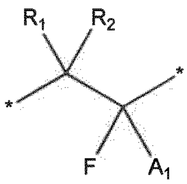
Formula 1
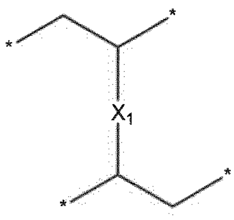
Formula 2
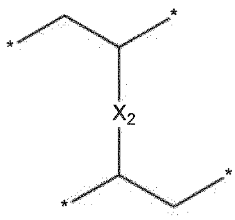
Formula 3

wherein $R_1$ and $R_2$ are each independently hydrogen, alkyl, cycloalkyl, or aryl; $A_1$ is carboxylic, phosphonic, or phosphoric; $X_1$ is arylene; and $X_2$ is alkylene, an ether moiety, or an amide moiety. In some instances, Formula 1, Formula 2, and Formula 3 are represented by the following structures:

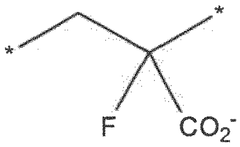
Formula 1A
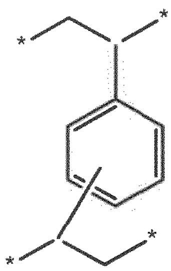
Formula 2A
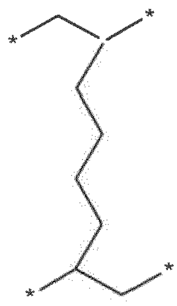
Formula 3A .

Another aspect of the invention is a pharmaceutical composition comprising a crosslinked cation exchange polymer salt and an effective amount of a linear polyol sufficient to stabilize the polymer salt, wherein the salt of the crosslinked cation exchange polymer comprises structural units corresponding to Formulae 1 and 2, Formulae 1 and 3, or Formulae 1, 2, and 3. In some instances, the structural units of Formula 1, Formula 2 and Formula 3 correspond to Formula 1A, Formula 2A, and Formula 3A, respectively. Optionally, the composition further comprises moisture.

A further aspect is a pharmaceutical composition comprising a crosslinked cation exchange polymer salt and from about 10 wt.% to about 40 wt.% of a linear polyol based on the total weight of the composition, the crosslinked cation exchange polymer being a reaction product of a polymerization mixture comprising monomers of either (i) Formulae 11 and 22, (ii) Formulae 11 and 33, or (iii) Formulae 11, 22, and 33. Formula 11, Formula 22, and Formula 33 are represented by the following structures:

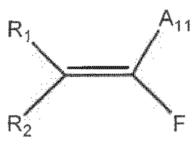
Formula 11
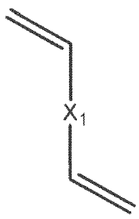
Formula 22
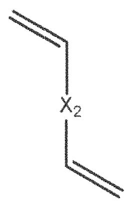
Formula 33

wherein $R_1$ and $R_2$ are each independently hydrogen, alkyl, cycloalkyl, or aryl; $A_{11}$ is an optionally protected carboxylic, phosphonic, or phosphoric; $X_1$ is arylene; and $X_2$ is alkylene, an ether moiety, or an amide moiety. In some instances, Formula 11, Formula 22, and Formula 33 are represented by the following structures:

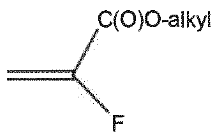
Formula 11A

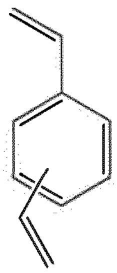
Formula 22A

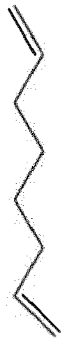
Formula 33A .

Another aspect of the invention is a pharmaceutical composition comprising a crosslinked cation exchange polymer salt and an effective amount of a linear polyol sufficient to stabilize the polymer salt, wherein the salt of the crosslinked cation exchange polymer is a reaction product of a polymerization mixture comprising monomers corresponding to Formulae 11 and 22, Formulae 11 and 33, or Formulae 11, 22, and 33. In some instances, Formula 1, Formula 2 and Formula 3 correspond to Formula 11A, Formula 22A, and Formula 33A, respectively. Optionally the composition further comprises moisture.

Yet another aspect is a method for removing potassium from the gastrointestinal tract of an animal subject in need thereof. The method comprises administering any one of the crosslinked cation exchange polymers or pharmaceutical compositions described herein to the subject, whereby the polymer or pharmaceutical composition passes through the gastrointestinal tract of the subject, and removes a therapeutically effective amount of potassium ion from the gastrointestinal tract of the subject. In some embodiments, the subject is a mammal, and preferably, a human.

A further aspect is a method for removing potassium from the gastrointestinal tract of an animal subject in need thereof, comprising administering an effective amount once per day or twice per day to the subject of a crosslinked cation exchange polymer or any pharmaceutical composition described herein, wherein the polymer comprises structural units corresponding to Formulae 1 and 2, Formulae 1 and 3, or Formulae 1, 2, and 3, wherein Formula 1, Formula 2, and Formula 3 are represented by the following structures:

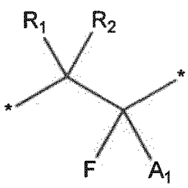
Formula 1

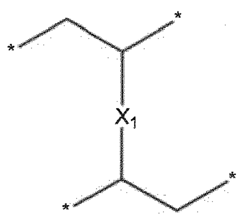
Formula 2

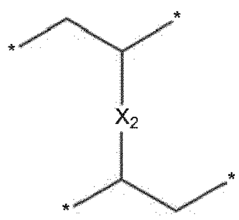
Formula 3

wherein $R_1$ and $R_2$ are each independently hydrogen, alkyl, cycloalkyl, or aryl; $A_1$ is carboxylic, phosphonic, or phosphoric; $X_1$ is arylene; and $X_2$ is alkylene, an ether moiety, or an amide moiety, wherein a daily amount of the polymer or composition has a potassium binding capacity of at least 75% of the binding capacity of the same polymer or composition administered at the same daily amount three times per day.

The present invention also provides a method of removing potassium in an animal subject in need thereof, comprising administering an effective amount once per day or twice per day to the subject of a crosslinked cation exchange polymer or any pharmaceutical composition described herein, wherein the polymer is the reaction product of a polymerization mixture comprising monomers of either (i) Formulae 11 and 22, (ii) Formulae 11 and 33, or (iii) Formulae 11, 22, and 33. Formula 11, Formula 22, and Formula 33 are represented by the following structures:

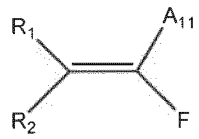

Formula 11

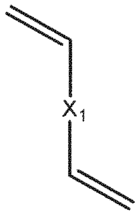

Formula 22

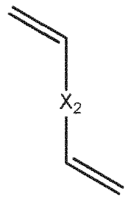

Formula 33

wherein $R_1$ and $R_2$ are each independently hydrogen, alkyl, cycloalkyl, or aryl; $A_{11}$ is an optionally protected carboxylic, phosphonic, or phosphoric; $X_1$ is arylene; and $X_2$ is alkylene, an ether moiety, or an amide moiety, wherein a daily amount of the polymer or the composition has a potassium binding capacity of at least 75% of the binding capacity of the same polymer or composition administered at the same daily amount three times per day.

In other embodiments, the present invention provides a method of removing potassium from the gastrointestinal tract of an animal subject in need thereof, comprising administering an effective amount once per day or twice per day to the subject of a daily amount of a crosslinked cation exchange polymer or a pharmaceutical composition as described herein, wherein either (1) less than 25% of subjects taking the polymer or composition once per day or twice per day experience mild or moderate gastrointestinal adverse events or (2) a daily amount of the polymer or composition has a potassium binding capacity of at least 75% of the same daily amount of the same polymer administered three times per day or (3) both.

It has also been found that use of a composition comprising a crosslinked aliphatic carboxylic polymer and an effective amount of, or in some instances from about 10 wt.% to about 40 wt.% of, a linear polyol has increased efficacy for removal of potassium as compared to a composition not containing the linear polyol. In this regard, increased efficacy is measured by the amount of fecal excretion of potassium. The compositions and/or methods of this invention include a composition comprising an effective amount, or in some instances from about 10 wt.% to about 40 wt.%, of a linear polyol, and a crosslinked aliphatic carboxylic polymer that extracts from an animal subject in need thereof about 5% more potassium as compared to the same dose and same administration frequency of the same polymer without stabilization by a linear polyol.

Among the various aspects of the invention are crosslinked cation exchange polymers having desirable particle size, particle shape, particle size distribution, yield stress, viscosity, compressibility, surface morphology, and/or swelling ratio, and methods of removing potassium by administering the polymer or a pharmaceutical composition including the polymer to an animal subject in need thereof.

Another aspect of the invention is a method for removing potassium and/or treating hyperkalemia from an animal subject in need thereof comprising administering a potassium binding polymer to the animal subject. The potassium binding polymer is a crosslinked cation exchange polymer comprising acid groups in their acid or salt form and in the form of substantially spherical particles having a mean diameter of from about 20 $\mu$m to about 200 $\mu$m and less than about 4 volume percent of the particles have a diameter of less than about 10 $\mu$m. The polymer particles also have a sediment yield stress of less than about 4000 Pa, and a swelling ratio of less than about 10 grams of water per gram of polymer.

A further aspect of the invention is a method for removing potassium and/or treating hyperkalemia in an animal subject in need thereof comprising administering a potassium binding polymer to the animal subject. The potassium binding polymer is a crosslinked cation exchange polymer comprising acid groups in their acid or salt form, is in the form of substantially spherical particles having a mean diameter of less than about 250 $\mu$m and less than about 4 volume percent of the particles having a diameter of less than about 10 $\mu$m. The polymer particles also have a swelling ratio of less than about 10 grams of water per gram of polymer, and a hydrated and sedimented mass of polymer particles having a viscosity of less than 1,000,000 pascal seconds (Pa·s) wherein the viscosity is measured at a shear rate of 0.01 $sec^{-1}$.

Thus, the present invention provides a method of removing potassium and/or treating hyperkalemia in an animal subject in need thereof, comprising administering an effective amount once per day or twice per day to the subject of a crosslinked cation exchange polymer in the form of substantially spherical particles having a mean diameter of less than about 250 $\mu$m and less than about 4 volume percent of the particles having a diameter of less than about 10 $\mu$m, wherein a daily amount of the polymer administered once per day or twice per day has a potassium binding capacity of at least 75% of the binding capacity of the same polymer administered at the same daily amount three times per day.

In other embodiments, the present invention provides a method of removing potassium and/or treating hyperkalemia in an animal subject in need thereof, comprising administering an effective amount once per day or twice of a daily amount of a crosslinked cation exchange polymer in the form of substantially spherical particles having a mean diameter of less than about 250 $\mu$m and less than about 4 volume percent of the particles having a diameter of less than about 10 $\mu$m, wherein less than 25% of subjects taking the polymer once per day or twice per day experience mild or moderate gastrointestinal adverse events. It is also a feature of this invention that the cation exchange polymers administered once a day or twice a day have about substantially the same tolerability as the same polymer of the same

daily amount administered three times a day.

The present invention provides a crosslinked polymer, which is the product of the polymerization of at least three different monomer units, and processes for preparing these polymers. Among the various aspects of the invention are crosslinked cation exchange polymers comprising a fluoro group and an acid group and being the product of the polymerization of at least three different monomer units and processes for the preparation thereof. Typically, one monomer comprises a fluoro group and an acid group, one monomer is a difunctional arylene monomer and another monomer is a difunctional alkylene, ether- or amide-containing monomer.

Another aspect of the invention is a crosslinked polymer comprising a reaction product of a polymerization mixture comprising three or more monomers. The monomers correspond to Formula 11, Formula 22, and Formula 33; wherein (i) the monomers corresponding to Formula 11 constitute at least about 85 wt.% or from about 80 wt.% to 95 wt.% based on the total weight of monomers of Formulae 11, 22, and 33 in the polymerization mixture, and the weight ratio of the monomer corresponding to Formula 22 to the monomer corresponding to Formula 33 is from about 4:1 to about 1:4, or (ii) the mole fraction of the monomer of Formula 11 in the polymerization mixture is at least about 0.87 or from about 0.87 to about 0.94 based on the total number of moles of the monomers of Formulae 11, 22, and 33, and the mole ratio of the monomer of Formula 22 to the monomer of Formula 33 in the polymerization mixture is from about 0.2:1 to about 7:1. Formula 11, Formula 22, and Formula 33 correspond to the following structures:

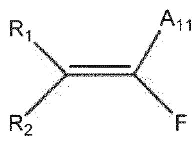
Formula 11

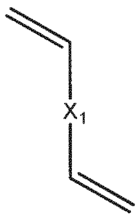
Formula 22

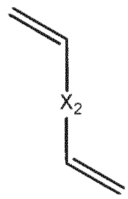
Formula 33

wherein $R_1$ and $R_2$ are each independently hydrogen, alkyl, cycloalkyl, or aryl; $A_{11}$ is an optionally protected carboxylic, phosphonic, or phosphoric; $X_1$ is arylene; and $X_2$ is alkylene, an ether moiety or an amide moiety.

Yet another aspect is a cation exchange polymer comprising structural units corresponding to Formulae 1, 2, and 3, wherein (i) the structural units corresponding to Formula 1 constitute at least about 85 wt.% or from about 80 wt.% to about 95 wt.% based on the total weight of structural units of Formulae 1, 2, and 3 in the polymer calculated from the amounts of monomers used in the polymerization reaction, and the weight ratio of the structural unit corresponding to Formula 2 to the structural unit corresponding to Formula 3 is from about 4:1 to about 1:4, or (ii) the mole fraction of the structural unit of Formula 1 in the polymer is at least about 0.87 or from about 0.87 to about 0.94 based on the total number of moles of the structural units of Formulae 1, 2, and 3, and the mole ratio of the structural unit of Formula 2 to the structural unit of Formula 3 is from about 0.2:1 to about 7:1 (calculated from the amounts of monomers used in the polymerization reaction). Formula 1, Formula 2, and Formula 3 correspond to the following structures:

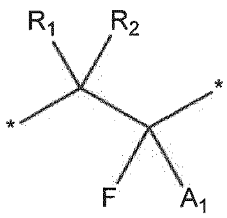
Formula 1

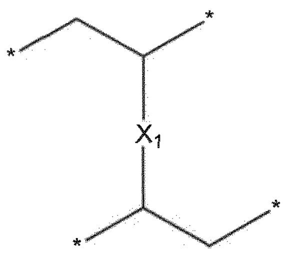
Formula 2

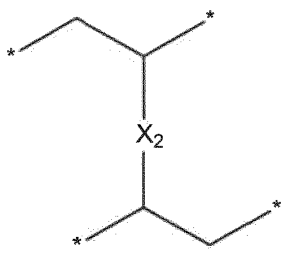
Formula 3

wherein $R_1$ and $R_2$ are independently hydrogen, alkyl, cycloalkyl, or aryl; $A_1$ is carboxylic, phosphonic, or phosphoric in its salt or acid form; $X_1$ is arylene; and $X_2$ is alkylene, an ether moiety or an amide moiety.

A further aspect of the invention is a crosslinked polymer comprising a reaction product of a polymerization mixture comprising three or more monomers. The monomers correspond to Formula 11A, Formula 22A, and Formula 33A; wherein (i) the monomers corresponding to Formula 11A constitute at least about 85 wt.% or from about 80 wt.% to about 95 wt.% based on the total weight of monomers of Formulae 11A, 22A, and 33A in the polymerization mixture and the weight ratio of monomers corresponding to Formula 22A to monomers corresponding to Formula 33A is from about 4:1 to about 1:4, or (ii) the mole fraction of the monomer of Formula 11A in the polymerization mixture is at least about 0.87 or from

about 0.87 to about 0.94 based on the total number of moles of the monomers of Formulae 11A, 22A, and 33A and the mole ratio of the monomer of Formula 22A to the monomer of Formula 33A in the polymerization mixture is from about 0.2:1 to about 7:1. Formula 11A, Formula 22A, and Formula 33A correspond to the following structures:

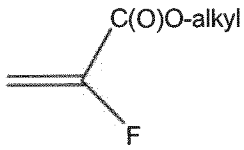

Formula 11A

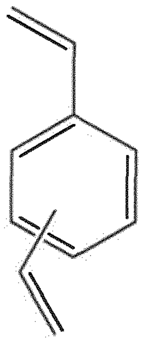

Formula 22A

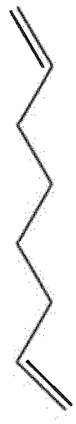

Formula 33A.

Another aspect is a cation exchange polymer comprising structural units corresponding to Formulae 1A, 2A, and 3A, wherein (i) the structural units corresponding to Formula 1A constitute at least about 85 wt.% or from about 80 wt.% to about 95 wt.% based on the total weight of structural units of Formulae 1A, 2A, and 3A in the polymer, and the weight ratio of the structural unit corresponding to Formula 2A to the structural unit corresponding to Formula 3A is from about 4:1 to about 1:4 (calculated from the amounts of monomers used in the polymerization reaction), or (ii) the mole fraction of the structural unit of Formula 1A in the polymer is at least about 0.87 or from about 0.87 to about 0.94 based on the total number of moles of the structural units of Formulae 1A, 2A, and 3A, and the mole ratio of the structural unit of Formula 2A to the structural unit of Formula 3A is from about 0.2:1 to about 7:1 (calculated from the amounts of monomers used in the polymerization reaction). Formula 1A, Formula 2A and Formula 3A correspond to the following structures:

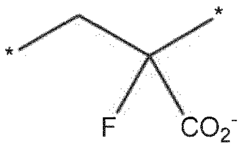

Formula 1A

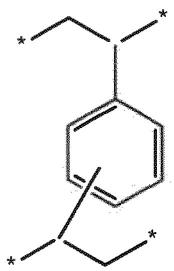

Formula 2A

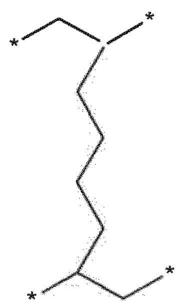

Formula 3A.

A further aspect is a pharmaceutical composition comprising any of the crosslinked cation exchange polymers described herein and a pharmaceutically acceptable excipient.

Yet another aspect of the invention is a method for removing potassium from the gastrointestinal tract of an animal subject, the method comprising administering a pharmaceutical composition described above to the subject, whereby the pharmaceutical composition passes through the gastrointestinal tract of the subject and removes a therapeutically effective amount of potassium ion from the gastrointestinal tract of the subject. In some instances, the animal subject is a mammal or a human.

Another aspect is a method of making a crosslinked cation exchange polymer comprising contacting a mixture comprising three or more monomers with a polymerization initiator to form a crosslinked polymer. The monomers correspond to Formula 11, Formula 22, and Formula 33; wherein (i) the monomers corresponding to Formula 11 constitute at least about 85 wt.% or from about 80 wt.% to about 95 wt.% based on the total weight of monomers of Formulae 11, 22, and 33 in the polymerization mixture, and the weight ratio of the monomer corresponding to Formula 22 to the monomer corresponding to Formula 33 is from about 4:1 to about 1:4, or (ii) the mole fraction of the monomer of Formula 11 in the polymerization mixture is at least about 0.87 or from about 0.87 to about 0.94 based on the total number of moles of the monomers of Formulae 11, 22, and 33, and the mole ratio of the monomer of Formula 22 to the monomer of Formula 33 in

the polymerization mixture is from about 0.2:1 to about 7:1. Formula 11, Formula 22, and Formula 33 correspond to the following structures:

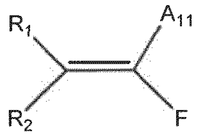

Formula 11

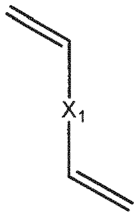

Formula 22

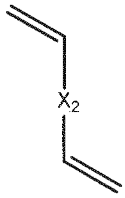

Formula 33

wherein $R_1$ and $R_2$ are each independently hydrogen, alkyl, cycloalkyl, or aryl; $A_{11}$ is protected carboxylic, phosphonic, or phosphoric; $X_1$ is arylene; and $X_2$ is alkylene, an ether moiety or an amide moiety.

A further aspect is a method of making a crosslinked cation exchange polymer comprising contacting a mixture comprising three or more monomers with a polymerization initiator to form a crosslinked polymer. The monomers correspond to Formula 11A, Formula 22A, and Formula 33A; wherein (i) the monomers corresponding to Formula 11A constitute at least about 85 wt.% or from about 80 wt.% to about 95 wt.% based on the total weight of monomers of Formulae 11A, 22A, and 33A in the polymerization mixture, and the weight ratio of the monomer corresponding to Formula 22 to the monomer corresponding to Formula 33A is from about 4:1 to about 1:4, or (ii) the mole fraction of the monomer of Formula 11A in the polymerization mixture is at least about 0.87 or from about 0.87 to about 0.94 based on the total number of moles of the monomers of Formulae 11A, 22A, and 33A, and the mole ratio of the monomer of Formula 22A to the monomer of Formula 33A in the polymerization mixture is from about 0.2:1 to about 7:1. Formulae 11A, 22A, and 33A correspond to the following structures:

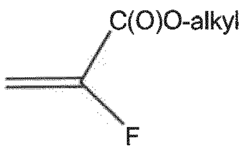

Formula 11A

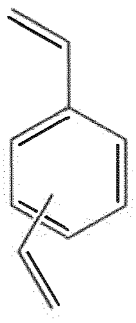

Formula 22A

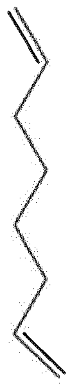

Formula 33A .

The methods of making the crosslinked cation exchange polymers described above can further comprise hydrolyzing the crosslinked polymer with a hydrolysis agent.

BRIEF DESCRIPTION OF THE DRAWINGS

Figure 1A shows a scanning electron microscope (SEM) micrograph of the surface of a bead prepared as described in Example 8A. Figure 1B shows cross-sectional SEM micrographs of Example 8A beads that were cracked by cryo-crushing.

Figures 2A and 2B show Atomic Force Microscope (AFM) images of the surfaces of two Ca(polyfluoroacrylate) samples prepared by the process of Example 8A and the measurements are described in Example 9.

Figures 3-A1 to 3-A6 show a series of SEM micrographs of crosslinked poly(FAA) beads prepared with increasing amounts of dichloroethane solvent as described in Example 11.

Figures 4-B1 to 4-B8 show a series of SEM micrographs of crosslinked poly(FAA) beads that were prepared with increasing amounts of sodium chloride as described in Example 12.

Figures 5A and 5B show SEM micrographs of crosslinked poly(FAA) beads prepared by polymerizing t-butyl fluoroacrylate monomer as described in Example 8D.

DETAILED DESCRIPTION

## Linear Polyol Stabilized Compositions

The present invention is directed to pharmaceutical compositions comprising a polyol and a salt of a crosslinked cation exchange polymer, with the polyol present in an amount sufficient to reduce the release of fluoride ion from the cation exchange polymer during storage. In some embodiments, the pharmaceutical compositions of this invention additionally comprise water also present in an amount sufficient to reduce or assist in the reduction of the release of fluoride ion from the cation exchange polymer during storage. Generally, the salt of a crosslinked cation exchange polymer comprised a fluoro group and an acid group is the product of the polymerization of at least two, and optionally three, different monomer units. Typically, one monomer comprises a fluoro group and an acid group and the other monomer is a difunctional arylene monomer or a difunctional alkylene, ether- or amide-containing monomer, or a combination thereof. These pharmaceutical compositions are useful to bind potassium in the gastrointestinal tract. In preferred embodiments, the linear polyol is a linear sugar alcohol. Increased efficacy, and/or tolerability in different dosing regimens, is seen as compared to compositions without the linear polyol, and optionally including water.

A linear polyol is added to the composition containing the salt of a crosslinked cation exchange polymer in an amount effective to stabilize the polymer salt, and generally from about 10 wt.% to about 40 wt.% linear polyol based on the total weight of the composition. The linear polyol is preferably a linear sugar (i.e, a linear sugar alcohol). The linear sugar alcohol is preferably selected from the group consisting of D-(+)arabitol, erythritol, glycerol, maltitol, D-mannitol, ribitol, D-sorbitol, xylitol, threitol, galactitol, isomalt, iditol, lactitol and combinations thereof, more preferably selected from the group consisting of D-(+)arabitol, erythritol, glycerol, maltitol, D-mannitol, ribitol, D-sorbitol, xylitol, and combinations thereof , and most preferably selected from the group consisting of xylitol, sorbitol, and a combination thereof. Preferably, the pharmaceutical composition contains from about 15 wt.% to about 35 wt.% stabilizing polyol based on the total weight of the composition. In various embodiments, this linear polyol concentration is sufficient to reduce the release of fluoride ion from the cation exchange polymer upon storage as compared to an otherwise identical composition containing no stabilizing polyol at the same temperature and storage time.

The moisture content of the composition can be balanced with the stabilizing linear polyol to provide a stabilized polymer within the composition. In general, as the moisture content of the composition increases, the concentration of polyol can be decreased. However, the moisture content should not rise so high as to prevent the composition from being free flowing during manufacturing or packaging operations. In general, the moisture content can range from about 1 to about 30 weight percent based on the total weight of the composition. More specifically, the moisture content can be from about 10 to about 25 wt.% based on the total weight of the composition of polymer, linear polyol and water. In one specific case, the pharmaceutical composition comprises about 10-40 wt.% linear polyol, about 1-30 wt.% water and the remainder crosslinked cation exchange polymer, with the weight percents based on the total weight of linear polyol, water and polymer. Also, in a specific case, the pharmaceutical composition comprises about 15 wt.% to about 35 wt. % linear polyol, about 10 wt.% to about 25 wt% water and the remainder crosslinked cation exchange polymer, with the weight percents based on the total weight of linear polyol, water and polymer. In another specific case, the pharmaceutical composition comprises from about 10 wt.% to about 40 wt. % linear polyol and the remainder crosslinked cation exchange polymer, with the weight percents based on the total weight of linear polyol and polymer.

The moisture content can be measured in a manner known to those of skill in the art. Moisture content in the composition may be determined by two methods: (a) thermogravimetric method via a moisture analyzer during in-process manufacturing or (b) measuring loss on drying in accordance with US Pharmacopeia (USP) <731>. The operating condition for the thermogravimetric method via moisture analyzer is 0.3 g of polymer composition heated at about 160°C for about 45 min. The operating condition for the USP <731> method is 1.5-2 g of polymer composition heated to about 130°C for about 16 hours under 25-35 mbar vacuum.

From a stabilizing viewpoint, the concentration of inorganic fluoride (e.g., from fluoride ion) in the pharmaceutical composition is less than about 1000 ppm, less than about 500 ppm or less than about 300 ppm under typical storage conditions. More particularly, the concentration of inorganic fluoride in the pharmaceutical composition is less than about 1000 ppm after storage at accelerated storage conditions (about 40°C for about 6 weeks), less than about 500 ppm after room temperature storage (about 25°C for about 6 weeks), or less than about 300 ppm after refrigerated storage (about 5°C for about 6 weeks). Additionally, the concentration of inorganic fluoride in the pharmaceutical composition is generally 50% less and preferably 75% less than the concentration of inorganic fluoride in the otherwise identical composition containing no stabilizing polyol at the same temperature and storage time.

## Crosslinked Cation Exchange Polymers of Improved Physical Properties

The present invention is directed to methods for removing potassium from or treating hyperkalemia in an animal subject in need thereof by administration of crosslinked cation exchange polymers having combinations of particular

particle sizes and particle size distributions, particle shape, yield stress, viscosity, compressibility, surface morphology, and/or swelling ratios. The polymers include cations that can exchange with potassium *in vivo* to remove potassium from the gastrointestinal tract of a subject in need thereof, and are therefore potassium-binding polymers. The terms crosslinked cation exchange polymer and potassium-binding polymer are used interchangeably herein. As those of skill in the art will understand, certain properties of the polymers result from the physical properties of the polymer form, and thus the term particle is generally used to refer to such properties.

The crosslinked cation exchange polymers used in the invention are in the form of substantially spherical particles. As used herein, the term "substantially" means generally rounded particles having an average aspect ratio of about 1.0 to about 2.0. Aspect ratio is the ratio of the largest linear dimension of a particle to the smallest linear dimension of the particle. Aspect ratios may be easily determined by those of ordinary skill in the art. This definition includes spherical particles, which by definition have an aspect ratio of 1.0. In some embodiments, the particles have an average aspect ratio of about 1.0, 1.2, 1.4, 1.6, 1.8 or 2.0. The particles may be round or elliptical when observed at a magnification wherein the field of view is at least twice the diameter of the particle. See, for example, Figure 1A.

The crosslinked cation exchange polymer particles have a mean diameter of from about 20 μm to about 200 μm. Specific ranges are where the crosslinked cation exchange particles have a mean diameter of from about 20 μm to about 200 μm, from about 20 μm to about 150 μm, or from about 20 μm to about 125 μm. Other ranges include from about 35 μm to about 150 μm, from about 35 μm to about 125 μm, or from about 50 μm to about 125 μm. Particle sizes, including mean diameters, distributions, etc. can be determined using techniques known to those of skill in the art. For example, U.S. Pharmacopeia (USP) <429> discloses methods for determining particle sizes.

Various crosslinked cation exchange polymer particles also have less than about 4 volume percent of the particles that have a diameter of less than about 10 μm; particularly, less than about 2 volume percent of the particles that have a diameter of less than about 10 μm; more particularly, less than about 1 volume percent of the particles that have a diameter of less than about 10 μm; and even more particularly, less than about 0.5 volume percent of the particles that have a diameter of less than about 10 μm. In other cases, specific ranges are less than about 4 volume percent of the particles that have a diameter of less than about 20 μm; less than about 2 volume percent of the particles that have a diameter of less than about 20 μm; less than about 1 volume percent of the particles that have a diameter of less than about 20 μm; less than about 0.5 volume percent of the particles that have a diameter of less than about 20 μm; less than about 2 volume percent of the particles that have a diameter of less than about 30 μm; less than about 1 volume percent of the particles that have a diameter of less than about 30 μm; less than about 1 volume percent of the particles that have a diameter of less than about 30 μm; less than about 1 volume percent of the particles that have a diameter of less than about 40 μm; or less than about 0.5 volume percent of the particles that have a diameter of less than about 40 μm. In various embodiments, the crosslinked cation exchange polymer has a particle size distribution wherein not more than about 5 volume% of the particles have a diameter less than about 30 μm (i.e., D(0.05) < 30 μm), not more than about 5 volume% of the particles have a diameter greater than about 250 μm (i.e., D(0.05) > 250 μm), and at least about 50 volume% of the particles have a diameter in the range from about 70 to about 150 μm.

The particle distribution of the crosslinked cation exchange polymer can be described as the span. The span of the particle distribution is defined as (D(0.9)-D(0.1))/D(0.5), where D(0.9) is the value wherein 90% of the particles have a diameter below that value, D(0.1) is the value wherein 10% of the particles have a diameter below that value, and D(0.5) is the value wherein 50% of the particles have a diameter above that value and 50% of the particles have a diameter below that value as measured by laser diffraction. The span of the particle distribution is typically from about 0.5 to about 1, from about 0.5 to about 0.95, from about 0.5 to about 0.90, or from about 0.5 to about 0.85. Particle size distributions can be measured using Niro Method No. A 8 d (revised September 2005), available from GEA Niro, Denmark, using the Malvern Mastersizer.

Another desirable property that the crosslinked cation exchange polymers may possess is a viscosity when hydrated and sedimented of from about 10,000 Pa•s to about 1,000,000 Pa·s, from about 10,000 Pa·s to about 800,000 Pa·s, from about 10,000 Pa·s to about 600,000 Pa·s, from about 10,000 Pa·s to about 500,000 Pa·s, from about 10,000 Pa·s to about 250,000 Pa·s, or from about 10,000 Pa·s to about 150,000 Pa·s, from about 30,000 Pa·s to about 1,000,000 Pa·s, from about 30,000 Pa·s to about 500,000 Pa·s, or from about 30,000 Pa·s to about 150,000 Pa·s, the viscosity being measured at a shear rate of 0.01 $sec^{-1}$. This viscosity is measured using a wet polymer prepared by mixing the polymer thoroughly with a slight excess of simulated intestinal fluid (per USP <26>), allowing the mixture to sediment for 3 days at 37°C, and decanting free liquid from the sedimented wet polymer. The steady state shear viscosity of this wet polymer can be determined using a Bohlin VOR Rheometer (available from Malvern Instruments Ltd., Malvern, U.K.) or equivalent with a parallel plate geometry (upper plate of 15 mm diameter and lower plate of 30 mm diameter, and gap between plates of 1 mm) and the temperature maintained at 37°C.

The crosslinked cation exchange polymers may further have a hydrated and sedimented yield stress of from about 150 Pa to about 4000 Pa, from about 150 Pa to about 3000 Pa, from about 150 Pa to about 2500 Pa, from about 150 Pa to about 1500 Pa, from about 150 Pa to about 1000 Pa, from about 150 Pa to about 750 Pa, or from about 150 Pa to about 500 Pa, from about 200 Pa to about 4000 Pa, from about 200 Pa to about 2500 Pa, from about 200 Pa to about 1000

Pa, or from about 200 Pa to about 750 Pa. Dynamic stress sweep measurements (i.e., yield stress) can be made using a Reologica STRESSTECH Rheometer (available from Reologica Instruments AB, Lund, Sweden) or equivalent in a manner known to those of skill in the art. This rheometer also has a parallel plate geometry (upper plate of 15 mm diameter, lower plate of 30 mm diameter, and gap between plates of 1 mm) and the temperature is maintained at 37°C. A constant frequency of 1 Hz with two integration periods can be used while the shear stress is increased from 1 to $10^4$ Pa.

Crosslinked cation exchange polymers used in this invention also have desirable compressibility and bulk density when in the form of a dry powder. Some of the particles of the crosslinked cation exchange polymers in the dry form have a bulk density of from about 0.8 g/$cm^3$ to about 1.5 g/$cm^3$, from about 0.82 g/$cm^3$ to about 1.5 g/$cm^3$, from about 0.84 g/$cm^3$ to about 1.5 g/$cm^3$, from about 0.86 g/$cm^3$ to about 1.5 g/$cm^3$, from about 0.8 g/$cm^3$ to about 1.2 g/$cm^3$, or from about 0.86 g/$cm^3$ to about 1.2 g/$cm^3$. The bulk density affects the volume of crosslinked cation exchange polymer needed for administration to a patient. For example, a higher bulk density means that a lower volume will provide the same number of grams of crosslinked cation exchange polymer. This lower volume can improve patient compliance by allowing the patient to perceive they are taking a smaller amount due to the smaller volume.

A powder composed of the particles of the crosslinked cation exchange polymer in dry form has a compressibility index of from about 3 to about 15, from about 3 to about 14, from about 3 to about 13, from about 3 to about 12, from about 3 to about 11, from about 5 to about 15, from about 5 to about 13, or from about 5 to about 11. The compressibility index is defined as 100*(TD-BD)/TD, wherein BD and TD are the bulk density and tap density, respectively. The procedure for measuring bulk density and tap density is described below in Example 10. Further, the powder form of the cation exchange polymers settles into its smallest volume more easily than polymers conventionally used to treat hyperkalemia. This makes the difference between the bulk density and the tap density (measured powder density after tapping a set number of times) from about 3% to about 14%, from about 3% to about 13%, from about 3% to about 12%, from about 3% to about 11%, from about 3% to about 10%, from about 5% to about 14%, from about 5% to about 12%, or from about 5% to about 10% of the bulk density.

Generally the potassium binding polymers in particle form are not absorbed from the gastrointestinal tract. The term "non-absorbed" and its grammatical equivalents is not intended to mean that the entire amount of administered polymer is not absorbed. It is expected that certain amounts of the polymer may be absorbed. Particularly, about 90% or more of the polymer is not absorbed, more particularly about 95% or more is not absorbed, even more particularly about 97% or more is not absorbed, and most particularly about 98% or more of the polymer is not absorbed.

The swelling ratio of the potassium binding polymers in physiological isotonic buffer, which is representative of the gastrointestinal tract, is typically from about 1 to about 7, particularly from about 1 to about 5, more particularly from about 1 to about 3, and more specifically, from about 1 to about 2.5. In some embodiments, crosslinked cation exchange polymers of the invention have a swelling ratio of less than 5, less than about 4, less than about 3, less than about 2.5, or less than about 2. A Polymers of the invention are crosslinked materials, meaning that they do not generally dissolve in solvents, and, at most, swell in solvents. As used herein, "swelling ratio" refers to the number of grams of solvent taken up by one gram of otherwise non-solvated crosslinked polymer when equilibrated in an aqueous environment. When more than one measurement of swelling is taken for a given polymer, the mean of the measurements is taken to be the swelling ratio. The polymer swelling can also be calculated by the percent weight gain of the otherwise non-solvated polymer upon taking up solvent. For example, a swelling ratio of 1 corresponds to polymer swelling of 100%.

Crosslinked cation exchange polymers having advantageous surface morphology are polymers in the form of substantially spherical particles with a substantially smooth surface. A substantially smooth surface is a surface wherein the average distance from the peak to the valley of a surface feature determined at random over several different surface features and over several different particles is less than about 2 μm, less than about 1 μm, or less than about 0.5 μm. Typically, the average distance between the peak and the valley of a surface feature is less than about 1 μm.

The surface morphology can be measured using several techniques including those for measuring roughness. Roughness is a measure of the texture of a surface. It is quantified by the vertical deviations of a real surface from its ideal form. If these deviations are large, the surface is rough; if they are small the surface is smooth. Roughness is typically considered to be the high frequency, short wavelength component of a measured surface. For example, roughness may be measured using contact or non-contact methods. Contact methods involve dragging a measurement stylus across the surface; these instruments include profilometers and atomic force microscopes (AFM). Non-contact methods include interferometry, confocal microscopy, electrical capacitance and electron microscopy. These methods are described in more detail in Chapter 4: Surface Roughness and Microtopography by L. Mattson in Surface Characterization, ed. by D. Brune, R. Hellborg, H.J. Whitlow, O.Hunderi, Wiley-VCH, 1997.

For three-dimensional measurements, the probe is commanded to scan over a two-dimensional area on the surface. The spacing between data points may not be the same in both directions. Another way to measure the surface roughness is to crack the sample particles and obtain a SEM micrograph similar to Figure 1B. In this way, a side view of the surface can be obtained and the relief of the surface can be measured.

Surface roughness can be controlled in a number of ways. For example, three approaches were determined for preparing poly(α-fluoroacrylate) particles having a smoother surface. The first approach was to include a solvent that was

an acceptable solvent for the monomers and the polymeric product. The second approach was to decrease the solvation of the organic phase in the aqueous phase by a salting out process. The third approach was to increase the hydrophobicity of the starting fluoroacrylate monomer. These approaches are described in more detail in Examples 11-13.

Dosing regimens for chronic treatment of hyperkalemia can increase compliance by patients, particularly for crosslinked cation exchange polymers that are taken in gram quantities. The present invention is also directed to methods of chronically removing potassium from a mammal in need thereof, and in particular chronically treating hyperkalemia with a potassium binder that is a crosslinked aliphatic carboxylic polymer, and preferably a salt of such polymer stabilized with a linear polyol, wherein the polymer is in the form of a substantially spherical particle.

It has now been found that in using the polymer particles, once-a-day potassium binding dosing is substantially equivalent to twice-a-day potassium binding dosing, which is also substantially equivalent to a three-times-a-day dosing. As shown in the examples, volunteers receiving a polyol stabilized, calcium salt of cross-linked poly-alpha-fluoroacrylic acid polymer particle once per day excreted 82.8% of the amount of fecal potassium as those volunteers who received substantially the same amount of the same binding polymer particle three-times per day. It is also shown that volunteers receiving a polyol stabilized, calcium salt of cross-linked poly-alpha-fluoroacrylic acid polymer particle twice per day excreted 91.5% of the amount of fecal potassium as those volunteers who received substantially the same amount of the same polymer particle three-times per day. Fecal excretion is an in vivo measure of efficacy that relates to the lowering of serum potassium in subjects in need thereof.

These results were not based on administration with meals nor were they based on any particular formulation. In particular, the potassium binding polymer particles as used in this invention are substantially unreactive with food and can be added to typical food products (e.g., water, pudding, apple sauce, baked goods, etc.), which adds to compliance enhancement (particularly for patients who are on a water restricted diet). Substantially unreactive in this context means that the polymer particles do not effectively change the taste, consistency or other properties of the food in which it is mixed or placed. Also, the polymer particles as used in this invention can be administered without regard to mealtime. In fact, since potassium being bound is not just from meals, but is potassium that is excreted into the gastrointestinal tract, administration can take place at any time. Dosing regimens also take into account the other embodiments discussed herein, including capacity, amount and particle form.

It has also been found that the polymer particles as used in this invention are well tolerated when administered once daily or twice daily as compared to three times daily. The invention is thus also directed to methods of removing potassium from an animal subject by administering the polymer particles or a pharmaceutical composition comprising the polymer particles and from about 10 wt.% to about 40 wt.% of a linear polyol once a day, wherein less than 25% of subjects taking the polymer particles or composition once per day experience mild or moderate gastrointestinal adverse events. Gastrointestinal adverse events may include flatulence, diarrhea, abdominal pain, constipation, stomatitis, nausea and/or vomiting. In some aspects, the polymer particles or composition are administered twice a day and less than 25% of subjects taking the polymer particles or composition twice per day experience mild or moderate gastrointestinal adverse events. In some instances, the subjects taking the polymer particles or composition once per day or twice per day experience no severe gastrointestinal adverse events. The polymers particles or compositions as used in the invention have about 50% or more tolerability as compared to the same polymer particles or composition of the same daily amount administered three times a day. For example, for every two patients in which administration of the polymer three times a day is well tolerated, there is at least one patient in which administration of the polymer once a day or twice a day is well tolerated. In some instances, the polymer particles or compositions have about 75% or more tolerability as compared to the same polymer particles or composition of the same daily amount administered three times a day. It is also a feature of this invention that the polymer particles or compositions of the invention administered once a day or twice a day have about 85% or more tolerability as the same polymer particles or composition of the same daily amount administered three times a day. It is also a feature of this invention that the polymer particles or compositions administered once a day or twice a day have about 95% or more tolerability as the same polymer particles or composition of the same daily amount administered three times a day. It is also a feature of this invention that the polymer particles or compositions administered once a day or twice a day have about substantially the same tolerability as the same polymer particles or composition of the same daily amount administered three times a day.

When administration is well tolerated, there should be little or no significant dose modification or dose discontinuation by the subject. In some embodiments, well tolerated means there is no apparent dose response relationship for gastrointestinal adverse events. In some of these embodiments, well tolerated means that the following gastrointestinal adverse effects are not reported from a statistically significant number of subjects, including those effects selected from the group consisting of flatulence, diarrhea, abdominal pain, constipation, stomatitis, nausea and vomiting. In particular, the examples also show that there were no severe gastrointestinal adverse events in subjects.

Having described certain properties of the potassium binding polymers, the structural and/or chemical features of the various polymers in particle form which provide these properties are now described. In some embodiments, the potassium-binding polymers are crosslinked cation exchange polymers derived from at least one crosslinker and at least one monomer containing acid groups in their protonated or ionized form, such as sulfonic, sulfuric, carboxylic, phosphonic,

phosphoric, or sulfamic groups, or combinations thereof. In general, the fraction of ionization of the acid groups of the polymers used in this invention is greater than about 75% at the physiological pH (e.g., about pH 6.5) in the colon and the potassium binding capacity in vivo is greater than about 0.6 mEq/gram, more particularly greater than about 0.8 mEq/gram and even more particularly greater than about 1.0 mEq/gram. Generally the ionization of the acid groups is greater than about 80%, more particularly it is greater than about 90%, and most particularly it is about 100% at the physiological pH of the colon (e.g., about pH 6.5). In certain embodiments, the acid containing polymers contain more than one type of acid group. In other instances, the acid containing polymers are administered in their substantially anhydrous or salt form and generate the ionized form when contacted with physiological fluids. Representative structural units of these potassium binding polymers are shown in Table 1 wherein the asterisk at the end of a bond indicates that bond is attached to another structural unit or to a crosslinking unit.

TABLE 1: Examples of cation exchange structural units - structures and theoretical binding capacities

| | Molar mass per charge | Theoretical capacity | Fraction of titrable H @pH 3 | Fraction of titrable H @ pH 6 | Expected Capacity @pH 3 | Expected Capacity @pH 6 |
|---|---|---|---|---|---|---|
| 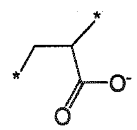 | 71 | 14.1 | 0.05 | .35 | 0.70 | 4.93 |
| 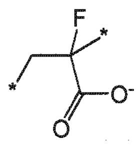 | 87 | 11.49 | 0.2 | 0.95 | 2.3 | 10.92 |
| 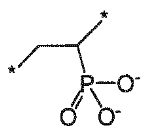 | 53 | 18.9 | 0.25 | 0.5 | 4.72 | 9.43 |
| 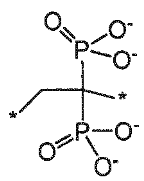 | 47.5 | 21.1 | 0.25 | 0.5 | 5.26 | 10.53 |
| 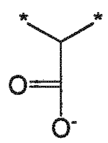 | 57 | 17.5 | 0.1 | 0.5 | 1.75 | 8.77 |
| 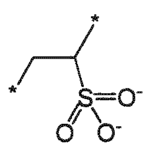 | 107 | 9.3 | 1 | 1 | 9.35 | 9.35 |
| 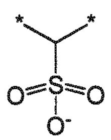 | 93 | 10.8 | 1 | 1 | 10.75 | 10.75 |
| 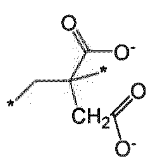 | 63 | 15.9 | 0 | 0.4 | 0 | 6.35 |

(continued)

| | Molar mass per charge | Theoretical capacity | Fraction of titrable H @pH 3 | Fraction of titrable H @ pH 6 | Expected Capacity @pH 3 | Expected Capacity @pH 6 |
|---|---|---|---|---|---|---|
| 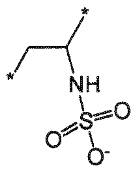 | 125 | 8 | 1 | 1 | 8 | 8 |
| 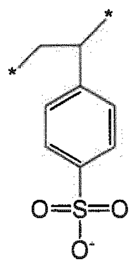 | 183 | 5.5 | 1 | 1 | 5.46 | 5.46 |
| 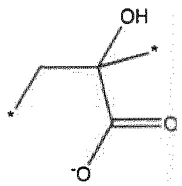 | 87 | 11.49 | .1 | .6 | 1.14 | 6.89 |

Other suitable cation exchange polymers contain repeat units having the following structures:

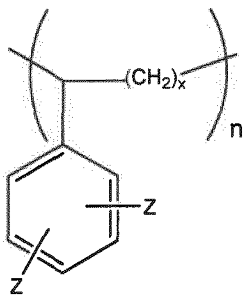

or

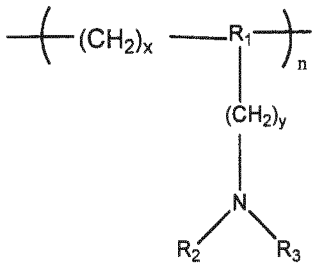

wherein $R_1$ is a bond or nitrogen, $R_2$ is hydrogen or Z, $R_3$ is Z or $-CH(Z)_2$, each Z is independently $SO_3H$ or $PO_3H$, x is 2 or 3, and y is 0 or 1, n is about 50 or more, more particularly n is about 100 or more, even more particularly n is about 200 or more, and most particularly n is about 500 or more.

Sulfamic (i.e. when $Z=SO_3H$) or phosphoramidic (i.e. when $Z= PO_3H$) polymers can be obtained from amine polymers or monomer precursors treated with a sulfonating agent such as sulfur trioxide/amine adducts or a phosphonating agent such as $P_2O_5$, respectively. Typically, the acidic protons of phosphonic groups are exchangeable with cations, like sodium or potassium, at pH of about 6 to about 7.

Suitable phosphonate monomers include vinyl phosphonate, vinyl-1,1-bis phosphonate, and ethylenic derivatives of phosphonocarboxylate esters, oligo(methylenephosphonates), and hydroxyethane-1,1-diphosphonic acid. Methods of synthesis of these monomers are well known in the art.

The cation exchange structural units and repeat units containing acid groups as described above are crosslinked to form the crosslinked cation exchange polymers of the invention. Representative crosslinking monomers include those shown in Table 2.

TABLE 2: Crosslinker Abbreviations and Structures

| Abbreviation | Chemical name | Structure | Molecular Weight |
|---|---|---|---|
| X-V-1 | ethylenebisacrylamide | 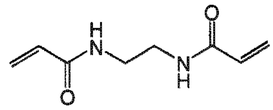 | 168.2 |
| X-V-2 | N,N'-(ethane-1,2-diyl)bis(3-(N-vinylformamido) propanamide) | 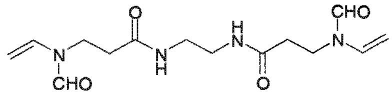 | 310.36 |
| X-V-3 | N,N'-(propane-1,3-diyl)diethenesulfonamide | 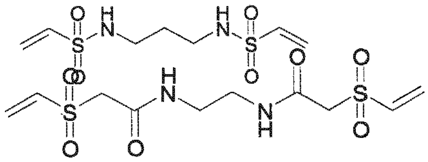 | 254.33 |
| X-V-4 | N,N'-bis(vinylsulfonylacetyl) ethylene diamine | | 324.38 |
| X-V-5 | 1,3-bis(vinylsulfonyl) 2-propanol | 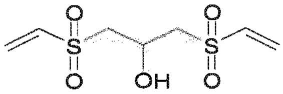 | 240.3 |
| X-V-6 | vinylsulfone | 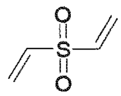 | 118.15 |
| X-V-7 | N,N'-methylenebisacrylamide | 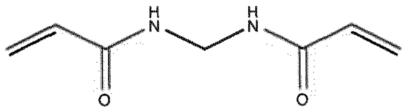 | 154.17 |
| ECH | epichlorohydrin | 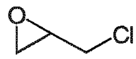 | 92.52 |
| DVB | Divinyl benzene | 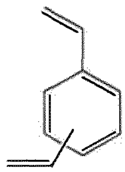 | 130.2 |
| ODE | 1,7-octadiene | 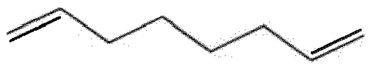 | 110.2 |
| HDE | 1,5-hexadiene | 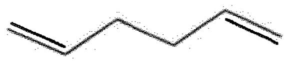 | 82.15 |

The ratio of repeat units to crosslinker can be chosen by those of skill in the art based on the desired physical properties of the polymer particles. For example, the swelling ratio can be used to determine the amount of crosslinking based on the general understanding of those of skill in the art that as crosslinking increases, the swelling ratio generally decreases. In one specific embodiment, the amount of crosslinker in the polymerization reaction mixture is in the range of 3 wt. % to 15 wt. %, more specifically in the range of 5 wt. % to 15 wt.% and even more specifically in the range of 8 wt. % to 12 wt.%, based on the total weight of the monomers and crosslinkers added to the polymerization reaction. Crosslinkers can include one or a mixture of those in Table 2.

In some embodiments, the crosslinked cation exchange polymer includes a pKa-decreasing group, preferably an electron-withdrawing substituent, located adjacent to the acid group, preferably in the alpha or beta position of the acid group. The preferred position for the electron-withdrawing group is attached to the carbon atom alpha to the acid group. Generally, electron-withdrawing substituents are a hydroxyl group, an ether group, an ester group, an acid group, or a halide atom. More preferably, the electron-withdrawing substituent is a halide atom. Most preferably, the electron-withdrawing group is fluoride and is attached to the carbon atom alpha to the acid group. Acid groups are carboxylic, phosphonic, phosphoric, or combinations thereof.

Other particularly preferred polymers result from the polymerization of alphafluoro acrylic acid, difluoromaleic

acid, or an anhydride thereof. Monomers for use herein include α-fluoroacrylate and difluoromaleic acid, with α-fluoroacrylate being most preferred. This monomer can be prepared from a variety of routes, see for example, Gassen et al, J. Fluorine Chemistry, 55, (1991) 149-162, KF Pittman, C. U., M. Ueda, et al. (1980). Macromolecules 13(5): 1031-1036. Difluoromaleic acid is prepared by oxidation of fluoroaromatic compounds (Bogachev et al, Zhumal Organisheskoi Khimii, 1986, 22(12), 2578-83), or fluorinated furan derivatives (See U.S. patent 5,112,993). A mode of synthesis of α-fluoroacrylate is given in EP 415214.

Generally, the salt of a crosslinked cation exchange polymer comprised a fluoro group and an acid group is the product of the polymerization of at least two, and optionally three, different monomer units. In some instances, one monomer comprises a fluoro group and an acid group and the other monomer is a difunctional arylene monomer or a difunctional alkylene, ether- or amide-containing monomer, or a combination thereof.

In a particular embodiment, the crosslinked cation exchange polymer comprises units having Formulae 1 and 2, Formulae 1 and 3, or Formulae 1, 2, and 3, wherein Formula 1, Formula 2, and Formula 3 are represented by the following structures:

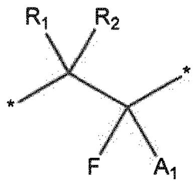
Formula 1

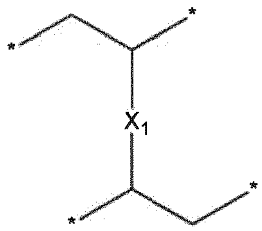
Formula 2

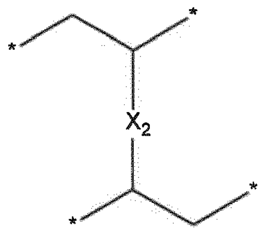
Formula 3

wherein $R_1$ and $R_2$ are each independently hydrogen, alkyl, cycloalkyl, or aryl; $A_1$ is carboxylic, phosphonic, or phosphoric; $X_1$ is arylene; and $X_2$ is alkylene, an ether moiety, or an amide moiety. More specifically, $R_1$ and $R_2$ are each independently hydrogen, alkyl, cycloalkyl, or aryl; $A_1$ is carboxylic, phosphonic, or phosphoric; $X_1$ is arylene; and $X_2$ is alkylene, an ether moiety, or an amide moiety.

When $X_2$ is an ether moiety, the ether moiety can be $-(CH_2)_d-O-(CH_2)_e-$ or $-(CH_2)_d-O-(CH_2)_e-O-(CH_2)_d-$, wherein d and e are independently an integer of 1 through 5. In some instances, d is an integer from 1 to 2 and e is an integer from 1 to 3. When $X_2$ is an amide moiety, the amide moiety can be $-C(O)-NH-(CH_2)_p-NH-C(O)-$ wherein p is an integer of 1 through 8. In some instances, p is an integer of 4 to 6.

The unit corresponding to Formula 2 can be derived from a difunctional crosslinking monomer having the formula $CH_2=CH-X_1-CH=CH_2$ wherein $X_1$ is as defined in connection with Formula 2. Further, the unit corresponding to Formula 3 can be derived from a difunctional crosslinking monomer having the formula $CH_2=CH-X_2-CH=CH_2$ wherein $X_2$ is as defined in connection with Formula 3.

In connection with Formula 1, in one embodiment, $R_1$ and $R_2$ are hydrogen and $A_1$ is carboxylic. In connection with Formula 2, in one embodiment, $X_1$ is an optionally substituted phenylene, and preferably phenylene. In connection with Formula 3, in one embodiment, $X_2$ is optionally substituted ethylene, propylene, butylene, pentylene, or hexylene; more specifically, $X_2$ is ethylene, propylene, butylene, pentylene, or hexylene; and preferably $X_2$ is butylene. In one specific embodiment, $R_1$ and $R_2$ are hydrogen, $A_1$ is carboxylic, $X_1$ is phenylene and $X_2$ is butylene.

Any of the pharmaceutical compositions of the invention can comprise a crosslinked carboxylic cation exchange polymer as described herein. Specifically, the compositions can include a crosslinked cation exchange polymer comprising structural units corresponding to Formulae 1 and 2, Formulae 1 and 3, or Formulae 1, 2, and 3.

In one embodiment, the crosslinked cation exchange polymer comprises at least about 80 wt.%, particularly at least about 85 wt.%, and more particularly at least about 90 wt.% or from about 80 wt.% to about 95 wt.%, from about 85 wt.% to about 95 wt.%, from about 85 wt.% to about 93 wt.% or from about 88 wt.% to about 92 wt.% of structural units corresponding to Formula 1 based on the total weight of the structural units as used in the polymerization mixture corresponding to (i) Formulae 1 and 2, (ii) Formulae 1 and 3, or (iii) Formulae 1, 2, and 3. Additionally, the polymer can comprise a unit of Formula 1 having a mole fraction of at least about 0.87 or from about 0.87 to about 0.94 or from about 0.90 to about 0.92 based on the total number of moles of the units corresponding to (i) Formulae 1 and 2, (ii) Formulae 1 and 3, or (iii) Formulae 1, 2, and 3.

In some aspects, the crosslinked cation exchange polymer comprises units corresponding to (i) Formulae 1A and 2A, (ii) Formulae 1A and 3A, or (iii) Formulae 1A, 2A, and 3A, wherein Formulae 1A, 2A and 3A are generally represented by the following structures.

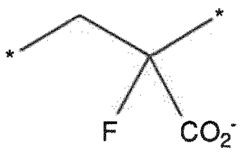

Formula 1A

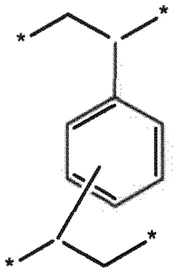

Formula 2A

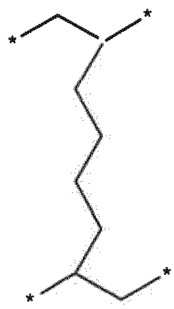

Formula 3A

In Formula 1 or 1A, the carboxylic acid is preferably in the salt form (i.e., balanced with a counter-ion such as $Ca^{2+}$, $Mg^{2+}$, $Na^{+}$, $NH^{4+}$, and the like). Preferably, the carboxylic acid is in the salt form and balanced with a $Ca^{2+}$ counterion. When the carboxylic acid of the crosslinked cation exchange form is balanced with a divalent counterion, two carboxylic acid groups can be associated with the one divalent cation.

The polymers described herein are generally random polymers wherein the exact order of the structural units of Formulae 1, 2, or 3 (derived from monomers of Formulae 11, 22, or 33), or 1A, 2A, or 3A (derived from monomers of Formulae 11A, 22A, or 33A) is not predetermined.

The present invention is also directed to particularly preferred crosslinked cation exchange polymers comprising a fluoro group and an acid group that is the polymerization product of at least three monomers and processes for the preparation thereof. The polymers or pharmaceutical compositions of these polymers are useful to bind potassium in the gastrointestinal tract.

In general, two of the three monomers should be difunctional cross-linking monomers having different rates of reaction with the methyl fluoroacrylate (MeFA) monomer. Without wishing to be bound by any particular theory, it is believed that during polymerization, the use of two different cross-linking monomers having different rates of reaction of the monomer of Formula 11 (e.g., MeFA) allows for the faster rate cross-linking monomer to be consumed before the other monomers, creating an intermediate that is rich in the faster rate monomer. This in turn allows the remaining monomers to be consumed so that a second, slower reactivity rate cross linker provides additional crosslinking. Demonstration, for example, may come from analysis of the polymer product that reveals a distribution of crosslinking units within the structure such that the higher reactive rate monomer is more richly present in those portion(s) of the polymer produced earlier in time in the polymerization reaction, while the lower reactivity rate monomer structure is more richly present in portion(s) of the final product produced later in time.

In one embodiment, the polymer contains structural units of Formulae 1, 2, and 3 and has a weight ratio of the structural unit corresponding to Formula 2 to the structural unit corresponding to Formula 3 of from about 4:1 to about 1:4, from about 2:1 to 1:2, or about 1:1. Additionally, this polymer can have a mole ratio of the structural unit of Formula 2 to the structural unit of Formula 3 of from about 0.2:1 to about 7:1, from about 0.2:1 to about 3.5:1; from about 0.5:1 to about 1.3:1, from about 0.8 to about 0.9, or about 0.85:1.

Generally, the Formulae 1, 2 and 3 structural units of the terpolymer have specific ratios, for example, wherein the structural units corresponding to Formula 1 constitute at least about 85 wt.% or from about 80 to about 95 wt.%, from about 85 wt.% to about 93 wt.%, or from about 88 wt.% to about 92 wt.% based on the total weight of structural units of Formulae 1, 2, and 3 in the polymer, calculated based on the amounts of the monomers and crosslinkers, or the monomers of Formulae 11, 22, and 33, that are used in the polymerization reaction, , and the weight ratio of the structural unit corresponding to Formula 2 to the structural unit corresponding to Formula 3 is from about 4:1 to about 1:4, or about 1:1. Further, the ratio of structural units when expressed as the mole fraction of the structural unit of Formula 1 in the polymer is at least about 0.87 or from about 0.87 to about 0.94, or from about 0.9 to about 0.92, based on the total number of moles of the structural units of Formulae 1, 2, and 3, and the mole ratio of the structural unit of Formula 2 to the structural unit of Formula 3 is from about 0.2:1 to about 7:1, from about 0.2:1 to about 3.5:1, or from about 0.8 to about 0.9; or 0.85:1; again these calculations are performed using the amounts the monomers and crosslinkers, or the monomers of Formulae 11, 22, and 33, that are used in the polymerization reaction. It is not necessary to calculate conversion.

In some aspects, the crosslinked cation exchange polymer comprises units corresponding to Formulae 1A, 2A, and 3A, wherein Formula 1A, Formula 2A and Formula 3A correspond to the following structures.

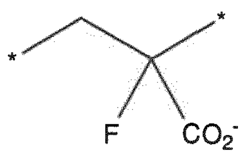

Formula 1A

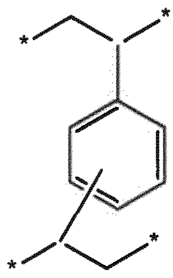

Formula 2A

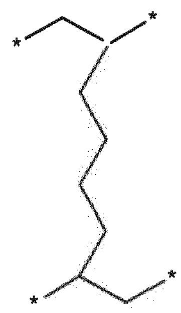

Formula 3A

In Formula 1 or 1A, the carboxylic acid can be in the acid form (i.e., balanced with hydrogen), in salt form (i.e., balanced with a counter-ion such as $Ca^{2+}$, $Mg^{2+}$, $Na^+$, $NH_4^+$, and the like) or in an ester form (i.e., balanced with an alkyl, such as methyl). Preferably, the carboxylic acid is in the salt form and balanced with a $Ca^{2+}$ counterion. When the carboxylic acid of the crosslinked cation exchange form is balanced with a divalent counterion, two carboxylic acid groups can be associated with the one divalent cation.

The structural units of the terpolymer can have specific ratios, for example, wherein the structural units corresponding to Formula 1A constitute at least about 85 wt.% or from about 80 to about 95 wt.%, from about 85 wt.% to about 93 wt.%, or from about 88 wt.% to about 92 wt.% based on the total weight of structural units of Formulae 1A, 2A, and 3A, calculated based on the amounts of monomers of Formulae 11A, 22A, and 33A used in the polymerization reaction, and the weight ratio of the structural unit corresponding to Formula 2A to the structural unit corresponding to Formula 3A is from about 4:1 to about 1:4, or about 1:1. Further, the ratio of structural units when expressed as the mole fraction of the structural unit of Formula 1A in the polymer is at least about 0.87 or from about 0.87 to about 0.94, or from about 0.9 to about 0.92 based on the total number of moles of the structural units of Formulae 1A, 2A, and 3A calculated from the amount of monomers of Formulae 11A, 22A, and 33A used in the polymerization reaction, and the mole ratio of the structural unit of Formula 2A to the structural unit of Formula 3A is from about 0.2:1 to about 7:1, from about 0.2:1 to about 3.5:1, from about 0.5:1 to about 1.3:1, from about 0.8:1 to about 0.9:1, or about 0.85:1.

A cation exchange polymer derived from monomers of Formulae 11, 22, and 33, followed by hydrolysis, can have a structure represented as follows:

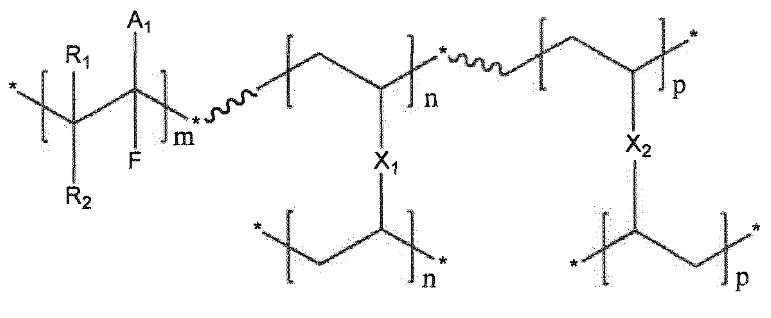

Formula 40

wherein $R_1$, $R_2$, $A_1$, $X_1$, and $X_2$ are as defined in connection with Formulae 1, 2, and 3 and m is in the range of from about 85 to about 93 mol%, n is in the range of from about 1 to about 10 mol% and p is in the range of from about 1 to about 10 mol%, calculated based on the ratios of monomers added to the polymerization mixture. The wavy bonds in the polymer structures of Formula 40 are included to represent the random attachment of structural units to one another wherein the structural unit of Formula 1 can be attached to another structural unit of Formula 1, a structural unit of Formula 2, or a structural unit of Formula 3; the structural units of Formulae 2 and 3 have the same range of attachment possibilities.

Using the polymerization process described herein, with monomers generally represented by Formulae 11A, 22A and 33A, followed by hydrolysis and calcium ion exchange, a polymer represented by the general structure shown below is obtained:

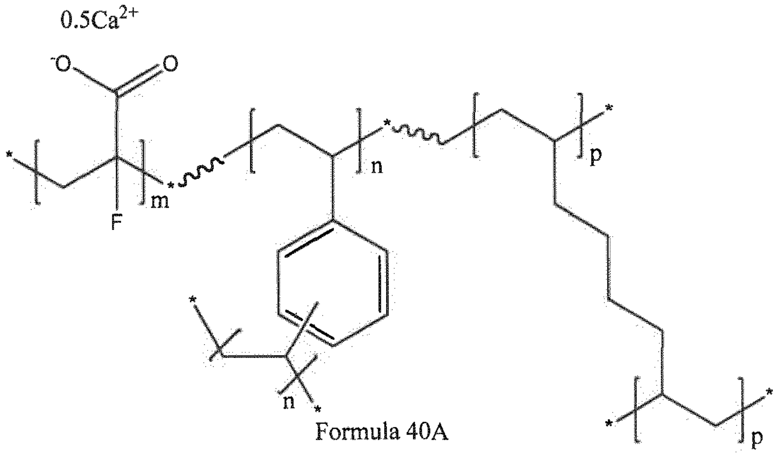

Formula 40A

wherein m is in the range of from about 85 to about 93 mol%, n is in the range of from about 1 to about 10 mol% and p is in the range of from about 1 to about 10 mol%, calculated based on the ratios of monomers added to the polymerization mixture. The wavy bonds in the polymer structures of Formula 40A are included to represent the random attachment of structural units to one another wherein the structural unit of Formula 1A can be attached to another structural unit of Formula 1A, a structural unit of Formula 2A, or a structural unit of Formula 3A; the structural units of Formulae 2A and 3A have the same range of attachment possibilities.

The crosslinked cation exchange polymer is generally a reaction product of a polymerization mixture that is subjected to polymerization conditions. The polymerization mixture may also contain components that are not chemically incorporated into the polymer. The crosslinked cation exchange polymer typically comprises a fluoro group and an acid group that is the product of the polymerization of three different monomer units where one monomer comprises a fluoro group and an acid group, another monomer is a difunctional arylene monomer and a third monomer is a difunctional alkylene, ether- or amide-containing monomer. More specifically, the crosslinked cation exchange polymer can be a reaction product of a polymerization mixture comprising monomers of Formulae 11, 22, 33. The monomer of Formula 11, the monomer of Formula 22, and the monomer of Formula 33 have the general formulas:

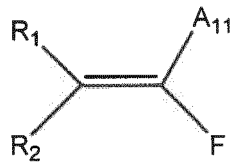

Formula 11

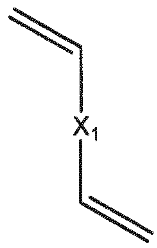

Formula 22

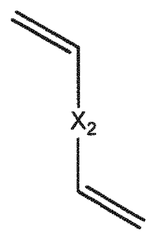

Formula 33

wherein $R_1$ and $R_2$ are as defined in connection with Formula 1, $X_1$ is as defined in connection with Formula 2, $X_2$ is as defined in connection with Formula 3, and $A_{11}$ is an optionally protected carboxylic, phosphonic, or phosphoric. In a preferred embodiment, $A_{11}$ is a protected carboxylic, phosphonic, or phosphoric. The polymerization mixture typically further comprises a polymerization initiator.

The reaction product of the polymerization mixture comprising Formulae 11, 22, 33 comprises a polymer having protected acid groups and comprising units corresponding to Formula 10 and units corresponding to Formulae 2 and 3.

Generally, the reaction mixture contains at least about 80 wt.%, particularly at least about 85 wt.%, and more particularly at least about 90 wt.% or from about 80 wt.% to about 95 wt.%, from about 85 wt.% to about 95 wt.%, from about 85 wt.% to about 93 wt.% or from about 88 wt.% to about 92 wt.% of monomers corresponding to Formula 11 based on the total weight of the monomers corresponding to Formulae 11, 22, and 33; and the mixture having a weight ratio of the monomer corresponding to Formula 22 to the monomer corresponding to Formula 33 from about 4:1 to about 1:4, from about 2:1 to 1:2, or about 1:1. Additionally, the reaction mixture can comprise a unit corresponding to Formula 11 having a mole fraction of at least about 0.87 or from about 0.87 to about 0.94 based on the total number of moles of the monomers corresponding to Formulae 11, 22, and 33 and the mixture having a mole ratio of the monomer corresponding to Formula 22 to the monomer corresponding to Formula 33 of from about 0.2:1 to about 7:1, from about 0.2:1 to about 3.5:1; from about 0.5:1 to about 1.3:1, from about 0.8 to about 0.9, or about 0.85:1.

In some embodiments, the polymer useful for treating hyperkalemia may be a resin having the physical properties discussed herein and comprising polystyrene sulfonate cross linked with divinyl benzene. Various resins having this structure are available from The Dow Chemical Company under the trade name Dowex, such as Dowex 50WX2, 50WX4 or 50WX8.

The crosslinked cation exchange polymer is generally the reaction product of a polymerization mixture that is subjected to polymerization conditions. The polymerization mixture may also contain components that are not chemically incorporated into the polymer. The crosslinked cation exchange polymer typically comprises a fluoro group and an acid group that is the product of the polymerization of at least two, and optionally three, different monomer units where one monomer comprises a fluoro group and an acid group and the other monomer is a difunctional arylene monomer or a difunctional alkylene, ether- or amide-containing monomer, or a combination thereof. More specifically, the crosslinked cation exchange polymer can be a reaction product of a polymerization mixture comprising monomers of either (i) Formulae 11 and 22, (ii) Formulae 11 and 33, or (iii) Formulae 11, 22, and 33. The monomers of Formulae 11, 22, and 33 are generally represented by

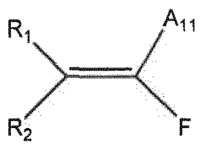

Formula 11

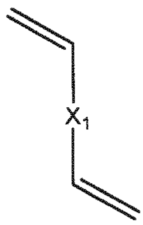

Formula 22

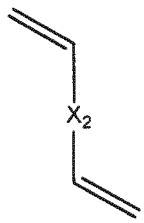

Formula 33

wherein $R_1$ and $R_2$ are as defined in connection with Formula 1, $X_1$ is as defined in connection with Formula 2, $X_2$ is as defined in connection with Formula 3, and $A_{11}$ is an optionally protected carboxylic, phosphonic, or phosphoric. In a preferred embodiment, $A_{11}$ is a protected carboxylic, phosphonic, or phosphoric.

The product of a polymerization reaction comprising monomers of (i) Formulae 11 and 22, (ii) Formulae 11 and 33, or (iii) Formulae 11, 22, and 33 comprises a polymer having optionally protected acid groups and comprising units corresponding to Formula 10 and units corresponding to Formulae 2 and 3. Polymer products having protected acid groups can be hydrolyzed to form a polymer having unprotected acid groups and comprising units corresponding to Formulae 1, 2, and 3. The structural units generally represented by Formula 10 have the structure

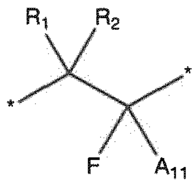

Formula 10

wherein $R_1$, $R_2$, and $A_{11}$ are as defined in connection with Formula 11.

In preferred embodiments of any of the methods of the invention wherein the crosslinked cation exchange polymer is a reaction product of a polymerization mixture of monomers, $A_{11}$ is a protected carboxylic, phosphonic, or phosphoric. The polymer formed in the polymerization reaction contains protected carboxylic, phosphonic, or phosphoric groups. A hydrolysis agent can be added to the polymer formed in the polymerization reaction to hydrolyze these protected groups, converting them to carboxylic, phosphonic, or phosphoric groups, or other methods of deprotection well known in the art can be used. The hydrolyzed polymer is preferably subjected to ion exchange to obtain a preferred polymer salt for therapeutic use.

In one embodiment, the reaction mixture comprises at least about 80 wt.%, particularly at least about 85 wt.%, and more particularly at least about 90 wt.% or from about 80 wt.% to about 95 wt.%, from about 85 wt.% to about 95 wt.%, from about 85 wt.% to about 93 wt.% or from about 88 wt.% to about 92 wt.% of monomers corresponding to Formula 11 based on the total weight of the monomers corresponding to (i) Formulae 11 and 22, (ii) Formulae 11 and 33, or (iii) Formulae 11, 22, and 33. Additionally, the reaction mixture can comprise a unit of Formula 11 having a mole fraction of at least about 0.87 or from about 0.87 to about 0.94 based on the total number of moles of the monomers corresponding to (i) Formulae 11 and 22, (ii) Formulae 11 and 33, or (iii) Formulae 11, 22, and 33.

In one embodiment, the polymerization reaction mixture contains monomers of Formulae 11, 22, and 33 and has a weight ratio of the monomer corresponding to Formula 22 to the monomer corresponding to Formula 33 from about 4:1 to

about 1:4, from about 2:1 to 1:2, or about 1:1. Additionally, this mixture can have a mole ratio of the monomer of Formula 22 to the monomer of Formula 33 from about 0.2:1 to about 7:1, from 0.2:1 to 3.5:1, from about 0.5:1 to about 1.3:1, from about 0.8:1 to about 0.9:1, or about 0.85:1.

Particular crosslinked cation exchange polymers are the reaction product of a polymerization mixture comprising monomers of (i) Formulae 11 and 22, (ii) Formulae 11 and 33, or (iii) Formulae 11, 22, and 33. The monomers are generally represented by Formulae 11A, 22A, and 33A having the structure:

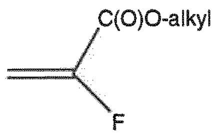

Formula 11A

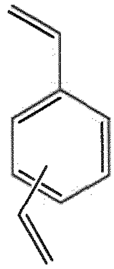

Formula 22A

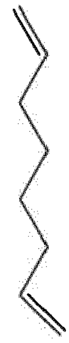

Formula 33A

wherein alkyl is preferably selected from methyl, ethyl, propyl, iso-propyl, butyl, iso-butyl, secbutyl, tert-butyl, pentyl, iso-pentyl, sec-pentyl, or tert-pentyl. Most preferably, the alkyl group is methyl or tert-butyl. The -O-alkyl moiety protects the carboxyl moiety from reacting with other reactive moieties during the polymerization reaction and can be removed by hydrolysis or other deprotection methods as described in more detail below.

Further, the polymerization reaction mixture contains at least about 80 wt.%, particularly at least about 85 wt.%, and more particularly at least about 90 wt.% or from about 80 wt.% to about 95 wt.%, from about 85 wt.% to about 95 wt.%, from about 85 wt.% to about 93 wt.% or from about 88 wt.% to about 92 wt.% of monomers corresponding to Formula 11A based on the total weight of the monomers which are generally represented by (i) Formulae 11A and 22A, (ii) Formulae 11A and 33A, or (iii) Formulae 11A, 22A, and 33A. Additionally, the reaction mixture can comprise a unit of Formula 11A having a mole fraction of at least about 0.87 or from about 0.87 to about 0.94 or from about 0.9 to about 0.92 based on the total number of moles of the monomers present in the polymer which are generally represented by (i) Formulae 11A and 22A, (ii) Formulae 11A and 33A, or (iii) Formulae 11A, 22A, and 33A.

In some instances, the reaction mixture contains monomers of Formulae 11, 22, and 33 and the weight ratio of the monomer generally represented by Formula 22A to the monomer generally represented by Formula 33A of from about 4:1 to about 1:4 or about 1:1. Also, this mixture has a mole ratio of the monomer of Formula 22A to the monomer of Formula 33A of from about 0.2:1 to about 7:1, from about 0.2:1 to about 3.5:1, from about 0.5:1 to about 1.3:1, from about 0.8:1 to about 0.9:1, or about 0.85:1.

In a preferred embodiment, an initiated polymerization reaction is employed where a polymerization initiator is used in the polymerization reaction mixture to aid initiation of the polymerization reaction. When preparing poly(methylfluoroacrylate) or (polyMeFA) or any other crosslinked cation exchange polymer used in the invention in a suspension polymerization reaction, the nature of the free radical initiator plays a role in the quality of the suspension in terms of polymer particle stability, yield of polymer particles, and the polymer particle shape. Use of water-insoluble free radical initiators, such as lauroyl peroxide, can produce polymer particles in a high yield. Without being bound by any particular theory, it is believed that a water-insoluble free radical initiator initiates polymerization primarily within the dispersed phase containing the monomers of Formulae 11 and 22, 11 and 33, or 11, 22, and 33. Such a reaction scheme provides polymer particles rather than a bulk polymer gel. Thus, the process uses free radical initiators with water solubility lower than 0.1 g/L, particularly lower than 0.01 g/L. In particular embodiments, polymethylfluoroacrylate particles are produced with a combination of a low water solubility free radical initiator and the presence of a salt in the aqueous phase, such as sodium chloride.

The polymerization initiator can be chosen from a variety of classes of initiators. For instance, initiators that generate polymer imitating radicals upon exposure to heat include peroxides, persulfates or azo type initiators (e.g., 2,2'-azobis(2-methylpropionitrile), lauroyl peroxide (LPO), tert-butyl hydro peroxide, dimethyl-2,2'-azobis(2-methylpropionate), 2,2'-azobis(2-methyl-N-(2-hydroxyethyl)propionamide), 2,2'-azobis(2-(2-imidazolin-2-yl)propane), (2,2"-azo bis(2,4-dimethylvaleronitrile), azobisisobutyronitrile (AIBN) or a combination thereof. Another class of polymer initiating radicals is radicals generated from redox reactions, such as persulfates and amines. Radicals can also be generated by exposing certain initiators to UV light or exposure to air.

For those polymerization reactions that contain additional components in the polymerization mixture that are not intended to be incorporated into the polymer, such additional components typically comprise surfactants, solvents, salts,

buffers, aqueous phase polymerization inhibitors and/or other components known to those of skill in the art. When the polymerization is carried out in a suspension mode, the additional components may be contained in an aqueous phase while the monomers and initiator may be contained in an organic phase. When an aqueous phase is present, the aqueous phase may be comprised of water, surfactants, stabilizers, buffers, salts, and polymerization inhibitors. A surfactant may be selected from the group consisting of anionic, cationic, nonionic, amphoteric, zwitterionic, or a combination thereof. Anionic surfactants are typically based on sulfate, sulfonate or carboxylate anions. These surfactants include, sodium dodecyl sulfate (SDS), ammonium lauryl sulfate, other alkyl sulfate salts, sodium laureth sulfate (or sodium lauryl ether sulfate (SLES)), N-lauroylsarcosine sodium salt, lauryldimethylamine-oxide (LDAO), ethyltrimethylammoniumbromide (CTAB), bis(2-ethylhexyl)sulfosuccinate sodium salt, alkyl benzene sulfonate, soaps, fatty acid salts, or a combination thereof. Cationic surfactants, for example, contain quaternary ammonium cations. These surfactants are cetyl trimethylammonium bromide (CTAB or hexadecyl trimethyl ammonium bromide), cetylpyridinium chloride (CPC), polyethoxylated tallow amine (POEA), benzalkonium chloride (BAC), benzethonium chloride (BZT), or a combination thereof. Zwitterionic or amphoteric surfactants include dodecyl betaine, dodecyl dimethylamine oxide, cocamidopropyl betaine, coco ampho glycinate, or a combination thereof. Nonionic surfactants include alkyl poly(ethylene oxide), copolymers of poly(ethylene oxide) and poly(propylene oxide) (commercially called Poloxamers or Poloxamines), alkyl polyglucosides (including octyl glucoside, decyl maltoside, fatty alcohols, cetyl alcohol, oleyl alcohol, cocamide MEA, cocamide DEA), or a combination thereof. Other pharmaceutically acceptable surfactants are well known in the art and are described in McCutcheon's Emulsifiers and Detergents, N. American Edition (2007).

Polymerization reaction stabilizers may be selected from the group consisting of organic polymers and inorganic particulate stabilizers. Examples include polyvinyl alcohol-co-vinylacetate and its range of hydrolyzed products, polyvinylacetate, polyvinylpyrolidinone, salts of polyacrylic acid, cellulose ethers, natural gums, or a combination thereof.

Buffers may be selected from the group consisting of for example, 4-2-hydroxyethyl-1-piperazineethanesulfonic acid, 2-{[tris(hydroxymethyl)methyl] amino}ethanesulfonic acid, 3-(N-morpholino)propanesulfonic acid, piperazine-N,N'-bis(2-ethanesulfonic acid), sodium phosphate dibasic heptahydrate, sodium phosphate monobasic monohydrate or a combination thereof.

Polymerization reaction salts may be selected from the group consisting of potassium chloride, calcium chloride, potassium bromide, sodium bromide, sodium bicarbonate, ammonium peroxodisulfate, or a combination thereof.

Polymerization inhibitors may be used as known in the art and selected from the group consisting of 1,1,3-tris(2-methyl-4-hydroxy-5-tert-butylphenyl)butane, 1,3,5-trimethyl-2,4,6-tris(3,5-di-tert-butyl-4-hydroxybenzyl)benzene, 1-aza-3,7-dioxabicyclo[3.3.0]octane-5-methanol, 2,2'-ethylidene-bis(4,6-di-tert-butylphenol), 2,2'-ethylidenebis(4,6-di-tert-butylphenyl) fluorophosphite, 2,2'-methylenebis(6-tert-butyl-4-ethylphenol), 2,2'-methylenebis(6-tert-butyl-4-methylphenol), 2,5-di-tert-butyl-4-methoxyphenol, 2,6-di-tert-butyl-4-(dimethylaminomethyl)phenol, 2-heptanone oxime, 3,3',5,5'-tetramethylbiphenyl-4,4'-diol, 3,9-bis(2,4-dicumylphenoxy)-2,4,8,10-tetraoxa-3,9-diphosphaspiro[5.5] undecane, 4,4-dimethyloxazolidine, 4-methyl-2-pentanone oxime, 5-ethyl-1-aza-3,7-dioxabicyclo[3.3.0]octane, 6,6'-dihydroxy-5,5'-dimethoxy-[1,1'-biphenyl]-3,3'-dicarboxaldehyde, distearyl-3,3'-thiodipropionate, ditetradecyl-3,3'-thiodipropionate, ditridecyl-3,3'-thiodipropionate, octadecyl-3-(3,5-di-tert-butyl-4-hydroxyphenyl)propionate, pentaerythritol tetrakis(3,5-di-tert-butyl-4-hydroxyhydrocinnamate), poly(1,2-dihydro-2,2,4-trimethylquinoline), sodium D-isoascorbate monohydrate, tetrakis(2,4-di-tert-butylphenyl)-4,4'-biphenyldiphosphonite, tris(3,5-di-tert-butyl-4-hydroxybenzyl) isocyanurate, tris(4-tert-butyl-3-hydroxy-2,6-dimethylbenzyl) isocyanurate, sodium nitrite or a combination thereof.

Generally, the polymerization mixture is subjected to polymerization conditions. While suspension polymerization is preferred, as already discussed herein, the polymers used in this invention may also be prepared in bulk, solution or emulsion polymerization processes. The details of such processes are within the skill of one of ordinary skill in the art based on the disclosure of this invention. The polymerization conditions typically include polymerization reaction temperatures, pressures, mixing and reactor geometry, sequence and rate of addition of polymerization mixtures and the like. Polymerization temperatures are typically in the range of from about 50 to 100°C. Polymerization pressures are typically run at atmospheric pressure, but can be run at higher pressures (for example 130 PSI of nitrogen). Polymerization mixing depends on the scale of the polymerization and the equipment used, and is within the skill of one of ordinary skill in the art. Various alpha-fluoroacrylate polymers and the synthesis of these polymers are described in U.S. Patent Application Publication No. 2005/0220752, herein incorporated by reference.

As described in more detail in connection with the examples herein, in various particular embodiments, the crosslinked cation exchange polymer can be synthesized by preparing an organic phase and an aqueous phase. The organic phase typically contains a polymerization initiator and (i) a monomer of Formula 11 and a monomer of Formula 22, (ii) a monomer of Formula 11 and a monomer of Formula 33, or (iii) monomers of Formulae 11, 22, and 33. The aqueous phase generally contains a polymerization suspension stabilizer, a water soluble salt, water, and optionally a buffer. The organic phase and the aqueous phase are then combined and stirred under nitrogen. The mixture is generally heated to about 60°C to about 80°C for about 2.5 to about 3.5 hours, allowed to rise up to 95°C after polymerization is initiated, and then cooled to room temperature. After cooling, the aqueous phase is removed. Water is added to the mixture, the mixture is stirred, and the resulting solid is filtered. The solid is washed with water, alcohol, or alcohol/water mixtures.

As described above, polymerization suspension stabilizers, such as polyvinyl alcohol, are used to prevent coalescence of particles during the polymerization process. Further, it has been observed that the addition of sodium chloride in the aqueous phase decreased coalescence and particle aggregation. Other suitable salts for this purpose include salts that are soluble in the aqueous phase. In this embodiment, water soluble salts are added at a concentration of from about 0.1 wt.% to about 10 wt.%, particularly from about 2 wt.% to about 5 wt.%, and even more particularly from about 3 wt.% to about 4 wt.%.

Preferably, an organic phase of methyl 2-fluoroacrylate (90 wt.%), 1,7-octadiene (5 wt.%) and divinylbenzene (5 wt.%) is prepared and 0.5 wt.% of lauroyl peroxide is added to initiate the polymerization reaction. Additionally, an aqueous phase of water, polyvinyl alcohol, phosphates, sodium chloride, and sodium nitrite is prepared. Under nitrogen and while keeping the temperature below about 30°C, the aqueous and organic phases are mixed together. Once mixed completely, the reaction mixture is gradually heated with continuous stirring. After the polymerization reaction is initiated, the temperature of the reaction mixture is allowed to rise up to about 95°C. Once the polymerization reaction is complete, the reaction mixture is cooled to room temperature and the aqueous phase is removed. The solid can be isolated by filtration once water is added to the mixture. The filtered solid is washed with water and then with a methanol/water mixture. The resulting product is a crosslinked (methyl 2-fluoroacrylate)-divinylbenzene-1,7-octadiene terpolymer.

As discussed herein, after polymerization, the product may be hydrolyzed or otherwise deprotected by methods known in the art. For hydrolysis of the polymer having ester groups to form a polymer having carboxylic acid groups, preferably, the polymer is hydrolyzed with a strong base (e.g., NaOH, KOH, $Mg(OH)_2$ or $Ca(OH)_2$) to remove the alkyl (e.g., methyl) group and form the carboxylate salt. Alternatively, the polymer can be hydrolyzed with a strong acid (e.g., HCl) to form the carboxylate salt. Preferably, the (methyl 2-fluoroacrylate)-divinylbenzene-1,7-octadiene terpolymer is hydrolyzed with an excess of aqueous sodium hydroxide solution at a temperature from about 30°C to about 100°C to yield (sodium 2-fluoroacrylate)-divinylbenzene-1,7-octadiene terpolymer. Typically, the hydrolysis reaction is carried out for about 15 to 25 hours. After hydrolysis, the solid is filtered and washed with water and/or an alcohol.

The cation of the polymer salt formed in the hydrolysis reaction or other deprotection step depends on the base used in that step. For example, when sodium hydroxide is used as the base, the sodium salt of the polymer is formed. This sodium ion can be exchanged for another cation by contacting the sodium salt with an excess of an aqueous metal salt to yield an insoluble solid of the desired polymer salt. After the desired ion exchange, the product is washed with an alcohol and/or water and dried directly or dried after a dewatering treatment with denatured alcohol; preferably, the product is washed with water and dried directly. For example, the sodium salt of the cation exchange polymer is converted to the calcium salt by washing with a solution that substitutes calcium for sodium, for example, by using calcium chloride, calcium acetate, calcium lactate gluconate, or a combination thereof. And, more specifically, to exchange sodium ions for calcium ions, the (sodium 2-fluoroacrylate)-divinylbenzene-1,7-octadiene terpolymer is contacted with an excess of aqueous calcium chloride to yield an insoluble solid of crosslinked (calcium 2-fluoroacrylate)-divinylbenzene-1,7-octadiene terpolymer.

Using this suspension polymerization process, cross-linked polyMeFA polymer is isolated in good yield, generally above about 85%, more specifically above about 90%, and even more specifically above about 93%. The yield of the second step (i.e., hydrolysis) preferably occurs in 100%, providing an overall yield above about 85%, more specifically above about 90%, and even more specifically above about 93%.

To add a linear polyol to the linear polyol stabilized compositions of the invention, the salt of the polymer is slurried with an aqueous solution of polyol (e.g., sorbitol), typically with the slurry containing an excess amount of polyol based on polymer weight. Performing this step can reduce inorganic fluoride in the composition. The slurry is maintained under conditions known to those of skill in the art, such as for at least 3 hours and ambient temperature and pressure. The solids are then filtered off and dried to desired moisture content.

The compositions of the invention are tested for their characteristics and properties using a variety of established testing procedures. For example, the percent inorganic fluoride in the composition is tested by mixing a dried sample of composition with C-Wax in a defined proportion, and making a pellet by pressing it with a force of about 40kN in an aluminum cup. Percent fluorine content is analyzed by X-ray fluorescence in a manner known to those of skill in the art, for example, using a Bruker AXS SRS 3400 (Bruker AXS, Wisconsin). In general, the amount of organic fluorine in the composition is less than 25 wt.%, preferably less than 20 wt. %, more preferably 7 wt.% to 25 wt.% and most preferably 7 wt.% to 20 wt.% based on the total weight of the composition. The percent calcium in the polymer or composition is tested after extraction with an appropriate acid (e.g., 3M hydrochloric acid) using inductively coupled plasma optical emission spectroscopy (ICP-OES) analysis in a manner known to those of skill in the art, for example, using a Thermo IRIS Intrepid II XSP (Thermo Scientific, Waltham, MA). In general, the amount of calcium in the polymer is in the range of from about 8 wt.% to about 25 wt.%, and preferably about 10 wt.% to about 20 wt.%, based on the total weight of the polymer.

Also for example, the potassium binding capacity can be used for polymer or composition characterization. In this example, the potassium binding capacity is performed in vitro by weighing and transferring approximately 300 mg of a dried sample of polymer or composition into a 40 mL screw-top vial, and then adding a calculated volume of 200 mM KCl solution to achieve a concentration of 20 mg/mL of test substance. The vial is shaken vigorously for two hours, and the

supernatant is filtered through a 0.45 μm filter followed by dilution to 1:20 in water. The supernatant is analyzed for potassium concentration via ICP-OES, and the potassium binding is calculated using the following formula.

$$\text{Potassium binding} = \frac{20\ \text{(dilution factor)}}{20\ \text{mg/mL (sample conc)}} \times ([K]_{blank} - [K]_{sample}) \ \frac{\text{mmol K}}{\text{g polymer}}$$

One aspect of the invention is a method of removing potassium ions from the gastrointestinal tract of an animal subject in need thereof with a crosslinked cation exchange polymer or a pharmaceutical composition of the invention. The crosslinked cation exchange polymer generally has a high overall exchange capacity. The overall exchange capacity is the maximum amount of cations bound by the cation exchange polymer measured in mEq/g. A higher exchange capacity is desired as it is a measure of the density of acid groups in the polymer and the more acid groups per unit weight, the greater the overall exchange capacity of the polymer.

The crosslinked cation exchange polymers and the compositions comprising linear polyol and crosslinked cation exchange polymer also generally have a high binding capacity for potassium. In particular, the *in vivo* binding capacity is relevant to therapeutic benefit in a patient. Generally, a higher *in vivo* binding capacity results in a more pronounced therapeutic effect. However, since patients can have a wide range of responses to the administration of cation exchange polymers, one measure of the *in vivo* binding capacity for potassium is the average *in vivo* binding capacity calculated over a sample group. The term "high capacity" as used herein encompasses an average *in vivo* binding of about 1.0 mEq or more of potassium per gram of polymer.

One measure of the *in vivo* potassium binding capacity is the use of *ex vivo* human aspirates. For this method, healthy patients are given a meal as a digestion mimic and aliquots of chyme are then sampled using a tube placed in the lumen of the small intestine and other portions of the intestines. For example, normal subjects are intubated with a double lumen polyvinyl tube, with a mercury weighted bag attached to the end of the tube to facilitate movement of the tube into the small intestine. One aspiration aperture of the double lumen tube is located in the stomach and the other aperture is at the Ligament of Treitz (in the upper jejunum). Placement takes place with the use of fluoroscopy. After the tube is placed, 550 mL of a liquid standard test meal (supplemented with a marker, polyethylene glycol (PEG) - 2 g/550mL) is infused into the stomach through the gastric aperture at a rate of 22 mL per minute. It requires approximately 25 minutes for the entire meal to reach the stomach. This rate of ingestion simulates the duration of time required to eat normal meals. Jejunal chyme is aspirated from the tube whose lumen is located at the Ligament of Treitz. This fluid is collected continuously during 30-minute intervals for a two and a half hour period. This process results in five specimens that are mixed, measured for volume, and lyophilized.

The potassium binding procedure is identical to the one described below with the non-interfering buffer experiment, except that the *ex vivo* aspirate liquid is used (after reconstitution of the freeze-dried material in the proper amount of de-ionized water). The binding capacity in the *ex vivo* aspirate (VA) is calculated from the concentration of potassium in the aspirate with and without polymer. In some embodiments, the average *ex vivo* potassium binding capacity of a human gastrointestinal aspirate can be equal to or more than about 0.7 mEq per gram of polymer. More specifically, the *ex vivo* potassium binding capacity of a human gastrointestinal aspirate is about 0.8 mEq or more per gram, more particularly is about 1.0 mEq or more per gram, even more particularly is about 1.2 mEq or more per gram, and most particularly is about 1.5 mEq or more per gram.

Another measure of the *in vivo* binding capacity for potassium is the *in vitro* binding capacity for potassium in non-interfering environment or an interfering environment at a particular pH. In a non-interfering environment, the crosslinked cation exchange polymer is placed in a solution having potassium ions as the only cation. This solution is preferably at an appropriate GI physiological pH (e.g., about 6.5). The *in vitro* binding capacity for potassium in a non-interfering environment is a measure of the total binding capacity for cations.

Further, in an interfering environment, the environment contains cations in concentrations relevant to the typical concentrations in the gastrointestinal tract and is at physiological pH (e.g., about 6.5). In the interfering environment, it is preferred that the polymer or the pharmaceutical composition exhibit selective binding for potassium ions.

In some embodiments, the *in vitro* potassium binding capacity is determined in solutions with a pH of about 5.5 or more. In various embodiments, *in vitro* potassium binding capacity in a pH of about 5.5 or more is equal to or more than 6 mEq per gram of polymer. A particular range of *in vitro* potassium binding capacity in a pH of about 5.5 or more is about 6 mEq to about 12 mEq per gram of polymer. Preferably the *in vitro* potassium binding capacity in a pH of about 5.5 or more is equal to about 6 mEq or more per gram, more particularly is about 7 mEq or more per gram, and even more particularly is about 8 mEq or more per gram.

The higher capacity of the polymer may enable the administration of a lower dose of the pharmaceutical composition. Typically the dose of the polymer used to obtain the desired therapeutic and/or prophylactic benefits is about 0.5 gram/day to about 60 grams/day. A particular dose range is about 5 grams/day to about 60 grams/day, and more particularly is about 5 grams/day to about 30 grams/day. In various administration protocols, the dose is administered

about three times a day, for example, with meals. In other protocols, the dose is administered once a day or twice a day. These doses can be for chronic or acute administration.

Generally, the polymers, polymer particles and pharmaceutical compositions described herein retain a significant amount of the bound potassium, and specifically, the potassium bound by the polymer is not released prior to excretion of the polymer in the feces. The term "significant amount" as used herein is not intended to mean that the entire amount of the bound potassium is retained prior to excretion. A sufficient amount of the bound potassium is retained, such that a therapeutic and/or prophylactic benefit is obtained. Particular amounts of bound potassium that can be retained range from about 5% to about 100%. The polymer or pharmaceutical composition should retain about 25% of the bound potassium, more particularly about 50%, even more particularly about 75% and most particularly retain about 100% of the bound potassium. The period of retention is generally during the time that the polymer or composition is being used therapeutically. In the embodiment in which the polymer or composition is used to bind and remove potassium from the gastrointestinal tract, the retention period is the time of residence of the polymer or composition in the gastrointestinal tract and more particularly the average residence time in the colon.

Generally, the cation exchange polymers and polymer particles are not significantly absorbed from the gastrointestinal tract. Depending upon the size distribution of the cation exchange polymer particles, clinically insignificant amounts of the polymers may be absorbed. More specifically, about 90% or more of the polymer is not absorbed, about 95% or more is not absorbed, even more specifically about 97% or more is not absorbed, and most specifically about 98% or more of the polymer is not absorbed.

In some embodiments of the invention, the polymers and polymer particlesused in the invention will be administered unformulated (i.e., containing no additional carriers or other components). In other instances, a pharmaceutical composition containing the polymer, a stabilizing linear polyol and optionally water will be administered as described herein.

The methods, polymers, polymer particles and compositions described herein are suitable for removal of potassium from a patient wherein a patient is in need of such potassium removal. For example, patients experiencing hyperkalemia caused by disease and/or use of certain drugs benefit from such potassium removal. Further, patients at risk for developing high serum potassium concentrations through use of agents that cause potassium retention could be in need of potassium removal. The methods described herein are applicable to these patients regardless of the underlying condition that is causing the high serum potassium levels.

Dosing regimens for chronic treatment of hyperkalemia can increase compliance by patients, particularly for crosslinked cation exchange polymers, polymer particles, or compositions of the invention that are taken in gram quantities. The present invention is also directed to methods of chronically removing potassium from an animal subject in need thereof, and in particular chronically treating hyperkalemia with a potassium binder that is a crosslinked aliphatic carboxylic polymer, and preferably a pharmaceutical composition comprising a crosslinked cation exchange polymer and a linear polyol as described herein.

It has now been found that when using the crosslinked cation exchange polymers, polymer particles and the compositions of the present invention, a once-a-day dose is substantially equivalent to a twice-a-day dose, which is also substantially equivalent to a three-times-a-day dose. Generally, the once per day or twice per day administration of a daily amount of the polymer or the composition, has a potassium binding capacity of at least 75% of the binding capacity of the same polymer or composition administered at the same daily amount three times per day. More specifically, the once per day or twice per day administration of a daily amount of the polymer or the composition has a potassium binding capacity of at least 80, 85, 90 or 95% of the binding capacity of the same polymer or composition administered at the same daily amount three times per day. Even more specifically, the once per day or twice per day administration of a daily amount of the polymer or the composition has a potassium binding capacity of at least 80% of the binding capacity of the same polymer or composition administered at the same daily amount three times per day. And even more specifically, the once per day or twice per day administration of a daily amount of the polymer or the composition has a potassium binding capacity of at least 90% of the binding capacity of the same polymer or composition administered at the same daily amount three times per day. Most preferably, the once per day or twice per day administration of a daily amount of the polymer or the composition has a potassium binding capacity that is not statistically significantly different from the binding capacity of the same polymer or composition at the same daily amount administered three times per day.

Additionally, the invention is directed to methods of removing potassium from an animal subject by administering a crosslinked cation exchange polymer or a pharmaceutical composition comprising a crosslinked cation exchange polymer and an effective amount or from about 10 wt.% to about 40 wt.% of a linear polyol to the subject once a day, wherein less than 25% of subjects taking the polymer or composition once per day experience mild or moderate gastrointestinal adverse events. Gastrointestinal adverse events may include flatulence, diarrhea, abdominal pain, constipation, stomatitis, nausea and/or vomiting. In some aspects, the polymer or composition is administered twice a day and less than 25% of subjects taking the polymer or composition twice per day experience mild or moderate gastrointestinal adverse events. In some instances, the subjects taking the polymer or composition once per day or twice per day experience no severe gastrointestinal adverse events. The crosslinked cation exchange polymers, polymer particles or pharmaceutical

compositions of the present invention have about 50% or more tolerability as compared to the same polymer or composition of the same daily amount administered three times a day. For example, for every two patients in which administration of the polymer three times a day is well tolerated, there is at least one patient in which administration of the polymer once a day or twice a day is well tolerated. The crosslinked cation exchange polymers, polymer particles or pharmaceutical compositions have about 75% or more tolerability as compared to the same polymer or composition of the same daily amount administered three times a day. It is also a feature of this invention that the cation exchange polymers, polymer particles or compositions administered once a day or twice a day have about 85% or more tolerability as the same polymer or composition of the same daily amount administered three times a day. It is also a feature of this invention that the cation exchange polymers, polymer particles or compositions administered once a day or twice a day have about 95% or more tolerability as the same polymer or composition of the same daily amount administered three times a day. It is also a feature of this invention that the cation exchange polymers, polymer particles or compositions administered once a day or twice a day have about substantially the same tolerability as the same polymer or composition of the same daily amount administered three times a day.

In other embodiments, the present invention provides a method of removing potassium from the gastrointestinal tract of an animal subject in need thereof, comprising administering an effective amount of any crosslinked cation exchange polymer, polymer particles, pharmaceutical composition, or a composition comprising a crosslinked cation exchange polymer and a linear polyol as described herein, once per day or twice per day to the subject, wherein the polymer, polymer particles or composition are as well tolerated as administering substantially the same amount of the same polymer or composition three times per day. In some instances, the subject is experiencing hyperkalemia and thus the method treats hyperkalemia. In other instances, the method lowers serum potassium. In particular embodiments, the potassium polymer is a crosslinked aliphatic carboxylic polymer.

The compositions and/or methods of this invention include a composition comprising a crosslinked cation exchange polymer and an effective amount or from about 10 wt.% to about 40 wt.% linear polyol that extracts from an animal subject in need thereof about 5% more potassium as compared to the same dose and same administration frequency of the same composition that does not contain the linear polyol. More specifically, the compositions and/or methods include a composition of the invention that extracts from an animal subject in need thereof about 10% more potassium as compared to the same dose and same administration frequency of the same composition that does not contain the linear polyol. And even more specifically, the compositions and/or methods include a composition of the invention that extracts from an animal subject in need thereof about 15% or about 20% more potassium as compared to the same dose and same administration frequency of the otherwise same composition that does not include the linear polyol.

If necessary, the crosslinked cation exchange polymers, polymer particles, pharmaceutical compositions, or compositions comprising a crosslinked cation exchange polymer and a linear polyol may be administered in combination with other therapeutic agents. The choice of therapeutic agents that can be co-administered with the compounds of the invention will depend, in part, on the condition being treated.

Further, patients suffering from chronic kidney disease and/or congestive heart failure can be particularly in need of potassium removal because agents used to treat these conditions may cause potassium retention in a significant population of these patients. For these patients, decreased renal potassium excretion results from renal failure (especially with decreased glomerular filtration rate), often coupled with the ingestion of drugs that interfere with potassium excretion, e.g., potassium-sparing diuretics, angiotensin-converting enzyme inhibitors (ACEs), angiotensin receptor blockers (ARBs), beta blockers, renin inhibitors, aldosterone synthase inhibitors, non-steroidal anti-inflammatory drugs, heparin, or trimethoprim. For example, patients suffering from chronic kidney disease can be prescribed various agents that will slow the progression of the disease; for this purpose, angiotensin-converting enzyme inhibitors (ACEs), angiotensin receptor blockers (ARBs), and aldosterone antagonists are commonly prescribed. In these treatment regimens the angiotensin-converting enzyme inhibitor is captopril, zofenopril, enalapril, ramipril, quinapril, perindopril, lisinopril, benazipril, fosinopril, or combinations thereof and the angiotensin receptor blocker is candesartan, eprosartan, irbesartan, losartan, olmesartan, telmisartan, valsartan, or combinations thereof and the renin inhibitor is aliskiren. The aldosterone antagonists can also cause potassium retention. Thus, it can be advantageous for patients in need of these treatments to also be treated with an agent that removes potassium from the body. The aldosterone antagonists typically prescribed are spironolactone, eplerenone, and the like.

In certain particular embodiments, the crosslinked cation exchange polymers, polymer particles or compositions described herein can be administered on a periodic basis to treat a chronic condition. Typically, such treatments will enable patients to continue using drugs that may cause hyperkalemia, such as potassium-sparing diuretics, ACEs, ARBs, aldosterone antagonists, $\beta$-blockers, renin inhibitors, non-steroidal anti-inflammatory drugs, heparin, trimethoprim, or combinations thereof. Also, use of the polymeric compositions described herein will enable certain patient populations, who were unable to use certain above-described drugs, to use such drugs.

In certain use situations, the crosslinked cation exchange polymers, polymer particles used are those that are capable of removing less than about 5 mEq of potassium per day, or in the range of about 5 mEq to about 60 mEq of potassium per day.

In certain other embodiments, the compositions and methods described herein are used in the treatment of hyperkalemia in patients in need thereof, for example, when caused by excessive intake of potassium. Excessive potassium intake alone is an uncommon cause of hyperkalemia. More often, hyperkalemia is caused by indiscriminate potassium consumption in a patient with impaired mechanisms for the intracellular shift of potassium or renal potassium excretion.

In the present invention, the crosslinked cation exchange polymers, polymer particles or compositions comprising a crosslinked cation exchange polymer and a linear polyol can be co-administered with other active pharmaceutical agents. This co-administration can include simultaneous administration of the two agents in the same dosage form, simultaneous administration in separate dosage forms, and separate administration. For example, for the treatment of hyperkalemia, the crosslinked cation exchange polymer or composition of the invention can be co-administered with drugs that cause the hyperkalemia, such as potassium-sparing diuretics, angiotensin-converting enzyme inhibitors (ACEs), angiotensin receptor blockers (ARBs), beta blockers, renin inhibitors, non-steroidal anti-inflammatory drugs, heparin, or trimethoprim. In particular, the crosslinked cation exchange polymer or composition can be co-administered with ACEs (e.g., captopril, zofenopril, enalapril, ramipril, quinapril, perindopril, lisinopril, benazipril, and fosinopril), ARBs (e.g., candesartan, eprosartan, irbesartan, losartan, olmesartan, telmisartan, and valsartan) and renin inhibitors (e.g. aliskiren). In particular embodiments, the agents are simultaneously administered, wherein both the agents are present in separate compositions. In other embodiments, the agents are administered separately in time (i.e., sequentially).

The term "treating" as used herein includes achieving a therapeutic benefit. By therapeutic benefit is meant eradication, amelioration, or prevention of the underlying disorder being treated. For example, in a hyperkalemia patient, therapeutic benefit includes eradication or amelioration of the underlying hyperkalemia. Also, a therapeutic benefit is achieved with the eradication, amelioration, or prevention of one or more of the physiological symptoms associated with the underlying disorder such that an improvement is observed in the patient, notwithstanding that the patient may still be afflicted with the underlying disorder. For example, administration of a potassium-binding polymer to a patient experiencing hyperkalemia provides therapeutic benefit not only when the patient's serum potassium level is decreased, but also when an improvement is observed in the patient with respect to other disorders that accompany hyperkalemia, like renal failure. In some treatment regimens, the crosslinked cation exchange polymer, polymer particles or composition of the invention may be administered to a patient at risk of developing hyperkalemia or to a patient reporting one or more of the physiological symptoms of hyperkalemia, even though a diagnosis of hyperkalemia may not have been made.

The pharmaceutical compositions of the present invention include compositions wherein the crosslinked cation exchange polymers or polymer particles are present in an effective amount, i.e., in an amount effective to achieve therapeutic or prophylactic benefit. The actual amount effective for a particular application will depend on the patient (e.g., age, weight, etc.), the condition being treated, and the route of administration. Determination of an effective amount is well within the capabilities of those skilled in the art, especially in light of the disclosure herein. The effective amount for use in humans can be determined from animal models. For example, a dose for humans can be formulated to achieve gastrointestinal concentrations that have been found to be effective in animals.

The polymers, polymer particles and compositions described herein can be used as food products and/or food additives. They can be added to foods prior to consumption or while packaging. The polymers, polymer particles and compositions can also be used in fodder for animals to lower potassium levels, which is desirable in fodders for pigs and poultry to lower the water secretion.

The crosslinked cation exchange polymers, polymer particles or pharmaceutically acceptable salts thereof, or compositions described herein, can be delivered to the patient using a wide variety of routes or modes of administration. The most preferred routes for administration are oral, intestinal, or rectal. Rectal routes of administration are known to those of skill in the art. Intestinal routes of administration generally refer to administration directly into a segment of the gastrointestinal tract, e.g., through a gastrointestinal tube or through a stoma. The most preferred route for administration is oral.

The polymers, polymer particles (or pharmaceutically acceptable salts thereof) may be administered per se or in the form of a pharmaceutical composition wherein the active compound(s) is in admixture or mixture with one or more pharmaceutically acceptable excipients. Pharmaceutical compositions for use in accordance with the present invention may be formulated in conventional manner using one or more pharmaceutically acceptable excipients comprising carriers, diluents, and auxiliaries which facilitate processing of the active compounds into preparations which can be used physiologically. Proper composition is dependent upon the route of administration chosen.

For oral administration, the polymers, polymer particles or compositions of the invention can be formulated readily by combining the polymer or composition with pharmaceutically acceptable excipients well known in the art. Such excipients enable the compositions of the invention to be formulated as tablets, pills, dragees, capsules, liquids, gels, syrups, slurries, suspensions, wafers, and the like, for oral ingestion by a patient to be treated. In one embodiment, the oral composition does not have an enteric coating. Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose or

sucrose; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinyl pyrrolidone (PVP); and various flavoring agents known in the art. If desired, disintegrating agents may be added, such as the cross-linked polyvinyl pyrrolidone, agar, or alginic acid or a salt thereof such as sodium alginate.

In various embodiments, the active ingredient (e.g., polymer) constitutes over about 20%, more particularly over about 40%, even more particularly over about 50%, and most particularly more than about 60% by weight of the oral dosage form, the remainder comprising suitable excipient(s). In compositions containing water and linear polyol, the polymer preferably constitutes over about 20%, more particularly over about 40%, and even more particularly over about 50% by weight of the oral dosage form.

In some embodiments, pharmaceutical compositions are in the form of liquid compositions. In various embodiments, the pharmaceutical composition contains a crosslinked cation exchange polymer dispersed in a suitable liquid excipient. Suitable liquid excipients are known in the art; see, e.g., Remington's Pharmaceutical Sciences.

Unless otherwise indicated, an alkyl group as described herein alone or as part of another group is an optionally substituted linear saturated monovalent hydrocarbon radical containing from one to twenty carbon atoms and preferably one to eight carbon atoms, or an optionally substituted branched saturated monovalent hydrocarbon radical containing three to twenty carbon atoms, and preferably three to eight carbon atoms. Examples of unsubstituted alkyl groups include methyl, ethyl, n-propyl, i-propyl, n-butyl, i-butyl, s-butyl, t-butyl, n-pentyl, i-pentyl, s-pentyl, t-pentyl, and the like.

The term "amide moiety" as used herein represents a bivalent (i.e., difunctional) group including at least one amido linkage (i.e.,

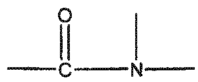

), such as $-C(O)-NR_A-R_C-NR_B-C(O)-$ wherein $R_A$ and $R_B$ are independently hydrogen or alkyl and Rc is alkylene. For example, an amide moiety can be $-C(O)-NH-(CH_2)_p-NH-C(O)-$ wherein p is an integer of 1 to 8.

The term "aryl" as used herein alone or as part of another group denotes an optionally substituted monovalent aromatic hydrocarbon radical, preferably a monovalent monocyclic or bicyclic group containing from 6 to 12 carbons in the ring portion, such as phenyl, biphenyl, naphthyl, substituted phenyl, substituted biphenyl or substituted naphthyl. Phenyl and substituted phenyl are the more preferred aryl groups. The term "aryl" also includes heteroaryl.

The terms "carboxylic acid group", "carboxylic" or "carboxyl" denote the monovalent radical -C(O)OH. Depending upon the pH conditions, the monovalent radical can be in the form $-C(O)O^- Q^+$ wherein $Q^+$ is a cation (e.g., sodium), or two of the monovalent radicals in close proximity can bond with a divalent cation $Q^{2+}$ (e.g., calcium, magnesium), or a combination of these monovalent radicals and -C(O)OH are present.

The term "cycloalkyl" as used herein denotes optionally an optionally substituted cyclic saturated monovalent bridged or non-bridged hydrocarbon radical containing from three to eight carbon atoms in one ring and up to 20 carbon atoms in a multiple ring group. Exemplary unsubstituted cycloalkyl groups include cyclopropyl, cyclobutyl, cyclopentyl, cyclohexyl, cycloheptyl, cyclooctyl, adamantyl, norbornyl, and the like.

The term "-ene" as used as a suffix as part of another group denotes a bivalent radical in which a hydrogen atom is removed from each of two terminal carbons of the group, or if the group is cyclic, from each of two different carbon atoms in the ring. For example, alkylene denotes a bivalent alkyl group such as methylene ($-CH_2-$) or ethylene ($-CH_2CH_2-$), and arylene denotes a bivalent aryl group such as o-phenylene, m-phenylene, or p-phenylene.

The term "ether moiety" as used herein represents a bivalent (i.e., difunctional) group including at least one ether linkage (i.e., -O-). For example, in Formulae 3 or 33 as defined herein, the ether moiety can be $-R_AOR_B-$ or $-R_AOR_COR_B-$ wherein $R_A$, $R_B$ and $R_C$ are independently alkylene.

The term "heteroaryl," as used herein alone or as part of another group, denotes an optionally substituted monovalent monocyclic or bicyclic aromatic radical of 5 to 10 ring atoms, where one or more, preferably one, two, or three, ring atoms are heteroatoms independently selected from N, O, and S, and the remaining ring atoms are carbon. Exemplary heteroaryl moieties include benzofuranyl, benzo[d]thiazolyl, isoquinolinyl, quinolinyl, thiophenyl, imidazolyl, oxazolyl, quinolinyl, furanyl, thazolyl, pyridinyl, furyl, thienyl, pyridyl, oxazolyl, pyrrolyl, indolyl, quinolinyl, isoquinolinyl, and the like.

The term "heterocyclo," as used herein alone or as part of another group, denotes a saturated or unsaturated monovalent monocyclic group of 4 to 8 ring atoms, in which one or two ring atoms are heteroatom(s), independently selected from N, O, and S, and the remaining ring atoms are carbon atoms. Additionally, the heterocyclic ring may be fused to a phenyl or heteroaryl ring, provided that the entire heterocyclic ring is not completely aromatic. Exemplary heterocyclo groups include the heteroaryl groups described above, pyrrolidino, piperidino, morpholino, piperazino, and the like.

The term "hydrocarbon" as used herein describes a compound or radical consisting exclusively of the elements carbon and hydrogen.

The term "phosphonic" or "phosphonyl" denotes the monovalent radical

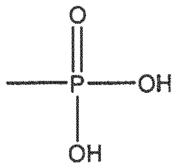.

The term "phosphoric" or "phosphoryl" denotes the monovalent radical

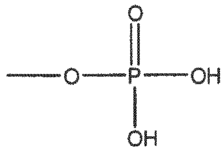.

The term "protected" as used herein as part of another group denotes a group that blocks reaction at the protected portion of a compound while being easily removed under conditions that are sufficiently mild so as not to disturb other substituents of the compound. For example, a protected carboxylic acid group-C(O)$OP_g$ or a protected phosphoric acid group - OP(O)(OH)$OP_g$ or a protected phosphonic acid group

-P(O)(OH)$OP_g$ each have a protecting group $P_g$ associated with the oxygen of the acid group wherein $P_g$ can be alkyl (e.g., methyl, ethyl, n-propyl, i-propyl, n-butyl, i-butyl, s-butyl, t-butyl, n-pentyl, i-pentyl, s-pentyl, t-pentyl, and the like), benzyl, silyl (e.g., trimethylsilyl (TMS), triethylsilyl (TES), triisopropylsilyl (TIPS), triphenylsilyl (TPS), t-butyldimethylsilyl (TBDMS), t-butyldiphenylsilyl (TBDPS) and the like. A variety of protecting groups and the synthesis thereof may be found in "Protective Groups in Organic Synthesis" by T.W. Greene and P.G.M. Wuts, John Wiley & Sons, 1999. When the term "protected" introduces a list of possible protected groups, it is intended that the term apply to every member of that group. That is, the phrase "protected carboxylic, phosphonic or phosphoric" is to be interpreted as "protected carboxylic, protected phosphonic or protected phosphoric." Likewise, the phrase "optionally protected carboxylic, phosphoric or phosphonic" is to be interpreted as "optionally protected carboxylic, optionally protected phosphonic or optionally protected phosphoric."

The term "substituted" as in "substituted aryl," "substituted alkyl," and the like, means that in the group in question (i.e., the alkyl, aryl or other group that follows the term), at least one hydrogen atom bound to a carbon atom is replaced with one or more substituent groups such as hydroxy (-OH), alkylthio, phosphino, amido

(-CON($R_A$)($R_B$), wherein $R_A$ and $R_B$ are independently hydrogen, alkyl, or aryl), amino(-N($R_A$)($R_B$), wherein $R_A$ and $R_B$ are independently hydrogen, alkyl or aryl), halo (fluoro, chloro, bromo, or iodo), silyl, nitro (-$NO_2$), an ether (-$OR_A$ wherein $R_A$ is alkyl or aryl), an ester (-OC(O)$R_A$ wherein $R_A$ is alkyl or aryl), keto (-C(O)$R_A$ wherein $R_A$ is alkyl or aryl), heterocyclo, and the like. When the term "substituted" introduces a list of possible substituted groups, it is intended that the term apply to every member of that group. That is, the phrase "optionally substituted alkyl or aryl" is to be interpreted as "optionally substituted alkyl or optionally substituted aryl."

Having described the invention in detail, it will be apparent that modifications and variations are possible without departing from the scope of the invention defined in the appended claims.

EXAMPLES

The following non-limiting examples are provided to further illustrate the present invention.

***Materials for Examples 1-5.*** Methyl 2-fluoroacrylate (MeFA; SynQuest Labs) contained 0.2 wt% hydroquinone and was vacuum distilled before use. Divinylbenzene (DVB; Aldrich) was technical grade, 80%, mixture of isomers. 1,7-octadiene (ODE 98%; Aldrich), lauroyl peroxide (LPO 99%; ACROS Organics), polyvinyl alcohol (PVA typical molecular weight 85,000-146,000, 87-89% hydrolyzed; Aldrich) , sodium chloride (NaCl; Aldrich), sodium phosphate dibasic heptahydrate ($Na_2HPO_4 \cdot 7H_2O$; Aldrich), and sodium phosphate monobasic monohydrate ($NaH_2PO_4 \cdot H_2O$; Aldrich) were used as received.

Example 1: DVB as crosslinking monomer

The polymerization was carried out in a 1 L three-neck Morton-type round bottom flask equipped with an overhead mechanical stirrer with a Teflon paddle and a water condenser. An organic phase was prepared by mixing MeFA (54g), DVB (6g) and LPO (0.6g), and an aqueous phase was prepared by dissolving PVA (3g) and NaCl (11.25g) in water (285.75g). The organic and aqueous phases were then mixed in the flask and stirred at 300 rpm under nitrogen. The flask was immersed in a 70°C oil bath for 3 hours, and cooled to room temperature. The internal temperature during the reaction

was about 65°C. The solid product was washed with water and collected by decanting off supernatant solution. The white solid was freeze-dried, affording dry solid polyMeFA particles (or beads) (56.15g, 94%).

Hydrolysis was carried out in the same setup as for the polymerization. PolyMeFA particles (48.93g) from above were suspended in KOH solution (500g, 10wt.%) and stirred at 300 rpm. The mixture was heated in a 95°C oil bath for 20 hours and cooled to room temperature. The solid product was washed with water and collected by decanting off the supernatant solution. After freeze-drying, poly fluoroacrylic acid (polyFAA) particles (48.54g, 82%) were obtained. These particles were in the form of beads.

Example 2: Polymer synthesis using two crosslinking monomers

Multiple suspension polymerizations were carried out in a manner substantially similar to Example 1. The synthesis conditions and results are summarized in Table 3. Compared to Example 1, the addition of ODE as a second crosslinker in all ratios tested increased the yield after the hydrolysis step. Therefore the overall yield for polyFAA bead synthesis was improved to a level of greater than 90%.

TABLE 3. Synthesis conditions and selected properties

| Exp # | Aqueous Phase | | | | Organic Phase | | | Yield | | | Swelling Ratio | BC mmol/g |
|---|---|---|---|---|---|---|---|---|---|---|---|---|
| | Buffer | NaCl | pH polymz | H after polymz | MeFA wt.% | DVB wt.% | ODE wt.% | Susp. | Hydro. | Overall | | |
| Comp 1 | no | 3.75% | nm | 4.00 | 95 | 5 | 0 | 98% | 64% | 63% | 2.66 | 9.59 |
| Comp 2 | no | 3.75% | nm | 3.90 | 90 | 10 | 0 | 94% | 82% | 77% | 1.52 | 8.72 |
| Comp 3 | no | 3.75% | nm | 3.50 | 80 | 20 | 0 | 89% | 90% | 80% | 1.01 | 5.96 |
| Ex 789 | no | 3.75% | 5.10 | 3.50 | 90 | 8 | 2 | 95% | 100% | 95% | 1.58 | 8.70 |
| Ex 792 | 0.25% | 3.50% | 8.30 | 3.95 | | | | 94% | 100% | 94% | 1.49 | 8.76 |
| Ex 793 | 0.50% | 3.25% | 8.45 | 5.28 | | | | 94% | 95% | 89% | 1.44 | 8.62 |
| Ex 808 | 0.50% | 3.25% | nm | nm | | | | nm | nm | 92% | nm | 8.76 |
| Ex 811 | 0.50% | 3.25% | 7.25 | 5.05 | | | | nm | nm | 93% | nm | nm |
| Ex 815 | 0.75% | 2.50% | 7.24 | 5.26 | | | | nm | nm | 88% | nm | nm |
| Ex 816 | 0.75% | 2.50% | 7.16 | 4.62 | | | | 87% | 94% | 82% | nm | nm |
| Ex 814 | 1.00% | 0.00% | 7.66 | 5.51 | | | | aggregates | | | nm | nm |
| Ex 794 | no | 3.75% | 5.78 | nm | 90 | 5 | 5 | 95% | 100% | 95% | 1.57 | 9.26 |
| Ex 803 | no | 3.75% | 5.17 | 3.94 | | | | nm | nm | 95% | 1.44 | 8.70 |
| Ex 805 | 0.50% | 3.25% | 7.00 | 5.23 | | | | nm | nm | 95% | 1.51 | 8.70 |
| Ex 812 | 0.50% | 3.25% | 7.29 | 5.21 | | | | nm | nm | 95% | nm | nm |
| Ex 801 | no | 3.75% | 5.18 | 3.11 | 90 | 2 | 8 | 93% | 100% | 93% | 1.80 | 9.05 |
| Ex 806 | 0.50% | 3.25% | 7.00 | 5.44 | | | | nm | nm | 94% | 1.67 | 8.21 |
| Ex 796 | no | 3.75% | nm | nm | 90 | 0 | 10 | 87% | 98% | 85% | 2.34 | 9.87 |
| Ex 800 | 0.50% | 3.25% | 8.24 | 4.93 | 90 | 0 | 10 | 92% | 95% | 87% | 2.51 | 9.46 |
| Ex 802 | 0.50% | 3.25% | 8.27 | 5.44 | 85 | 0 | 15 | 88% | 95% | 84% | 2.33 | 8.98 |

Note: (1) buffer, $Na_2HPO_4$/$NaH_2PO_4$; (2) swelling ratio, measured using salt form; (3) BC, binding capacity, measured using H form in 100mM KOH solution; (4) In Ex 816, 200ppm $NaNO_2$ was added in aqueous phase; (5) nm, means not measured; (6) polymz means polymerization; (7) Susp. means suspension; (8) Hydro. means hydrolysis.

Examples 3-5: Synthesis of FAA beads with DVB/ODE

The polymers of examples 3-5 were prepared as follows. A polymerization was carried out in a 1 L three-neck Morton-type round bottom flask equipped with an overhead mechanical stirrer with a Teflon paddle and a water condenser. An organic phase was prepared by mixing MeFA, DVB, ODE and LPO (0.6g), and an aqueous phase was prepared by dissolving PVA (3g) and NaCl (11.25g) in water (285.75g). The organic and aqueous phases were then mixed in the flask,

and stirred at 300 rpm under nitrogen. The flask was immersed in a 70°C oil bath for 5 hours, and cooled to room temperature. The internal temperature during reaction was about 65°C. The solid product was washed with water and collected by filtration. The white solid was freeze-dried, affording dry solid polyMeFA beads.

Hydrolysis was carried out in the same setup as for the polymerization. PolyMeFA beads from the polymerization reaction were suspended in a NaOH solution (400g, 10wt%) and stirred at 200 rpm. The mixture was heated in a 95°C oil bath for 20 hours and cooled to room temperature. The solid product was washed with water and collected by filtration. After freeze-drying, polyFAA beads were obtained. The synthesis conditions and selected properties are summarized below:

| Ex m# | Organic Phase | | | | | | Hydrolysis | Yield | |
|---|---|---|---|---|---|---|---|---|---|
| | MeF A (g) | DV B | OD E (g) | MeF A wt.% | DV B wt. | OD E wt. | polyMeFA (g) | Susp. (g), % | Hydro. (g), % |
| 3 | 54 | 4.8 | 1.2 | 90 | 8 | 2 | 40.26 | 56.74, 95% | 43.16, 100% |
| 4 | 54 | 3 | 3 | 90 | 5 | 5 | 39.17 | 56.91, 95% | 42.31, 100% |
| 5 | 54 | 1.2 | 4.8 | 90 | 2 | 8 | 38.23 | 55.94, 93% | 41.62, 100% |

The calcium form of the polyFAA beads of Example 4 was prepared by exposing the (sodium 2-fluoroacrylate)-divinylbenzene-1,7-octadiene copolymer to an excess of aqueous calcium chloride solution to yield insoluble cross-linked (calcium 2-fluoroacrylate)-divinylbenzene-1,7-octadiene copolymer. After the calcium ion exchange, the Ca(polyFAA) final product was washed with ethanol and water.

Example 6: Preparation of compositions with Ca(polyFAA) and stabilizing polyol and stability testing of such compositions during storage

Composition Preparation: To a 500 mL 3-necked round bottom flask equipped with a magnetic stirrer and nitrogen inlet adapter was charged D-sorbitol (60g ; 0.3 moles) followed by 240g of water. The mixture was stirred until a clear solution was obtained. Ca(polyFAA) (30g) prepared by the process described in Example 4 was added in one portion to the sorbitol solution and the resultant slurry was stirred at ambient temperature (20-25°C) for three hours. The solids were filtered off and dried under reduced pressure to the desired water content. The solids (35.1g) were analyzed for sugar alcohol content, loss on drying (LOD), and calcium content. This same sample preparation technique was used for the other compositions, with the specific details of varying D-sorbitol concentrations, times of mixing and drying as set forth in Table 4.

The samples prepared as discussed above were placed in storage at the temperatures and times listed in Tables 5-14. For the samples stored at 5°C and ambient temperature, the samples were transferred to a vial, which was placed in a Sure-Seal bag and sealed, and then placed in a second Sure-Seal bag with a desiccant (calcium sulfate) in the second bag, which was also sealed. For the samples at higher temperatures, the samples were placed in vials and stored at the stated temperatures. At the specified time (1 week, 3 weeks, 5 weeks, 7 weeks, etc.), aliquots of the samples were removed from storage and tested for their weight, moisture content, LOD and free inorganic fluoride. These tests were carried out as detailed in the specification above. Fluoride concentrations shown in Tables 5-14 below have been corrected for water and polyol weight.

TABLE 4.

| Sample No. | SORBITOL CONCENTRATION USED FOR LOADING (W/W %) | SORBITOL LOADING (W/W %) | MIXING TIME | DRYING METHOD |
|---|---|---|---|---|
| 6A | 2 | 3.1 | 1.5h | lyophilization |
| 6B | 5 | 7.3 | 3h | lyophilization |
| 6C | 10 | 12.3 | 3h | lyophilization |
| 6D | 20 | 17.2 | 3h | lyophilization |
| 6E | 20 | 18.3 | 3h | air dried under vacuum |
| 6F | 20 | 18.3 | 3h | lyophilization |
| 6G | 30 | 22.5 | 1.5h | air dried under vacuum |

(continued)

| Sample No. | SORBITOL CONCENTRATION USED FOR LOADING (W/W %) | SORBITOL LOADING (W/W %) | MIXING TIME | DRYING METHOD |
|---|---|---|---|---|
| 6H | 30 | 22.5 | 3h | lyophilization |
| 6I | 45 | 24.9 | 3h | air dried under vacuum |
| 6J | 45 | 24.9 | 1.5h | lyophilization |

TABLE 5. Sample 6A

| TIME POINT | STORAGE CONDITION S | Sample Weight (g) | Moisture Content (%) | Sample Dry Weight (g) | Fluoride Reading (ppm) | Fluoride Conc. (ug/g) |
|---|---|---|---|---|---|---|
| T = 0 | 5-8°C | 0.498 | 4.80 | 0.474 | 2.79 | 607 |
| | 20-25°C | | | | | |
| | 40°C | | | | | |
| T= 1 WEEK | 5-8°C | 0.496 | 5.72 | 0.468 | 3.04 | 671 |
| | 20-25°C | 0.504 | 6.00 | 0.474 | 4.53 | 987 |
| | 40°C | 0.545 | 5.48 | 0.515 | 9.79 | 1961 |
| T=3 WEEKS | 5-8°C | 0.508 | 4.99 | 0.483 | 3.53 | 754 |
| | 20-25°C | 0.505 | 4.97 | 0.480 | 6.28 | 1351 |
| | 40°C | n/a | n/a | n/a | n/a | n/a |
| T=5 WEEKS | 5-8°C | 0.315 | 8.06 | 0.290 | 4.69 | 1003 |
| | 20-25°C | 0.317 | 6.03 | 0.298 | 7.33 | 1523 |
| | 40°C | n/a | n/a | n/a | n/a | n/a |
| T=7 WEEKS | 5-8°C | 0.513 | 8.06 | 0.472 | 4.6 | 1006 |
| | 20-25°C | 0.513 | 6.03 | 0.482 | 7.63 | 607 |
| | 40°C | n/a | n/a | n/a | n/a | n/a |

TABLE 6. Sample 6B

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C | 0.514 | 5.34 | 0.487 | 1.74 | 385 |
| | 20-25°C | | | | | |
| | 40°C | | | | | |
| T = 1 WEEK | 5-8°C | 0.537 | 6.31 | 0.503 | 1.99 | 427 |
| | 20-25°C | 0.518 | 6.57 | 0.484 | 3.08 | 686 |
| | 40°C | 0.52 | 7.03 | 0.483 | 7.03 | 1569 |
| T = 3 WEEKS | 5-8°C | 0.513 | 5.21 | 0.486 | 2.15 | 477 |
| | 20-25°C | 0.501 | 6.07 | 0.471 | 4.3 | 986 |
| | 40°C | n/a | n/a | n/a | n/a | n/a |
| T = 5 WEEKS | 5-8°C | 0.5031 | 5.97 | 0.473 | 2.77 | 632 |
| | 20-25°C | 0.5092 | 6.79 | 0.475 | 5.17 | 1175 |
| | 40°C | n/a | n/a | n/a | n/a | n/a |

(continued)

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 7 WEEKS | 5-8°C | 0.507 | 5.97 | 0.477 | 2.76 | 625 |
| | 20-25°C | 0.508 | 6.79 | 0.474 | 5.67 | 1291 |
| | 40°C | n/a | n/a | n/a | n/a | n/a |
| T = 9 WEEKS | 5-8°C | 0.504 | 5.97 | 0.474 | 2.81 | 640 |
| | 20-25°C | n/a | n/a | n/a | n/a | n/a |
| | 40°C | n/a | n/a | n/a | n/a | n/a |

TABLE 7. Sample 6C

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C | 0.512 | 5.98 | 0.481 | 1.1 | 228.7 |
| | 20-25°C | | | | | |
| | 40°C | | | | | |
| T = 1 WEEK | 5-8°C | 0.576 | 5.98 | 0.542 | 1.28 | 269 |
| | 20-25°C | 0.506 | 5.71 | 0.477 | 1.88 | 449 |
| | 40°C | 0.52 | 5.63 | 0.491 | 4.61 | 1071 |
| T = 3 WEEKS | 5-8°C | 0.527 | 6.86 | 0.491 | 1.3 | 302 |
| | 20-25°C | 0.512 | 6.56 | 0.478 | 2.46 | 586 |
| | 40°C | 0.506 | 6.74 | 0.472 | 6.44 | 1556 |
| T = 5 WEEKS | 5-8°C | 0.5104 | 7.19 | 0.474 | 1.80 | 433 |
| | 20-25°C | 0.5118 | 6.95 | 0.476 | 3.29 | 788 |
| | 40°C | n/a | n/a | n/a | n/a | n/a |
| T = 7 WEEKS | 5-8°C | 0.513 | 7.19 | 0.476 | 1.75 | 420 |
| | 20-25°C | 0.521 | 6.95 | 0.485 | 3.4 | 799 |
| | 40°C | 0.508 | 6.74 | 0.474 | 7.84 | 1887 |
| T = 9 WEEKS | 5-8°C | 0.527 | 7.19 | 0.489 | 1.81 | 422 |
| | 20-25°C | n/a | n/a | n/a | n/a | n/a |
| | 40°C | n/a | n/a | n/a | n/a | n/a |

TABLE 8. Sample 6D

| TIME POINT | STORAGE CONDITION S | Sample Weight (g) | Moisture Content (%) | Sample Dry Weight (g) | Fluoride Reading (ppm) | Fluoride Conc. (ug/g) |
|---|---|---|---|---|---|---|
| T = 0 | 5-8°C | 0.517 | 7.41 | 0.479 | 0.5 | 126 |
| | 20-25°C | | | | | |
| | 40°C | | | | | |

(continued)

| TIME POINT | STORAGE CONDITION S | Sample Weight (g) | Moisture Content (%) | Sample Dry Weight (g) | Fluoride Reading (ppm) | Fluoride Conc. (ug/g) |
|---|---|---|---|---|---|---|
| T = 1 WEEK | 5-8°C | 0.503 | 7.52 | 0.465 | 0.649 | 169 |
| | 20-25°C | 0.534 | 8.2 | 0.490 | 1.03 | 254 |
| | 40°C | 0.562 | 6.95 | 0.523 | 2.55 | 589 |
| T = 3 WEEKS | 5-8°C | 0.525 | 6.73 | 0.490 | 0.659 | 163 |
| | 20-25°C | 0.524 | 6.91 | 0.488 | 1.2 | 297 |
| | 40°C | 0.514 | 6.63 | 0.480 | 2.75 | 692 |
| T = 5 WEEKS | 5-8°C | 0.5157 | 7.08 | 0.479 | 0.819 | 207 |
| | 20-25°C | 0.5062 | 7.56 | 0.468 | 1.47 | 379 |
| | 40°C | 0.5416 | 8.8 | 0.494 | 4.15 | 1014 |
| T = 7 WEEKS | 5-8°C | 0.525 | 7.08 | 0.488 | 0.809 | 200 |
| | 20-25°C | 0.519 | 7.56 | 0.480 | 1.65 | 415 |
| | 40°C | 0.524 | 8.8 | 0.478 | 4.56 | 1152 |
| T = 9 WEEKS | 5-8°C | 0.513 | 7.56 | 0.474 | 0.734 | 187 |
| | 20-25°C | n/a | n/a | n/a | n/a | n/a |
| | 40°C | n/a | n/a | n/a | n/a | n/a |

TABLE 9. Sample 6E

| TIME POINT | STORAGE CONDITIONS | Sample Wt (g) | Moisture Content (%) | Dry Weight (g) | Fluoride Reading (ppm) | Fluoride Conc. (ug/g) |
|---|---|---|---|---|---|---|
| T = 0 | 5-8°C | 0.55 | 17.00 | 0.457 | 0.05 | 13 |
| | 20-25°C | | | | | |
| | 40°C | | | | | |
| T= 2 WEEKS | 5-8°C | 0.504 | 16.53 | 0.421 | 0.04 | 12 |
| | 20-25°C | 0.507 | 16.30 | 0.424 | 0.08 | 23 |
| | 40°C | 0.507 | 16.20 | 0.425 | 0.75 | 217 |
| T=4 WEEKS | 5-8°C | 0.519 | 16.60 | 0.433 | 0.04 | 11 |
| | 20-25°C | 0.508 | 15.60 | 0.429 | 0.09 | 26 |
| | 40°C | 0.513 | 13.50 | 0.444 | 0.95 | 262 |
| T=6 WEEKS | 5-8°C | 0.506 | 15.34 | 0.428 | 0.03 | 9 |
| | 20-25°C | 0.511 | 15.57 | 0.431 | 0.05 | 15 |
| | 40°C | 0.507 | 14.72 | 0.432 | 1.35 | 382 |
| T =8 WEEKS | 5-8°C | 0.514 | 16.81 | 0.428 | 0.04 | 11 |
| | 20-25°C | 0.5 | 16.09 | 0.420 | 0.06 | 17 |
| | 40°C | 0.511 | 14.28 | 0.438 | 1.36 | 379 |
| T=9 WEEKS | 5-8°C | 0.509 | 17.11 | 0.422 | 0.05 | 15 |
| | 20-25°C | 0.502 | 16.00 | 0.422 | 0.28 | 81 |
| | 40°C | 0.525 | 15.60 | 0.443 | 2.03 | 561 |

(continued)

| TIME POINT | STORAGE CONDITIONS | Sample Wt (g) | Moisture Content (%) | Dry Weight (g) | Fluoride Reading (ppm) | Fluoride Conc. (ug/g) |
|---|---|---|---|---|---|---|
| T =10 WEEKS | 5-8°C | 0.514 | 17.19 | 0.426 | 0.05 | 15 |
| | 20-25°C | 0.524 | 15.56 | 0.442 | 0.31 | 86 |
| | 40°C | 0.502 | 15.10 | 0.426 | 2.2 | 632 |
| T =12 WEEKS | 5-8°C | 0.503 | 17.20 | 0.416 | 0.26 | 7 |
| | 20-25°C | 0.505 | 15.60 | 0.426 | 6.3 | 181 |
| | 40°C | 0.514 | 15.10 | 0.436 | 2.46 | 690 |

TABLE 10. Sample 6F

| TIME POINT | STORAGE CONDITIONS | Sample Wt (g) | Moisture Content (%) | Sample Dry Weight (g) | Fluoride Reading (ppm) | Fluoride Conc. (ug/g) |
|---|---|---|---|---|---|---|
| T = 0 | 5-8°C | 0.519 | 6.85 | 0.483 | 0.16 | 39 |
| | 20-25°C | | | | | |
| | 40°C | | | | | |
| T= 2 WEEKS | 5-8°C | 0.504 | 8.08 | 0.463 | 0.15 | 39 |
| | 20-25°C | 0.557 | 7.78 | 0.514 | 0.58 | 138 |
| | 40°C | 0.516 | 9.55 | 0.467 | 1.40 | 367 |
| T=4 WEEKS | 5-8°C | 0.533 | 8.33 | 0.489 | 0.16 | 40 |
| | 20-25°C | 0.540 | 7.40 | 0.500 | 0.56 | 137 |
| | 40°C | 0.510 | 7.50 | 0.472 | 2.25 | 584 |
| T = 6 WEEKS | 5-8°C | 0.507 | 7.74 | 0.468 | 0.09 | 23 |
| | 20-25°C | 0.501 | 7.14 | 0.465 | 0.55 | 144 |
| | 40°C | 0.504 | 7.59 | 0.466 | 2.39 | 628 |
| T =8 WEEKS | 5-8°C | 0.503 | 7.88 | 0.463 | 0.08 | 21 |
| | 20-25°C | 0.502 | 7.54 | 0.464 | 0.53 | 140 |
| | 40°C | 0.510 | 8.59 | 0.466 | 2.36 | 619 |
| T = 9 WEEKS | 5-8°C | 0.509 | 7.49 | 0.471 | 0.33 | 86 |
| | 20-25°C | 0.509 | 7.57 | 0.470 | 1.05 | 273 |
| | 40°C | 0.492 | 8.04 | 0.452 | 2.61 | 706 |
| T =10 WEEKS | 5-8°C | 0.503 | 7.49 | 0.465 | 0.33 | 87 |
| | 20-25°C | 0.52 | 7.57 | 0.481 | 1.12 | 285 |
| | 40°C | 0.504 | 8.04 | 0.463 | 3.03 | 800 |
| T =12 WEEKS | 5-8°C | 0.502 | 7.49 | 0.464 | 2.48 | 65 |
| | 20-25°C | 0.504 | 7.57 | 0.466 | 6.82 | 179 |
| | 40°C | 0.498 | 8.04 | 0.458 | 4.02 | 1075 |

TABLE 11. Sample 6G

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C<br>20-25°C<br>40°C | 0.588 | 17.5 | 0.485 | 0.06 | 15 |
| T= 2 WEEKS | 5-8°C | 0.501 | 16.7 | 0.417 | 0.05 | 15 |
| | 20-25°C | 0.532 | 16.6 | 0.444 | 0.07 | 21 |
| | 40°C | 0.509 | 15.8 | 0.429 | 0.54 | 161 |
| T=4 WEEKS | 5-8°C | 0.506 | 16.1 | 0.425 | 0.02 | 6 |
| | 20-25°C | 0.505 | 15.2 | 0.428 | 0.03 | 9 |
| | 40°C | 0.523 | 15.1 | 0.444 | 0.613 | 178 |
| T = 6 WEEKS | 5-8°C | 0.502 | 15.62 | 0.424 | 0.02 | 6 |
| | 20-25°C | 0.501 | 14.39 | 0.429 | 0.04 | 12 |
| | 40°C | 0.517 | 14.28 | 0.443 | 1.11 | 323 |
| T = 8 WEEKS | 5-8°C | 0.515 | 16.32 | 0.431 | 0.04 | 12 |
| | 20-25°C | 0.512 | 15.95 | 0.430 | 0.04 | 12 |
| | 40°C | 0.508 | 14.46 | 0.435 | 1.09 | 324 |
| T=9 WEEKS | 5-8°C | 0.5 | 16.83 | 0.416 | 0.03 | 9 |
| | 20-25°C | 0.51 | 15.41 | 0.431 | 0.206 | 62 |
| | 40°C | 0.503 | 15.34 | 0.426 | 1.43 | 434 |
| T =10 WEEKS | 5-8°C | 0.506 | 16.36 | 0.423 | 0.04 | 12 |
| | 20-25°C | 0.508 | 15.82 | 0.428 | 0.22 | 66 |
| | 40°C | 0.507 | 15.2 | 0.430 | 1.67 | 501 |
| T =12 WEEKS | 5-8°C | 0.504 | 16.36 | 0.422 | 0.26 | 8 |
| | 20-25°C | 0.501 | 15.82 | 0.422 | 1.8 | 55 |
| | 40°C | 0.508 | 15.2 | 0.431 | 1.94 | 581 |

TABLE 12. Sample 6H

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C<br>20-25°C<br>40°C | 0.511 | 7.82 | 0.471 | 0.19 | 50 |
| T= 2 WEEKS | 5-8°C | 0.510 | 7.07 | 0.474 | 0.17 | 46 |
| | 20-25°C | 0.544 | 7.18 | 0.505 | 0.40 | 102 |
| | 40°C | 0.502 | 8.16 | 0.461 | 1.10 | 308 |
| T=4 WEEKS | 5-8°C | 0.538 | 7.2 | 0.499 | 0.20 | 52 |
| | 20-25°C | 0.508 | 6.21 | 0.476 | 0.38 | 103 |
| | 40°C | 0.501 | 7.47 | 0.464 | 2.03 | 565 |

(continued)

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T=6 WEEKS | 5-8°C | 0.509 | 6.38 | 0.477 | 0.16 | 44 |
| | 20-25°C | 0.521 | 6.91 | 0.485 | 0.39 | 103 |
| | 40°C | 0.500 | 7.08 | 0.465 | 2.04 | 566 |
| T = 8 WEEKS | 5-8°C | 0.523 | 7.16 | 0.486 | 0.14 | 37 |
| | 20-25°C | 0.530 | 7.31 | 0.491 | 0.31 | 81 |
| | 40°C | 0.500 | 7.67 | 0.462 | 1.89 | 528 |
| T=9 WEEKS | 5-8°C | 0.531 | 7.89 | 0.489 | 0.35 | 92 |
| | 20-25°C | 0.501 | 7.8 | 0.462 | 0.79 | 221 |
| | 40°C | 0.518 | 8.19 | 0.476 | 2.41 | 654 |
| T =10 WEEKS | 5-8°C | 0.510 | 7.89 | 0.470 | 0.33 | 90 |
| | 20-25°C | 0.516 | 7.80 | 0.476 | 0.88 | 239 |
| | 40°C | 0.501 | 8.19 | 0.460 | 2.58 | 724 |
| T =12 WEEKS | 5-8°C | 0.504 | 7.89 | 0.464 | 2.03 | 57 |
| | 20-25°C | 0.502 | 7.80 | 0.463 | 5.75 | 160 |
| | 40°C | 0.495 | 8.19 | 0.454 | 3.20 | 908 |

TABLE 13. Sample 61

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C | 0.502 | 16.1 | 0.421 | <0.07 | <15 |
| | 20-25°C | | | | | |
| | 40°C | | | | | |
| T= 2 WEEKS | 5-8°C | 0.520 | 16.9 | 0.432 | 0.03 | 9 |
| | 20-25°C | 0.510 | 15.8 | 0.429 | 0.06 | 19 |
| | 40°C | 0.510 | 14.5 | 0.436 | 0.70 | 214 |
| T=4 WEEKS | 5-8°C | 0.505 | 16.2 | 0.423 | 0.04 | 12 |
| | 20-25°C | 0.519 | 14.7 | 0.443 | 0.03 | 9 |
| | 40°C | 0.507 | 14.5 | 0.433 | 0.91 | 280 |
| T=6 WEEKS | 5-8°C | 0.513 | 16.8 | 0.427 | 0.02 | 7 |
| | 20-25°C | 0.504 | 14.8 | 0.429 | 0.03 | 9 |
| | 40°C | 0.554 | 14.1 | 0.476 | 1.09 | 305 |
| T = 8 WEEKS | 5-8°C | 0.511 | 16.09 | 0.429 | 0.03 | 9 |
| | 20-25°C | 0.505 | 15.58 | 0.426 | 0.03 | 9 |
| | 40°C | 0.554 | 14.46 | 0.474 | 1.13 | 317 |
| T=9 WEEKS | 5-8°C | 0.506 | 16.69 | 0.422 | 0.04 | 12 |
| | 20-25°C | 0.516 | 15.49 | 0.436 | 0.22 | 67 |
| | 40°C | 0.526 | 15.07 | 0.447 | 1.75 | 522 |

(continued)

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T =10 WEEKS | 5-8°C | 0.509 | 16.69 | 0.424 | 0.04 | 12 |
| | 20-25°C | 0.505 | 15.49 | 0.427 | 0.23 | 72 |
| | 40°C | 0.517 | 15.07 | 0.439 | 1.74 | 527 |
| T =12 WEEKS | 5-8°C | 0.503 | 16.69 | 0.419 | 0.314 | 9 |
| | 20-25°C | 0.501 | 15.49 | 0.423 | 1.76 | 56 |
| | 40°C | 0.517 | 15.07 | 0.439 | 2.22 | 674 |

TABLE 14. Sample 6J

| TIME POINT | STORAGE CONDITION S | Sample Weight (g) | Moisture Content (%) | Sample Dry Weight (g) | Fluoride Reading (ppm) | Fluoride Conc (ug/g) |
|---|---|---|---|---|---|---|
| T = 0 | 5-8°C | 0.563 | 8.59 | 0.515 | 0.13 | 33 |
| | 20-25°C | | | | | |
| | 40°C | | | | | |
| T= 2 WEEKS | 5-8°C | 0.545 | 7.60 | 0.504 | 0.12 | 32 |
| | 20-25°C | 0.520 | 7.35 | 0.482 | 0.25 | 69 |
| | 40°C | 0.501 | 8.21 | 0.460 | 0.66 | 192 |
| T=4 WEEKS | 5-8°C | 0.513 | 7.22 | 0.476 | 0.11 | 31 |
| | 20-25°C | 0.526 | 7.83 | 0.485 | 0.22 | 60 |
| | 40°C | 0.516 | 7.83 | 0.476 | 0.91 | 254 |
| T=6 WEEKS | 5-8°C | 0.519 | 7.93 | 0.478 | 0.09 | 25 |
| | 20-25°C | 0.503 | 8.00 | 0.463 | 0.21 | 60 |
| | 40°C | 0.511 | 7.80 | 0.471 | 0.94 | 266 |
| T = 8 WEEKS | 5-8°C | 0.518 | 8.16 | 0.476 | 0.11 | 31 |
| | 20-25°C | 0.532 | 7.91 | 0.490 | 0.22 | 60 |
| | 40°C | 0.509 | 8.11 | 0.468 | 0.97 | 276 |
| T=9 WEEKS | 5-8°C | 0.510 | 9.19 | 0.463 | 0.19 | 55 |
| | 20-25°C | 0.535 | 8.44 | 0.490 | 0.62 | 168 |
| | 40°C | 0.511 | 8.07 | 0.470 | 1.86 | 527 |
| T =10 WEEKS | 5-8°C | 0.503 | 9.19 | 0.457 | 0.18 | 52 |
| | 20-25°C | 0.511 | 8.44 | 0.468 | 0.61 | 174 |
| | 40°C | 0.509 | 8.07 | 0.468 | 1.87 | 533 |
| T =12 WEEKS | 5-8°C | 0.500 | 9.19 | 0.454 | 1.45 | 43 |
| | 20-25°C | 0.510 | 8.44 | 0.467 | 4.57 | 130 |
| | 40°C | 0.518 | 8.07 | 0.476 | 2.36 | 660 |

Example 7: Potassium Binding Capacity of Polyol Stabilized FAA

*Materials.* The materials used were potassium chloride (Reagent Plus grade, ≥99%, Sigma #P4504 or equivalent); de-ionized water greater than 18 megaöhm resistivity; IC potassium standard (1,000 ppm, Alltech

Cat#37025 or equivalent); ion chromatography (IC) potassium standard, 1000 ppm from a secondary source (e.g. Fisher Scientific #CS-K2-2Y); and methanesulfonic acid (MSA, 99.5%; Aldrich #471356). The MSA was used to make the IC mobile phase if the apparatus used was unable to generate the mobile phase electrolytically.

*Preparation of 200 mM KCl solution.* Potassium chloride (14.91 g) was dissolved in 800 mL of water. A graduated cylinder was used and water was added to make a 1L solution. This solution was the 200 mM potassium chloride solution for the binding assay.

*QC and Linear Curve Preparation for IC Analysis.* Potassium standard solutions (100, 250, 500 ppm) for IC were prepared by diluting a stock 1000 ppm solution with distilled (DI) water. The QC check standard was obtained by diluting a second source certified 1000 ppm potassium standard with DI water to achieve 250 ppm concentration.

*Preparation of Sample Solution.* Two samples of Ca(polyFAA) prepared by the method of Example 4 (500 mg) were placed into separate screw top vials. Using the equation below, the amount of 200 mM KCl solution to add to the vial was calculated:

i.

$$\frac{\frac{M}{100}\times\left[100-S\times(1-\frac{W}{100})-W\right]}{20} \quad (mL)$$

where M is Ca(polyFAA) sample weight (mg), S is sorbitol content based on dry weight of Ca(polyFAA), and W is loss on drying (%). The calculated volume of 200 mM KCl solution was added to each vial using a 10 mL pipettor. The vials were capped tightly. Two blank vials containing 15 mL of 200 mM KCl solution were prepared. The vials were tumbled on a rotary tumbler for two hours at about 35 rpm. After two hours, the vials were removed from the tumbler. The contents were allowed to settle for 5 minutes. Each sample (2-10 mL) and a blank were filtered over a 0.45 micron filter. Each filtered sample was diluted 1:20 by adding 500 μL of each sample or blank to 9500 μL of water. The diluted filtrate was analyzed for potassium content using IC.

*Sample Analysis by IC.* If a 20mM MSA mobile phase could not be generated electrolytically, the 20 mM stock MSA mobile phase was made by diluting MSA in water. The IC had the following settings: injection volume: 5μL; flow rate: 1mL/min; column temperature: 35°C; sample compartment temperature: ambient; run time: 20min; and CD25 settings: current 88mA, cell temperature 35°C, autorange. Each blank and sample was injected twice.

The IC system used was a Dionex IC System 2000 equipped with AS50 autosampler, conductivity Detector CD25 and DS3 flow cell. The column used was a CS12A 250x4mm ID analytical column, Dionex #016181 coupled with a CG12A 50x4mm ID guard column (optional), Dionex#046074. The suppressor used was a Dionex CSRS-Ultra II (4mm) Suppressor, Dionex#061563. The software used for data acquisition was Dionex Chromeleon Chromatography Software. The eluent cartridge was a Dionex #058902 to generate the methanesulfonic acid (MSA) mobile phase electrolytically.

*Data Analysis.* The concentration of potassium was reported in mM. The equation below was used to calculate the binding capacity of each sample:

$$\text{Binding capacity (mmol/g)} = \left(c_{Blank} - c_{Sample}\right)$$

where $c_{Blank}$ is average concentration of potassium in the 20-fold diluted blank by IC analysis (mM), and $c_{sample}$ is average concentration of potassium in the 20-fold diluted sample solution by IC analysis (mM). The average of the duplicates was reported. The deviation of each individual value was a maximum of 10% from the mean. When a larger deviation was obtained, the assay was repeated.

*Results.* A Ca(polyFAA) sample prepared by the process described in Example 4 had a potassium binding capacity of 1.60 mmol/g. A similar Ca(polyFAA) sample was slurried with a 20 wt.%, 25 wt.%, 30 wt.% , and a 45 wt.% solution of D-sorbitol using the process described in Example 6. The potassium binding capacities for those stabilized Ca(polyFAA) samples are described in the Table 15.

TABLE 15.

| Ca(polyFAA) slurried with | Potassium Binding Capacity (mmol/g) |
|---|---|
| 20 wt.% sorbitol | 1.62 |
| 25 wt.% sorbitol | 1.67 |

(continued)

| Ca(polyFAA) slurried with | Potassium Binding Capacity (mmol/g) |
|---|---|
| 30 wt.% sorbitol | 1.61 |
| 45 wt.% sorbitol | 1.63 |

Example 8: Polymer Synthesis

*Materials.* Methyl 2-fluoroacrylate (MeFA; SynQuest Labs) contained 0.2 wt% hydroquinone and was vacuum distilled before use. Divinylbenzene (DVB; Aldrich) was technical grade, 80%, mixture of isomers. 1,7-octadiene (ODE 98%; Aldrich), lauroyl peroxide (LPO 99%; ACROS Organics), polyvinyl alcohol (PVA typical molecular weight 85,000-146,000, 87-89% hydrolyzed; Aldrich) , sodium chloride (NaCl; Aldrich), sodium phosphate dibasic heptahydrate ($Na2HPO4 \cdot 7H2O$; Aldrich), and sodium phosphate monobasic monohydrate ($NaH2PO4 \cdot H2O$; Aldrich) were used as received.

**Example 8A:**

In a 25L reactor with appropriate stirring and other equipment, a 180:10:10 weight ratio mixture of organic phase of monomers was prepared by mixing methyl 2-fluoroacrylate (~3 kg), 1,7-octadiene (~0.16 kg), and divinylbenzene (~0.16 kg). One part of lauroyl peroxide (~0.016 kg) was added as an initiator of the polymerization reaction. A stabilizing aqueous phase was prepared from water, polyvinyl alcohol, phosphates, sodium chloride, and sodium nitrite. The aqueous and monomer phases were mixed together under nitrogen at atmospheric pressure, while maintaining the temperature below 30°C. The reaction mixture was gradually heated while stirring continuously. Once the polymerization reaction has started, the temperature of the reaction mixture was allowed to rise to a maximum of 95°C. After completion of the polymerization reaction, the reaction mixture was cooled and the aqueous phase was removed. Water was added, the mixture was stirred, and the solid material was isolated by filtration. The solid was then washed with water to yield about 2.1 kg of a crosslinked (methyl 2-fluoroacrylate)-divinylbenzene-1,7-octadiene polymer.

The (methyl 2-fluoroacrylate)-divinylbenzene-1,7-octadiene copolymer was hydrolyzed with an excess of aqueous sodium hydroxide solution at 90 °C for 24 hours to yield (sodium 2-fluoroacrylate)-divinylbenzene-1,7-octadiene polymer. After hydrolysis, the solid was filtered and washed with water. The (sodium 2-fluoroacrylate)-divinylbenzene-1,7-octadiene polymer was exposed at room temperature to an excess of aqueous calcium chloride solution to yield insoluble cross-linked (calcium 2-fluoroacrylate)-divinylbenzene-1,7-octadiene polymer. After the calcium ion exchange, the product was washed with water and dried.

Beads produced by the process of Example 8A are shown in Figures 1A and 1B, which show that the beads generally have a rougher and more porous surface than beads made by the processes described in Examples 11-13.

**Example 8B:**

In a 2L reactor with appropriate stirring and other equipment, a 180:10:10 weight ratio mixture of organic phase of monomers was prepared by mixing methyl 2-fluoroacrylate (~0.24 kg), 1,7-octadiene (~0.0124 kg), and divinylbenzene (~0.0124 kg). One part of lauroyl peroxide (~0.0012 kg) was added as an initiator of the polymerization reaction. A stabilizing aqueous phase was prepared from water, polyvinyl alcohol, phosphates, sodium chloride, and sodium nitrite. The aqueous and monomer phases were mixed together under nitrogen at atmospheric pressure, while maintaining the temperature below 30°C. The reaction mixture was gradually heated while stirring continuously. Once the polymerization reaction has started, the temperature of the reaction mixture was allowed to rise to a maximum of 95°C. After completion of the polymerization reaction, the reaction mixture was cooled and the aqueous phase was removed. Water was added, the mixture was stirred, and the solid material was isolated by filtration, and then washed with water.

The polymerization reaction was repeated 5 more times, the polymer from the batches were combined together to yield about 1.7 kg of a crosslinked (methyl 2-fluoroacrylate)-divinylbenzene-1,7-octadiene polymer. The (methyl 2-fluoroacrylate)-divinylbenzene-1,7-octadiene polymer was hydrolyzed with an excess of aqueous sodium hydroxide and isopropanol solution at 65 °C for 24 hours to yield (sodium 2-fluoroacrylate)-divinylbenzene-1,7-octadiene polymer. After hydrolysis, the solid was filtered and washed with water. The (sodium 2-fluoroacrylate)-divinylbenzene-1,7-octadiene polymer was exposed at room temperature to an excess of aqueous calcium chloride solution to yield insoluble cross-linked (calcium 2-fluoroacrylate)-divinylbenzene-1,7-octadiene polymer. After the calcium ion exchange, the product was washed with water and dried.

**Example 8C:**

In a 20L reactor with appropriate stirring and other equipment, a 180:10:10 weight ratio mixture of organic phase of monomers was prepared by mixing methyl 2-fluoroacrylate (~2.4 kg), 1,7-octadiene (~0.124 kg), and divinylbenzene (~0.124 kg). One part of lauroyl peroxide (~0.0124 kg) was added as an initiator of the polymerization reaction. A stabilizing aqueous phase was prepared from water, polyvinyl alcohol, phosphates, sodium chloride, and sodium nitrite. The aqueous and monomer phases were mixed together under nitrogen at a pressure of 1.5 bar, while maintaining the temperature below 30°C. The reaction mixture was gradually heated while stirring continuously. Once the polymerization reaction started, the temperature of the reaction mixture was allowed to rise to a maximum of 95°C. After completion of the polymerization reaction, the reaction mixture was cooled and the aqueous phase was removed. Water was added, the mixture was stirred, and the solid material was isolated by filtration. The solid was then washed with water to yield about 1.7 kg of a crosslinked (methyl 2-fluoroacrylate)-divinylbenzene-1,7-octadiene polymer.

The (methyl 2-fluoroacrylate)-divinylbenzene-1,7-octadiene copolymer was hydrolyzed with an excess of aqueous sodium hydroxide solution at 85 °C for 24 hours to yield (sodium 2-fluoroacrylate)-divinylbenzene-1,7-octadiene polymer. After hydrolysis, the solid was filtered and washed with water. The (sodium 2-fluoroacrylate)-divinylbenzene-1,7-octadiene polymer was exposed at room temperature to an excess of aqueous calcium chloride solution to yield insoluble cross-linked (calcium 2-fluoroacrylate)-divinylbenzene-1,7-octadiene polymer. After the calcium ion exchange, the product was washed with toluene and dried using an azeotropic distillation.

**Example 8D:**

A stock aqueous solution of sodium chloride (NaCl; 4.95 g), water (157.08 g), polyvinylalcohol (1.65 g), $Na_2HPO_4 \cdot 7H_2O$ (1.40 g), $NaH_2PO_4 \cdot H_2O$ (0.09 g), and $NaNO_2$ (0.02 g) was prepared. A stock solution of the organic components that consisted of t-butyl-fluoroacrylate (30.00 g), divinylbenzene (1.19 g), octadiene (1.19 g), and lauroyl peroxide (0.24 g) was prepared. Components were weighed manually into a 500 mL 3-necked reaction flask with baffles, so that the weight (g) of each component matched the values as described above. The flask was fitted with an overhead stirrer, and a condenser. Nitrogen was blown over the reaction for 10 minutes and a blanket of nitrogen was maintained throughout the reaction. The stir rate was set to 180 rpm. The bath temperature was set to 70°C. After 12 hours the heat was increased to 85°C for 2 hours and the reaction was allowed to cool to room temperature. The beads were isolated from the reaction flask and were washed with isopropyl alcohol, ethanol and water. The poly($\alpha$-fluoroacrylate, t-butyl ester) beads were dried at room temperature under reduced pressure.

Into a 500 mL 3-necked reaction flask with baffles, was weighed 28.02 g of poly($\alpha$-fluoroacrylate, t-butyl ester), 84 g of concentrated hydrochloric acid (3 times the weight of bead, 3 moles of hydrochloric acid to 1 t-butyl-ester), and 84 g water (3 times bead). The flask was fitted with an overhead stirrer, and a condenser. Nitrogen was blown over the reaction for 10 minutes and a blanket of nitrogen was maintained throughout the reaction. The stir rate was set to 180 rpm. The bath temperature was set to 75°C. After 12 hours the heat turned off and the reaction was allowed to cool to room temperature. The beads were isolated from the reaction flask and were washed with isopropyl alcohol, ethanol and water. The proton-form beads were dried at room temperature under reduced pressure.

The proton-form beads were then placed in a glass column and washed with 1 N NaOH until the eluent pH was strongly alkaline and the appearance of the beads in the column was uniform. Then the beads were washed again with deionized water until the eluent pH was again neutral. The purified and sodium-loaded beads were then transferred to a fritted funnel attached to a vacuum line where they were rinsed again with deionized water and excess water was removed by suction. The resulting material was then dried in a 60°C oven.

After isolation of the beads and subsequent examination by scanning electron microscopy, the beads were found to have a smooth surface morphology (see Figure 5).

<u>Example 9</u>: Property Measurements

**Example 9A: Sample Preparation**

*Ion exchange of poly(a-fluoroacrylic acid) from calcium form to sodium form.* Samples of the materials from Examples 8A, 8B and 8C were exchanged to sodium form as follows. Ten grams of resin was placed in a 250 mL bottle, 200 ml of 1N hydrochloric acid (HCl) was added, and the mixture was agitated by swirling for approximately 10 minutes. The beads were allowed to sediment, the supernatant was decanted, and the procedure was repeated. After decanting the acid, the beads were washed once with approximately 200 mL of water, then twice with 200 mL of 1M sodium hydroxide (NaOH) for approximately 10 minutes. The beads were then washed again with 200 mL of water and finally were transferred to a fritted funnel and washed (with suction) with 1 L of deionized water. The resulting cake was dried overnight at 60°C. The resulting materials are denoted as Ex. 8A-Na, Ex. 8B-Na, and Ex. 8C-Na.

*Ion exchange from sodium form to calcium form for Example 8D.* Aliquots of Example 8D (in sodium form) were exchanged to calcium form as follows. Ten grams of resin were placed in a 200 mL bottle, and washed three times with 150 mL of 0.5 M calcium chloride ($CaCl_2$). The duration of the first wash was approximately one day, followed by a water rinse before the second wash (duration overnight). After decanting the second calcium chloride ($CaCl_2$) wash solution, the third calcium chloride wash solution was added (without a water rinse between). The final calcium chloride wash duration was 2 hours. The beads were then washed with 1L of deionized water on a fritted funnel with suction and dried overnight at 60°C. The material was denoted as Ex. 8D-Ca.

*Ion exchange from sodium form to calcium form in Kayexalate and Kionex.* Kayexalate (from Sanofi-Aventis) and Kionex (from Paddock Laboratories, Inc.) were purchased. The polymers were used as purchased and converted to calcium form as follows. Ten grams of each resin (purchased in sodium form) were placed in a 200 mL bottle and washed overnight with 100 mL of 0.5 M calcium chloride. The suspension was removed from the shaker the next day and allowed to sediment overnight. The supernatant was decanted, 150 mL of 0.5 M calcium chloride was added, and the suspension was shaken for two hours. The suspension was then transferred to a fritted funnel and washed with 150 mL of 0.5 M calcium chloride, followed by 1L of deionized water, using suction. The resulting beads were dried overnight at 60°C. These materials were denoted as Kayexalate-Ca and Kionex-Ca.

### Example 9B: Viscosity, Yield Stress and Moisture Content

*Preparation of hydrated resin samples for rheology testing.*

*Buffer used for hydration of resins.* For all experiments, USP Simulated Intestinal Fluid was used (USP 30 - NF25) as the buffer for swelling of the resin. Monobasic potassium phosphate (27.2 gram, $KH_2PO_4$) was dissolved in 2 liters of deionized water and 123.2 mL of 0.5 N sodium hydroxide was added. The resulting solution was mixed, and the pH was adjusted to 6.8 $\pm$ 0.1 by addition of 0.5 N sodium hydroxide. Additional deionized water was added to bring the volume to 4 liters.

The following procedure for resin hydration was employed: Each resin (3 gram $\pm$ 0.1 gram) was placed in a 20 mL scintillation vial. Buffer was added in 1 mL aliquots until the resins were nearly saturated. The mixture was then homogenized with a spatula and more buffer was added, until the resin was fully saturated and formed a free suspension upon stirring. The suspension was then vigorously stirred, and the vials were tightly capped and placed upright in a 37°C incubator for three days. The vials were then carefully removed. In all cases, the resins had settled to the bottom of the vial, forming a mass with 1-2 mL of clear supernatant on top. The supernatant was decanted by suction with a pipette tip connected to a vacuum bottle, leaving only the saturated/sedimented paste in each container, which was sealed prior to testing.

The steady state shear viscosity of the hydrated polymers was determined using a Bohlin VOR Rheometer with a parallel plate geometry (upper plate was 15 mm in diameter and lower plate was 30 mm in diameter). The gap between plates was 1 mm and the temperature was maintained at 37°C. The viscosity was obtained as a function of shear rate from 0.0083 to 1.32 $s^{-1}$. A power-law shear-thinning behavior was found for all of the samples. See Barnes et al., "An Introduction to Rheology," 1989, page 19.

Yield stress was measured using a Reologica STRESSTECH Rheometer. This rheometer also had a parallel plate geometry (upper plate was 15 mm in diameter and lower plate was 30 mm in diameter). The gap between plates was 1 mm and the temperature was maintained at 37°C. A constant frequency of 1 Hz with two integration periods was used while the shear stress was increased from 1 to $10^4$ Pa.

For both viscosity and yield stress, after the samples were loaded and gently tapped, the upper plate was slowly lowered to the testing gap. For the STRESSTECH Rheometer, this process was automatically controlled with the loading force never exceeding 20 N. For the Bohlin VOR Rheometer, this was achieved manually. After trimming material which had been extruded from the edges at a gap of 1.1 mm, the upper plate continued to move down to the desired gap of 1 mm. Then, an equilibrium time of 300 s was used to allow the sample to relax from the loading stresses and to reach a thermal equilibrium.

*Moisture content.* The moisture content of the hydrated samples was determined using thermogravimetric analysis (TGA). Because the samples were prepared by sedimentation and decanting, the measured moisture content included both moisture absorbed within the beads and interstitial water between the beads.

Samples of approximately 20 mg weight were loaded into pre-tarred aluminum pans with lids and crimped to seal (thereby preventing moisture loss). The samples were loaded onto the auto-sampler carousel of a TA Instruments Q5000-IR TGA. The lid was pierced by the automated piercing mechanism prior to analysis of each sample, and the pierced pan was then loaded into the furnace. Weight and temperature were monitored continuously as the temperature was ramped from room temperature to 300°C at a rate of 20°C per minute. The moisture content was defined as the % weight loss from room temperature to 250°C. For polystyrene sulfonate resins, there was no significant weight loss between 225°C and 300°C (upper end of the scan), so this was an accurate definition. For poly($\alpha$-fluoroacrylate) resins, there was some decomposition of the material ongoing in the 200-300°C temperature range, even after all water had been evaporated, so

the moisture content measurement was less accurate and likely to be overestimated.

The results are shown in Tables 16 and 17, wherein stdev means standard deviation.

TABLE 16. Yield stress and viscosity for cation exchange polymers in sodium form.

| Material name | Number of samples tested | Moisture content, average (wt.%) | Moisture content, stdev | Yield stress, Pa, average | Yield stress, Pa, stdev | Viscosity (Pa·s), shear rate =0.01 $sec^{-1}$, average | Viscosity (Pa·s), shear rate =0.01 $sec^{-1}$, stdev |
|---|---|---|---|---|---|---|---|
| Kavexalate® | 3 | 62.9 | 2.7 | 2515 | 516 | 5.3E+05 | 2.4E+05 |
| Kionex® | 3 | 58.6 | 3.3 | 3773 | 646 | 9.4E+05 | 1.8E+05 |
| Ex. 8D | 2 | 78.3 | 0.9 | 67 | 25 | 6.0E+04 | 5.7E+02 |
| Ex. 8A-Na | 1 | 76.7 | - | 816 | - | 1.2E+05 | - |
| Ex. 8B-Na | 1 | 73.1 | - | 1231 | - | 1.7E+05 | - |
| Ex. 8C-Na | 2 | 72.5 | 1.0 | 1335 | 147 | 1.5E+05 | 3.5E+03 |

TABLE 17. Yield stress and viscosity for cation exchange polymers in calcium form.

| Material name | Number of samples tested | Moisture content, average (wt.%) | Moisture content, stdev | Yield stress, Pa, average | Yield stress, Pa, stdev | Viscosity (Pa·s), shear rate =.01 $sec^{-1}$, average | Viscosity (Pa·s), shear rate =.01 $sec^{-1}$, stdev |
|---|---|---|---|---|---|---|---|
| Kayexalate-Ca | 1 | 67.7 | - | 3720 | - | 1.2E+06 | - |
| Kionex-Ca | 1 | 56.7 | - | 4389 | - | 1.1E+06 | - |
| Ex. 8D-Ca | 2 | 80.1 | 1.3 | 177 | 150 | 4.8E+05 | 8.9E+04 |
| Ex. 8A | 2 | 69.0 | 2.0 | 2555 | 757 | 1.3E+06 | 4.0E+05 |
| Ex. 8B | 2 | 66.7 | 2.1 | 2212 | 1454 | 7.1E+05 | 3.3E+05 |
| Ex. 8C | 4 | 64.5 | 4.4 | 3420 | 421 | 9.5E+05 | 1.6E+05 |

**Example 9C: Particle Size and Surface Roughness**

Particle size measurements were performed using a Malvern Mastersizer 2000 particle size analyzer with Hydro 2000μP dispersion unit on the samples prepared as in Example 9A or as purchased or synthesized. The method for measuring particle sizes was (1) the sample cell was filled with Simulated Intestinal Fluid (SIF, pH=6.2) using a syringe; (2) an anaerobic fill to remove bubbles was run before a background measurement was taken; (3) a sample powder was added to the sample cell containing the SIF until obscuration of 15-20% was reached and a few drops of methanol were added to the sample well to aid powder dispersion in the SIF media; and (4) the sample measurement was performed followed by a flush of the system with distilled, deionized water and isopropanol at least four times.

The instrument settings were as follows: measurement time: 12 seconds; background measurement time: 12 seconds; measurement snaps: 12,000; background snaps: 12,000; pump speed 2,000; ultrasonics: 50%; repeat measurement: 1 per aliquot; refractive index of dispersant: 1.33 (water); refractive index of particle: 1.481; and obscuration range: from 15% to 20%. The results are shown in Table 18

TABLE 18.

<table>
<tr><th>Sample ID</th><th>D(0.1), μm</th><th>D(0.5), μm</th><th>D(0.9), μm</th><th>span (D(0.9)-D(0.1))/D(0.5)</th><th colspan="2">% of particles w/diameter <10 μm</th></tr>
<tr><td rowspan="2">Ex. 8A-Na</td><td rowspan="2">94</td><td rowspan="2">143</td><td rowspan="2">219</td><td rowspan="2">0.88</td><td>Average</td><td>0.00</td></tr>
<tr><td>STDEV</td><td>0.00</td></tr>
</table>

(continued)

| Sample ID | D(0.1), µm | D(0.5), µm | D(0.9), µm | span (D(0.9)-D(0.1))/D(0.5) | % of particles w/diameter <10 µm | |
|---|---|---|---|---|---|---|
| Ex. 8B-Na | 86 | 128 | 188 | 0.79 | Average | 0.00 |
| | | | | | STDEV | 0.00 |
| Ex. 8D | 202 | 295 | 431 | 0.78 | Average | 0.00 |
| | | | | | STDEV | 0.00 |
| Kayexalate-Na | 17 | 56 | 102 | 1.52 | Average | 6.70 |
| | | | | | STDEV | 0.26 |
| Kionex-Na | 15 | 31 | 49 | 1.14 | Average | 6.60 |
| | | | | | STDEV | 0.23 |

Atomic Force Microscope (AFM) images of samples prepared by the processes substantially described in Example 8A-8C were obtained. The AFM images were collected using a NanoScope III Dimension 5000 (Digital Instruments, Santa Barbara, CA). The instrument was calibrated against a NIST traceable standard with an accuracy better than 2%. NanoProbe silicon tips were used and image processing procedures involving auto-flattening, plane fitting, or convolution were used. One 10 um x 10 um area was imaged near the top of one bead on each sample. Figures 2A and 2B show perspective view of the surfaces of the beads with vertical exaggerations wherein the z-axis was marked in 200 nm increments. Roughness analyses were performed and expressed in root-mean-square roughness (RMS), mean roughness ($R_a$), and peak-to-valley maximum height ($R_{max}$). These results are detailed in Table 19.

TABLE 19.

| Sample | RMS (Å) | $R_a$ (Å) | $R_{max}$ (Å) |
|---|---|---|---|
| 1 | 458.6 | 356.7 | 4312.3 |
| 2 | 756.1 | 599.7 | 5742.2 |

Example 10: Compressibility Index (bulk and tap density)

Bulk density (BD) and tapped density (TD) are used to calculate a compressibility index (CI). Standardized procedures for this measurement are specified as USP <616>. A quantity of the powder is weighed into a graduated cylinder. The mass M and initial (loosely packed) volume $V_o$ are recorded. The cylinder is then placed on an apparatus which raises and then drops the cylinder, from a height of 3 mm $\pm$10%, at a rate of 250 times (taps) per minute. The volume is measured after 500 taps and then again after an additional 750 taps (1250 total). If the difference in volumes after 500 and 1250 taps is less than 2%, then the final volume is recorded as $V_f$ and the experiment is complete. Otherwise, tapping is repeated in increments of 1250 taps at a time, until the volume change before and after tapping is less than 2%. The following quantities are calculated from the data:

$$\text{Bulk Density (BD)} = M/V_o$$

$$\text{Tapped Density (TD)} = M/V_f$$

Compressibility Index (CI, also called Carr's Index) = 100*(TD-BD)/TD

Kayexalate and Kionex were used as purchased. Samples of poly($\alpha$-fluoroacrylate) resins were synthesized substantially as in Example 8. The samples were tested for their CI, in the manner discussed above. The results are shown in Table 20. The results show that values of CI above 15% are characteristic of finely milled cation exchange resins (Kayexalate and Kionex), whereas substantially spherical bead resins have values of CI below 15% (samples prepared substantially as in Example 8). It was observed that after completion of the test the spherical beads could be readily poured out of the cylinder by tipping; whereas the finely milled resins required inversion of the cylinder and numerous hard taps to the cylinder with a hard object (such as a spatula or screwdriver) to dislodge the powder. The compressibility index data

and observations of the flow of the packed powders are consistent with poorer flow properties of the milled resins in dry form, compared to the spherical beads, and are also consistent with the poorer flow properties of the milled resins when wet.

TABLE 20.

| Sample | Weight (g) | $V_o$ ($cm^3$) | $V_f$ ($cm^3$) | Compressibility Index | Bulk Density ($g/cm^3$) | Tap Density ($g/cm^3$) |
|---|---|---|---|---|---|---|
| Kayexalate® | 36.1 | 49 | 40 | 18.4 | 0.737 | 0.903 |
| Kayexalate® | 42.3 | 58 | 48 | 17.2 | 0.729 | 0.881 |
| Kionex® | 38.9 | 60 | 46 | 23.3 | 0.648 | 0.846 |
| Kionex® | 42.4 | 65 | 50 | 23.1 | 0.652 | 0.848 |
| Ex. 3[a] | 47.5 | 55 | 47 | 14.5 | 0.864 | 1.011 |
| Ex. 3[a] | 62.5 | 70 | 63 | 10.0 | 0.893 | 0.992 |
| Ex. 3[a] | 85.2 | 96 | 86 | 10.4 | 0.888 | 0.991 |
| [a]Ca(FAA) prepared substantially as in Example 8. | | | | | | |

Example 11: Poly(α-fluoroacrylate) beads in the presence of varying solvent amount

The following reagents were used in the Examples 11-12: methyl 2-fluoroacrylate (MeFA); divinylbenzene (DVB), tech, 80%, mixture of isomers; 1,7-Octadiene (ODE), 98%; Lauroyl peroxide (LPO), 99% ; poly(vinyl alcohol) (PVA): 87-89% hydrolyzed; NaCl: sodium chloride; $Na_2HPO_4 \cdot 7H_2O$: sodium phosphate dibasic heptahydrate; and deionized (DI) water. The reagents are obtained from commercial sources (see Example 8), and used in accord with standard practice for those of skill in the art.

A series of polymerization reactions were run in a varying amount of dichloroethane, with increasing amounts of dichloroethane solvent from sample 11A1 to sample 11A6. The range of dichloroethane added in the synthesis was from 0 to 1g of dichloroethane for every 1 g of methylfluoroacrylate plus divinylbenzene plus octadiene.

Reaction mixtures were prepared using a liquid dispensing robot and accompanying software (available from Symyx Technologies, Inc., Sunnyvale, CA). A stock aqueous solution of NaCl, water, polyvinyl alcohol (PVA 87%), $Na_2HPO_4$•$7H_2O$ ($Na_2HPO_4$), $NaH_2PO_4$•$H_2O$ ($NaH_2PO_4$), and $NaNO_2$ was prepared. This was then dispensed into reaction tubes using the liquid dispensing robot such that the weights (g) within each tube measured what is depicted in Table 21. A stock solution of the organic components that consisted of methyl-fluoroacrylate (MeFA), divinylbenzene (DVB), octadiene (ODE), and lauroyl peroxide (LPO) was prepared and delivered using the liquid dispensing robot. Dichloroethane (DiCl Et) was also added to the tubes so that the weight (g) of each component matched the values as described in Table 21, in which all units are weight in grams (g).

TABLE 21.

| Well Number | NaCl | Water | PVA | $Na_2HPO_4$ | MeFA | DVB | ODE | LPO | DiCl Et |
|---|---|---|---|---|---|---|---|---|---|
| 11A1 | 0.13 | 4.19 | 0.04 | 0.04 | 0.80 | 0.04 | 0.04 | 0.01 | 0.00 |
| 11A 2 | 0.13 | 4.19 | 0.04 | 0.04 | 0.80 | 0.04 | 0.04 | 0.01 | 0.18 |
| 11A 3 | 0.13 | 4.19 | 0.04 | 0.04 | 0.80 | 0.04 | 0.04 | 0.01 | 0.36 |
| 11A 4 | 0.13 | 4.19 | 0.04 | 0.04 | 0.80 | 0.04 | 0.04 | 0.01 | 0.53 |
| 11A 5 | 0.13 | 4.19 | 0.04 | 0.04 | 0.80 | 0.04 | 0.04 | 0.01 | 0.71 |
| 11A 6 | 0.13 | 4.19 | 0.04 | 0.04 | 0.80 | 0.04 | 0.04 | 0.01 | 0.89 |

Reactions were run in a suspension type format, in parallel, sealed, heated reactors fitted with overhead stirrers. The parallel reactor apparatus is described in detail in U.S. Patent No. 6,994,827. In general, the stoichiometry of the reaction was maintained throughout all the wells, but solvent was added with differing concentrations within each well. The tubes with the complete recipe were loaded into the parallel reactor and stirred at 300 rpm. Nitrogen was blown over the reaction for 10 minutes and a blanket of nitrogen was maintained through out the reaction. The following heating profile was used: room temperature to 55°C over 1 hour; maintain at 55°C for 4 hours; 55°C to 80°C over 1 hour; maintain at 80°C

for 2 hours; 80°C to room temperature over 2 hours. The polymer beads were isolated from the tubes and were washed with isopropyl alcohol, ethanol, and water. The beads were dried at room temperature under reduced pressure.

Figure 3 shows the beads from the reactions, with micrograph A1 displaying a rougher surface structure than the beads prepared under other conditions. In micrographs A2 to A6, the concentration of dichloroethane was increased in the process. Examining the scanning electron microscope (SEM) results in Figure 3 from A2 to A6, there is a progression from a rougher surface to a smoother surface. Further, the reactions that contained dichloroethane had a clearer aqueous phase when compared to the reaction that did not contain dichloroethane (sample 11A1). After purification and subsequent isolation of the beads prepared in the presence of a solvent, the beads appeared transparent and their surfaces reflected light (shiny appearance). This contrasted with the beads prepared without solvent, where the beads appeared white and contained a matt (non-reflective) surface.

Example 12: Use of a salting out process to affect bead surface roughness.

A series of parallel polymerization experiments were carried out with MeFA monomer, using a salt gradient across the reactions to decrease the solubility of MeFA in the aqueous phase of a suspension polymerization. As in Example 11, polymerization reaction mixtures were prepared using a liquid dispensing robot. A stock aqueous solution of sodium chloride (NaCl), water, methylhydroxyethylcellulose (MWn 723,000), $Na_2HPO_4 \cdot 7H_2O$, $NaH_2PO_4 \cdot H_2O$, and $NaNO_2$ was prepared. This was dispensed into test tubes using a liquid dispensing robot so that each tube contained the amounts of reactants in Table 20. A stock solution of the organic components that consisted of methyl-fluoroacrylate, divinylbenzene, octadiene, lauroyl peroxide was prepared and delivered using the liquid dispensing robot. Walocel® is a purified sodium carboxymethyl cellulose that was purchased and used as received as a surfactant. Dichloroethane was also added to the tubes so that the weight (g) of each component matched the values as described in Table 22, wherein all units are weight in grams (g).

TABLE 22.

| Tube | NaCl | Water | Walocel® | $Na_2HPO_4$ | MeFA | DVB | ODE | LPO |
|---|---|---|---|---|---|---|---|---|
| B1 | 0.13 | 4.19 | 0.04 | 0.02 | 0.80 | 0.04 | 0.04 | 0.01 |
| B2 | 0.20 | 4.19 | 0.04 | 0.02 | 0.80 | 0.04 | 0.04 | 0.01 |
| B3 | 0.26 | 4.19 | 0.04 | 0.02 | 0.80 | 0.04 | 0.04 | 0.01 |
| B4 | 0.33 | 4.19 | 0.04 | 0.02 | 0.80 | 0.04 | 0.04 | 0.01 |
| B5 | 0.41 | 4.19 | 0.04 | 0.02 | 0.80 | 0.04 | 0.04 | 0.01 |
| B6 | 0.47 | 4.19 | 0.04 | 0.02 | 0.80 | 0.04 | 0.04 | 0.01 |
| B7 | 0.53 | 4.19 | 0.04 | 0.02 | 0.80 | 0.04 | 0.04 | 0.01 |
| B8 | 0.64 | 4.19 | 0.04 | 0.02 | 0.80 | 0.04 | 0.04 | 0.01 |

The tubes with the complete reaction mixtures were loaded into a parallel reactor equipped with overhead stirrers, as described in US Patent 6,994,827. The stir rate was set to 300 rpm. Nitrogen was blown over the reaction for 10 minutes and a blanket of nitrogen was maintained throughout the reaction. The following heating profile was used: room temperature to 55°C over 1 hour; maintained at 55°C for 4 hours; 55°C to 80°C over 1 hour; maintained at 80°C for 2 hours; 80°C to room temperature over 2 hours. The beads were isolated from the tubes and were washed with isopropyl alcohol, ethanol, and water. The beads were dried at room temperature under reduced pressure.

After purification of the beads from the reaction, the surface morphology of the beads was examined using SEM. As Figure 4 shows, beads from reaction B1 had a rough surface structure. Going from B1 to B8, the concentration of sodium chloride increased in the aqueous phase from 3 wt.% to 13 wt.%. A more homogeneous surface structure was observed for the surfaces of the beads that were run at higher sodium chloride concentration (e.g., SEMs B7 and B8).

Example 13: Human clinical study

Part A:

Methyl 2-fluoroacrylate (MeFA) was purchased and was vacuum distilled before use. Divinylbenzene (DVB) was purchased from Aldrich, technical grade, 80%, mixture of isomers, and was used as received. 1,7-octadiene (ODE), lauroyl peroxide (LPO), polyvinyl alcohol (PVA) (typical molecular weight 85,000-146,000, 87-89% hydrolyzed), sodium chloride (NaCl), sodium phosphate dibasic heptahydrate ($Na_2HPO_4 \cdot 7H_2O$) and sodium phosphate monobasic mono-

hydrate ($NaH_2PO_4 \cdot H_2O$) were purchased from commercial sources and used as received.

In an appropriately sized reactor with appropriate stirring and other equipment, a 90:5:5 weight ratio mixture of organic phase of monomers was prepared by mixing methyl 2-fluoroacrylate, 1,7-octadiene, and divinylbenzene. One-half part of lauroyl peroxide was added as an initiator of the polymerization reaction. A stabilizing aqueous phase was prepared from water, polyvinyl alcohol, phosphates, sodium chloride, and sodium nitrite. The aqueous and monomer phases were mixed together under nitrogen at atmospheric pressure, while maintaining the temperature below 30°C. The reaction mixture was gradually heated while stirring continuously. Once the polymerization reaction has started, the temperature of the reaction mixture was allowed to rise to a maximum of 95°C.

After completion of the polymerization reaction, the reaction mixture was cooled and the aqueous phase was removed. Water was added, the mixture was stirred, and the solid material was isolated by filtration. The solid was then washed with water to yield a crosslinked (methyl 2-fluoroacrylate)-divinylbenzene-1,7-octadiene polymer. The (methyl 2-fluoroacrylate)-divinylbenzene-1,7-octadiene copolymer was hydrolyzed with an excess of aqueous sodium hydroxide solution at 90 °C for 24 hours to yield (sodium 2-fluoroacrylate)-divinylbenzene-1,7-octadiene polymer. After hydrolysis, the solid was filtered and washed with water. The (sodium 2-fluoroacrylate)-divinylbenzene-1,7-octadiene polymer was exposed at room temperature to an excess of aqueous calcium chloride solution to yield insoluble cross-linked (calcium 2-fluoroacrylate)-divinylbenzene-1,7-octadiene polymer.

After the calcium ion exchange, the wet polymer is slurried with 25-30 % w/w aqueous solution of sorbitol at ambient temperature to yield sorbitol-loaded polymer. Excess sorbitol is removed by filtration. The resulting polymer is dried at 20-30 °C until the desired moisture content (10-25 w/w/%) is reached. This provides a sorbitol loaded, cross-linked (calcium 2-fluoroacrylate)-divinylbenzene-1,7-octadiene polymer.

Part B:

The objective of the study was to evaluate the equivalence of once a day, two times a day and three times a day dosing of the polymer from Part A of this example. After a four day period to control diet, 12 healthy volunteers were randomized in an open-label, multiple-dose crossover study. The polymer was administered orally as an aqueous suspension of 30 grams (g) once a day for six days, 15 g twice a day for six days, and 10 g three times a day for 6 days in a randomly assigned order based upon 1 of 6 dosing sequences. Laboratory and adverse event assessments were performed throughout the study to monitor safety and tolerability. Subjects were required to consume a controlled diet for the duration of the study. Feces and urine were collected over 24 hour intervals on certain study days to assess potassium excretion.

Subjects were healthy adult males or females without a history of significant medical disease, 18 to 55 years of age, with a body mass index between 19 and 29 $kg/m^2$ at the screening visit, serum potassium level >4.0 and ≤5.0 mEq/L, and serum magnesium, calcium, and sodium levels within normal range. Females of childbearing potential must have been non-pregnant and non-lactating and must have used a highly effective form of contraception before, during, and after the study.

Multiple-dose administration of 30g polymer for 6 days each as either 30g once daily, 15g twice daily or 10g three-times daily, respectively was well tolerated. No serious adverse events were reported, and all adverse events were mild or moderate in severity. An effect was apparent for fecal and urinary excretion of potassium.

For fecal potassium excretion, the mean daily values and change from baseline values were significantly increased for all three dosing regimens. The volunteers receiving the polymer once per day excreted 82.8% of the amount of fecal potassium as those volunteers who received substantially the same amount of the same polymer three-times per day. It is also shown that volunteers receiving the polymer twice per day excreted 91.5% of the amount of fecal potassium as those volunteers who received substantially the same amount of the same polymer three-times per day. For urinary potassium excretion, the mean daily values and change from baseline values were significantly decreased for all three dosing regimens. Surprisingly, there was no statistically significant difference between the three dosing regimens.

Regarding tolerability, 2 of the 12 subjects receiving once a day dosing or twice a day dosing reported mild or moderate gastrointestinal adverse events (including flatulence, diarrhea, abdominal pain, constipation, stomatitis, nausea and/or vomiting). Also, 2 of 12 subjects reported mild or moderate gastrointestinal adverse events on the baseline control diet. Thus, less than 16.7% of these subjects reported mild or moderate gastrointestinal adverse events, an indication that, as used herein, dosing once or twice a day was well tolerated. None of the subjects reported severe gastrointestinal adverse events for any of the dosing regimens or at baseline.

Part C:

Another study was performed to assess the safety and efficacy of a binding polymer that was the same as described above in Part A of this example, but without the sorbitol loading. Thirty-three healthy subjects (26 male and 7 female) between the ages of 18 and 55 years received single and multiple doses of polymer or placebo in a double-blind,

randomized, parallel-group study. Eight subjects each were randomly assigned to one of four treatment groups receiving polymer or matching placebo. The subjects received 1, 5, 10, or 20 g of polymer or placebo as a single dose on study day 1, followed by three times daily dosing for eight days following seven days of diet control. Subjects were required to consume a controlled diet for the duration of the study.

The polymer was well-tolerated by all subjects. No serious adverse events occurred. Gastrointestinal adverse events reported were mild to moderate in severity for one subject. There was no apparent dose response relationship in gastrointestinal or overall adverse event reporting, and no increase in adverse event reports versus placebo.

At the end of the multiple-dose study period, a dose response effect was apparent for fecal and urinary excretion of potassium. For fecal potassium excretion, the mean daily values and change from baseline values were significantly increased in a dose-related manner. For urinary potassium excretion, the mean daily values and change from baseline values were decreased in a dose-related manner.

In comparison of Part C to Part B, those volunteers receiving the same amount of polymer that had the sorbitol loading (Part B) excreted about 20% more potassium in the feces as compared to those volunteers receiving the non-sorbitol loaded polymer (Part C).

Example 14: Preparation of Sample A

In a 2L reactor with appropriate stirring and other equipment, a 180:10:10 weight ratio mixture of organic phase of monomers was prepared by mixing methyl 2-fluoroacrylate (~0.24 kg), 1,7-octadiene (~0.0124 kg), and divinylbenzene (~0.0124 kg). One part of lauroyl peroxide (~0.0012 kg) was added as an initiator of the polymerization reaction. A stabilizing aqueous phase was prepared from water, polyvinyl alcohol, phosphates, sodium chloride, and sodium nitrite. The aqueous and monomer phases were mixed together under nitrogen at atmospheric pressure, while maintaining the temperature below 30°C. The reaction mixture was gradually heated while stirring continuously. Once the polymerization reaction has started, the temperature of the reaction mixture was allowed to rise to a maximum of 95°C. After completion of the polymerization reaction, the reaction mixture was cooled and the aqueous phase was removed. Water was added, the mixture was stirred, and the solid material was isolated by filtration, and then washed with water.

The polymerization reaction was repeated 5 more times, the polymer from the batches were combined together to yield about 1.7 kg of a crosslinked (methyl 2-fluoroacrylate)-divinylbenzene-1,7-octadiene polymer. The (methyl 2-fluoroacrylate)-divinylbenzene-1,7-octadiene polymer was hydrolyzed with an excess of aqueous sodium hydroxide and isopropanol solution at 65 °C for 24 hours to yield (sodium 2-fluoroacrylate)-divinylbenzene-1,7-octadiene polymer. After hydrolysis, the solid was filtered and washed with water. The (sodium 2-fluoroacrylate)-divinylbenzene-1,7-octadiene polymer was exposed at room temperature to an excess of aqueous calcium chloride solution to yield insoluble cross-linked (calcium 2-fluoroacrylate)-divinylbenzene-1,7-octadiene polymer. After the calcium ion exchange, the Sample A-Ca product was washed with water and dried.

To prepare the sodium form of the polymer, ten grams of resin from above was placed in a 250 mL bottle, 200 ml of 1N hydrochloric acid (HCl) was added, and the mixture was agitated by swirling for approximately 10 minutes. The beads were allowed to sediment, the supernatant was decanted, and the procedure was repeated. After decanting the acid, the beads were washed once with approximately 200 mL of water, then twice with 200 mL of 1M sodium hydroxide (NaOH) for approximately 10 minutes. The beads were then washed again with 200 mL of water and finally were transferred to a fritted funnel and washed (with suction) with 1 L of deionized water. The resulting cake was dried overnight at 60°C, resulting in Sample A-Na.

Example 15: Ex vivo potassium binding studies

Potassium binding by Sample A-Na and Sample A-Ca, from Example 14, was evaluated in *ex vivo* human fecal and colonic extracts. Two fecal samples, and one colonic sample obtained through use of a colostomy bag, were provided by three human volunteers. The samples were centrifuged, and the resulting supernatant was isolated for use as a test medium in the binding study. Sample A in both sodium and calcium form was added to the extract samples at 20 mg/mL, and incubated for 24 hours at 37°C. Binding of potassium, as well as other cations present in the extracts was determined per gram of Sample A.

Both test agents were dried by lyophilization before use. The sodium form (Sample A-Na) bound and removed an average of 1.54 milliequivalents (mEq) of potassium per gram, while the calcium form (Sample A-Ca) bound an average of 0.85 mEq potassium per gram from the three extracts.

Fecal samples were supplied by two healthy male volunteers (subjects #1 and #2), ages 36 and 33, of Caucasian and Asian descent, respectively. Fecal samples were collected in one-gallon Ziploc bags and immediately mixed and transferred into centrifuge tubes. The colonic sample was provided by an 81-year-old Caucasian female donor (subject #3) through use of a colostomy bag. The colostomy bag contents were shipped on dry ice, thawed, mixed and transferred into centrifuge tubes. The fecal and colonic samples were centrifuged at 21,000 rpm for 20 hours at 4°C (Beckman JS-25.50

rotor in Beckman-Coulter Avanti J-E centrifuge). The resulting supernatant was pooled per subject, and filtered using a Nalgene 0.2 µm disposable filter unit. The fecal and colonic extracts were then either used fresh, or were frozen at -20°C until needed.

*Method to determine cation binding of Sample A in fecal and colonic extracts.* Fecal and colonic extracts were thawed in a room temperature water bath and stirred on a magnetic stir plate. Penicillin G / Streptomycin (Gibco, 15140-122) (1/100 volume of 100x stock solution) and sodium azide (1/1000 volume of 10% stock solution) were added to each extract sample to discourage bacterial or fungal growth during the assay. Sample A-Na and Sample A-Ca were added to 16x100 mm glass tubes in duplicate, with each tube receiving 140 to 170 mg of dried, accurately weighed sample. While stirring, fecal or colonic extract was dispensed into the tubes to create a final concentration of 20 mg of test sample per mL of extract. Each extract was additionally dispensed into duplicate tubes containing no test sample. All tubes were sealed and incubated for 24 hours at 37°C, rotating on a rotisserie mixer. Following incubation, 25 µL of each sample was diluted into 475 µL of Milli-Q purified water (1:20 dilution). The diluted samples were then filtered by centrifugation at 13,200 rpm through Microcon YM-3 filter units (3000 MWCO) for 1 hour. Filtrates were transferred to a 1 mL 96-well plate and submitted for analysis of cation concentrations by ion chromatography.

*Ion chromatography method for measurement of cation concentrations in fecal and colonic extracts.* Cation concentrations in the fecal and colonic extract samples were analyzed using a strong cation exchange column set (Dionex CG16 50x5mm ID and CS16 250x5mm ID), on a Dionex ICS2000 system equipped with a Dionex WPS3000 auto sampler, DS3 conductivity flow cell and CSRS-Ultra II 4mm Suppressor. The ion chromatography detection method included an isocratic elution using 30 mM of methanesulfonic acid at a flow rate of 1 mL/minute, and the total run time was 30 minutes per sample.

*Data Analysis.* Cation binding was calculated as ($C_{start}$ - $C_{eq}$) / 20 * valency of the ion, where $C_{start}$ is the starting concentration of cation in the fecal or colonic extract (in mM), $C_{eq}$ is the concentration of cation remaining in the sample at equilibrium after exposure to the test agent (in mM), and 20 corresponds to the concentration of the test agent (in mg/mL). Multiplying by the valency of the ion (1 for potassium, ammonium and sodium; 2 for calcium and magnesium) gives a binding value expressed in milliequivalents (mEq) of ion bound per gram of test agent. All samples were tested in duplicate with values reported as an average (Avg), +/- standard deviation (SD).

TABLE 23.

| No. | Extract Sample | $C_{start}$ (mM) | $C_{eq}$ (mM) | $K^+$ Binding (mEq/g) | $K^+$ Binding in Individual Extracts | | All Extract Samples Avg ± SD |
|---|---|---|---|---|---|---|---|
| | | | | | Avg | SD | |
| Sample A-Na | Fecal, subject #1 | 92.7 | 65.3 | 1.37 | 1.33 | 0.06 | 1.54 ± 0.18 |
| | | | 67.0 | 1.29 | | | |
| | Fecal, subject #2 | 106.6 | 73.9 | 1.64 | 1.63 | 0.01 | |
| | | | 74.3 | 1.62 | | | |
| | Colonic, subject #3 | 128.8 | 93.9 | 1.74 | 1.67 | 0.10 | |
| | | | 96.6 | 1.61 | | | |
| Sample A-Ca | Fecal, subject #1 | 92.7 | 77.8 | 0.75 | 0.77 | 0.03 | 0.85 ± 0.10 |
| | | | 76.9 | 0.79 | | | |
| | Fecal, subject #2 | 106.6 | 90.2 | 0.82 | 0.82 | 0.00 | |
| | | | 90.2 | 0.82 | | | |
| | Colonic, subject #3 | 128.8 | 109.0 | 0.99 | 0.97 | 0.02 | |
| | | | 109.7 | 0.96 | | | |
| | | | | | Avg | SD | |

Potassium binding in mEq/g was determined for calcium- and sodium-loaded Sample A following a 24-hour incubation in two human fecal extracts and one colonic extract. Initial potassium levels in the three extract samples ranged from 92.7 mM to 128.8 mM. With the addition of 20 mg/ml of sodium-loaded Sample A-Na, the potassium concentration in

the extracts was reduced by approximately 28%. The potassium bound per gram of polymer averaged 1.54 mEq/g. Calcium-loaded Sample A-Ca bound an average of 0.85 mEq/g.

Example 16: Pig model cation binding studies

Pigs with normal renal function were used as a model to assess the pharmacological effects of Ca(polyFAA) in binding and removing potassium from the gastrointestinal tract. A pig model is used based on the well known similarities between the pig and human gastrointestinal tracts. The pigs were fed a diet supplemented with Ca(polyFAA) at a concentration of 1 gram per kilogram of body weight per day. As a control, pigs were fed the diet without Ca(polyFAA).

*Materials.* Ca(polyFAA) was synthesized using a method similar to that described in Example 14 and used in its calcium form. Ferric oxide (purchased from Fisher Scientific), lot number 046168, was added as an indigestible marker. The ferric oxide was used as a daily visible marker to determine the passage rate of the digesta through the gastrointestinal tract of each animal.

*Animals.* Fourteen approximately nine-week old grower barrows (Camborough 15 or 22 dams x Terminal Sire boars; PIC Canada Inc.) weighing approximately 25 kg were used in this study. At the start of the experiment, fourteen pigs were weighed and randomized by weight into control and treatment groups. The experiment was divided into two feeding periods. The first period was the acclimation period, days (D(-7) to D(-1)), and the second was the test period, (D(1) to D(9)).

Before the acclimation period, the pigs were fed a standard production diet. During the acclimation period, pigs were progressively offered increasing amounts of the control diet as a ratio to a standard production grower diet.

On the same day the pigs were fed the ferric oxide, the seven test pigs were switched to the test diet. The control pigs remained on the control (acclimation) diet. The test diet was fed for ten days (D(1) to D(10)). Throughout the entire study, daily feed allowance for individual pigs was divided in two equal sizes and offered at approximately 08:30 and 15:30. The pigs were trained to clean up their daily feed allowance once it was provided; any feed that was not eaten was weighed and removed before the next feeding.

*Urine Collection.* Urine collection began with the offering of the ferric oxide bolus on D(1). Each day's sample was kept separate for each pig. Following the completion of urine collection, the daily samples for each pig were thawed, mixed well and sub-sampled. The sub-sample of at least 10 mL of each pig's 24-hour sample was analyzed for electrolyte concentrations as described below.

*Fecal Collections.* Fecal collection began with the offering of the ferric oxide bolus on D(1). Each day's sample was kept separate for each pig.

*Urine electrolytes.* Urine samples were thawed, diluted 30 fold in 50 mM hydrochloric acid and then filtered (Whatman 0.45 micron PP filter plate, 1000xg for 10 minutes). The cation concentrations in these urine samples were analyzed using a strong cation exchange column set (Dionex CG16 50x5mm ID and CS16 250x5mm ID), on a Dionex ICS2000 system equipped with a Dionex AS50 auto sampler, DS3 conductivity flow cell and CSRS-Ultra II 4mm Suppressor. The ion chromatography detection method included an isocratic elution using 31 mM methanesulfonic acid at a flow rate of 1mL/minute, and the total run time was 33 minutes per sample.

*Fecal electrolytes.* To a 15mL conical tube, 200mg of feces and 10mL of 1M hydrochloric acid was added. The fecal mixture was incubated for approximately 40 hours on a rotisserie mixer at room temperature. A sample of fecal supernatant was isolated after centrifugation (2000xg, 15 minutes) and then filtered (Whatman 0.45 micron PP filter plate, 1000xg for 10 minutes). The filtrate was diluted 2 fold with Milli-Q water.

Diluted filtrate cation content was measured by inductively coupled plasma optical emission spectrometry (ICP-OES) using a Thermo Intrepid II XSP Radial View. Samples were infused into the spray chamber using a peristaltic pump and CETAC ASX-510 autosampler. An internal standard, yttrium (10ppm in 1M hydrochloric acid), was employed for correcting variation in sample flow as well as plasma conditions. The emission line that was used for quantifying potassium was 7664nm (internal standard 437.4nm).

*Data Analysis.* Fecal electrolytes were calculated in milliequivalents per day (mEq/day) using the following equation:

$$\text{mEq/day} = \left[\frac{(\text{mEq/L electrolyte x assay volume (L)})}{(\text{grams feces in assay})}\right] \text{x} \left[\frac{\text{Total feces (grams)}}{\text{Day}}\right]$$

In the above equation, mEq/L electrolyte was the concentration of an electrolyte reported by ICP spectrometry after adjusting for dilution factor and valence, and total feces per day was the amount, in grams, of feces collected in a 24 hour period after lyophilization.

Urinary electrolytes were calculated in mEq electrolyte excreted per day (mEq/day) using the following equation:

(mEq electrolyte per L) * (24 hour urine volume). Data was presented using means ± standard deviation, and/or by scatter plot. Statistical analysis was performed in GraphPad Prism, version 4.03. For urine and fecal analyses, probability (p) values were calculated using a two-tailed t-test to compare the Ca(polyFAA) treated group to the non-treatment control group. Statistical significance is indicated if the calculated p value is less than 0.05.

For fecal analysis, the mean result from each group was determined by averaging the combined mEq/day electrolyte values from treatment days three through day eight for each animal and then averaging this result for each treatment group. This methodology was also employed for urinary electrolytes, but the average for each animal was from treatment (1) through day (8).

*GI Transit Time.* The transit times of the ferric oxide marker dosed on day (1) of the study, based on the appearance of red in the feces is shown in Table 24. In no pig was the transit time greater than 60 hours. Therefore, feces from day 3 onward were assessed for cation content.

TABLE 24. Transit time of Ferric Oxide

| Transit Time of Ferric Oxide | Average (hours) | Standard Deviation |
|---|---|---|
| hours to first appearance | 23.9 | 11.3 |
| hours to last appearance | 54.6 | 5.2 |

*Fecal Electrolytes.* On day 1, the baseline fecal cations were measured in samples collected before the presence of ferric oxide was seen in the feces. Baseline fecal potassium values are summarized in Table 25. Fecal potassium values for treatment days 3-8 are summarized in Table 26. The Ca(polyFAA) treated pigs had significantly higher levels of fecal potassium excretion than the non-treatment group ($p<0.05$).

TABLE 25. Fecal electrolytes, baseline (day 1)

| | Potassium mEq/day |
|---|---|
| Non-treatment | 31.2 ± 5.5 |
| Ca(polyFAA) | 27.0 ± 7.2 |
| p* | ns |
| *p values calculated using a two-tailed t-test<br>ns= not statistically significant | |

TABLE 26. Fecal electrolytes, average (days 3-8)

| | Potassium mEq/day |
|---|---|
| Non-treatment | 37.4 ± 7.8 |
| Ca(polyFAA) | 45.3 ± 5.3 |
| p* | p<0.05 |
| *p values calculated using a two-tailed t-test | |

*Urine electrolytes.* No baseline urine electrolyte measurements were taken. Urine electrolyte values for treatment days 1-8 are summarized in Table 27.

TABLE 27. Urine electrolytes, average (days 1-8)

| | Potassium mEq/day |
|---|---|
| Non-treatment | 88.9 ± 15.5 |
| Ca(polyFAA) | 71.8 ± 9.7 |
| p* | p<0.05 |
| *p values calculated using a two-tailed t-test | |

When introducing elements of the present invention or the embodiments(s) thereof, the articles "a", "an", "the"

and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above compositions and methods without departing from the scope of the invention, it is intended that all matter contained in the above description shall be interpreted as illustrative and not in a limiting sense.

EMBODIMENTS

Embodiment 1 A crosslinked cation exchange polymer comprising structural units corresponding to Formulae 1, 2, and 3, wherein

(i) the structural units corresponding to Formula 1 constitute at least 85 wt.% based on the total weight of structural units of Formulae 1, 2, and 3 in the polymer, calculated from the amounts of monomers used in the polymerization reaction, and the weight ratio of the structural unit corresponding to Formula 2 to the structural unit corresponding to Formula 3 is from 4:1 to 1:4, or

(ii) the mole fraction of the structural unit of Formula 1 in the polymer is at least 0.87 based on the total number of moles of the structural units of Formulae 1, 2, and 3, calculated from the amounts of monomers used in the polymerization reaction, and the mole ratio of the structural unit ofFormula 2 to the structural unit of Formula 3 is from 0.2:1 to 7: 1, and Formula 1, Formula 2, and Formula 3 correspond to the following structures:

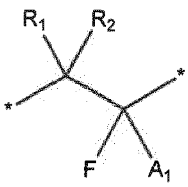

Formula 1

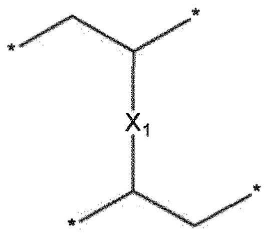

Formula 2

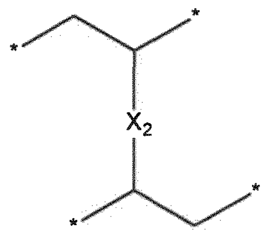

Formula 3

wherein

$R_1$ and $R_2$ are independently hydrogen, alkyl, cycloalkyl, or aryl;
$A_1$ is carboxylic, phosphonic, or phosphoric, in its salt or acid form;
$X_1$ is arylene;
$X_2$ is alkylene, an ether moiety, or an amide moiety.

Embodiment 2 The polymer of embodiment 1 wherein Formula 1, Formula 2, and Formula 3 correspond to the following structures:

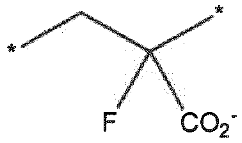

Formula 1A

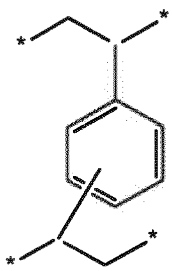

Formula 2A

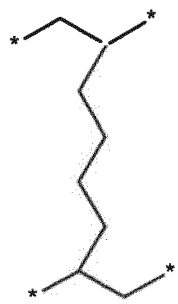

Formula 3A .

Embodiment 3 A crosslinked cation exchange polymer comprising a reaction product of a polymerization mixture comprising three or more monomers, the monomers corresponding to Formula 11, Formula 22 and Formula 33; wherein

(i) the monomers corresponding to Formula 11 constitute at least 85 wt.% based on the total weight of monomers of Formulae 11, 22, and 33 in the polymerization mixture, and the weight ratio of the monomer corresponding to Formula 22 to the monomer corresponding to Formula 33 is from 4:1 to 1:4, or

(ii) the mole fraction of the monomer of Formula 11 in the polymerization mixture is at least 0.87 based on the total number of moles of the monomers of Formulae 11, 22, and 33, and the mole ratio of the monomer of Formula 22 to the monomer of Formula 33 in the polymerization mixture is from 0.2:1 to 7:1, and

Formula 11, Formula 22, and Formula 33 correspond to the following structures:

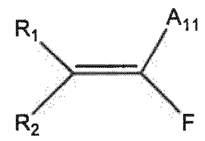

Formula 11

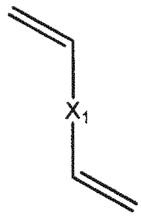

Formula 22

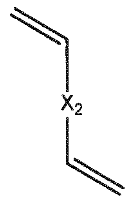

Formula 33

wherein

$R_1$ and $R_2$ are each independently hydrogen, alkyl, cycloalkyl, or aryl;
$A_{11}$ is an optionally protected carboxylic, phosphonic, or phosphoric;
$X_1$ is arylene; and
$X_2$ is alkylene, an ether moiety, or an amide moiety.

Embodiment 4 The polymer of embodiment 3 wherein Formulae 11, 22, and 33 correspond to the following structures:

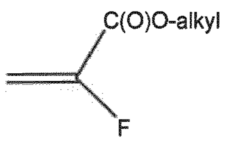

Formula 11A

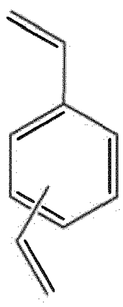

Formula 22A

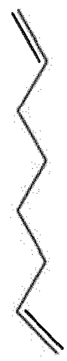

Formula 33A .

Embodiment 5 The polymer of embodiment 3 wherein $A_{11}$ is protected carboxylic, phosphonic, or phosphoric

Embodiment 6 The polymer of any one of embodiments 3 to 5 wherein the polymerization mixture further comprises a polymerization initiator.

Embodiment 7 A crosslinked cation exchange polymer in an acid or salt form, the cation exchange polymer comprising a reaction of the crosslinked polymer of any one of embodiments 3 to 6 and a hydrolysis agent.

Embodiment 8 The polymer of any one of embodiments 1, 3, and 5 to 7 wherein $X_2$ of Formulae 3 or 33 is either (a) an ether moiety selected from

either $-(CH_2)_d-O-(CH_2)_e-$ or $-(CH_2)_d-O-(CH_2)_e-O-(CH_2)_d-$ wherein d and e are independently an integer of 1 through 5, or (b) an amide moiety of the formula $-C(O)-NH-(CH_2)_p-NH-C(O)-$ wherein p is an integer of 1 through 8, or (c) Formula 3 or 33 is a mixture of structural units having the ether moiety and the amide moiety.

Embodiment 9 The polymer of any one of embodiments 1, 3, and 5 to 7 wherein $X_2$ is alkylene, optionally ethylene, propylene, butylene, pentylene, or hexylene.

Embodiment 10 The polymer of any one of embodiments 1, 3, and 5 to 9 wherein $X_1$ is phenylene.

Embodiment 11 The polymer of any one of embodiments 1, 3, and 5 to 10 wherein $R_1$ and $R_2$ are hydrogen.

Embodiment 12 The polymer of any one of embodiments 1, 2, and 7 to 11 wherein the cation of the salt is calcium, sodium, or a combination thereof; preferably wherein the cation of the salt is calcium.

Embodiment 13 A crosslinked cation exchange polymer or a pharmaceutical composition for removing potassium from the gastrointestinal tract wherein the therapy comprises administering a polymer of any one of embodiments 1 to 12 to an animal subject in need thereof, whereby the pharmaceutical composition or the polymer passes through the gastrointestinal tract of the subject, and removes a therapeutically effective amount of potassium ion from the gastrointestinal tract of the subject.

Embodiment 14 The pharmaceutical composition of embodiment 13 wherein the subject is experiencing hyperkalemia, suffering from chronic kidney disease, suffering from congestive heart failure, and/or undergoing dialysis.

Embodiment 15 The pharmaceutical composition of embodiment 13 or 14 wherein the subject is a human and the human is being treated with an agent that causes potassium retention, the agent that causes potassium retention being an angiotensin-converting enzyme inhibitor (optionally captopril, zofenopril, enalapril, ramipril, quinapril, perindopril, lisinopril, benazipril, fosinopril, or a combination thereot), an angiotensin receptor blocker (optionally candesartan, eprosartan, irbesartan, losartan, olmesartan, telmisartan, valsaitan, or a combination thereof), an aldosterone antagonist (optionally spironolactone, eplerenone, or a combination thereof).

Embodiment 16. A crosslinked cation exchange polymer comprising structural units corresponding to Formulae 1, 2, and 3, wherein

(i) the structural units corresponding to Formula 1 constitute at least 85 wt.% based on the total weight of structural units of Formulae 1, 2, and 3 in the polymer, calculated from the amounts of monomers used in the polymerization reaction, and the weight ratio of the structural unit corresponding to Formula 2 to the structural unit corresponding to Formula 3 is from 4:1 to 1:4, or

(ii) the mole fraction of the structural unit of Formula 1 in the polymer is at least 0.87 based on the total number of moles of the structural units of Formulae 1, 2, and 3, calculated from the amounts of monomers used in the polymerization reaction, and the mole ratio of the structural unit of Formula 2 to the structural unit of Formula 3 is from 0.2:1 to 7: 1, and Formula 1, Formula 2, and Formula 3 correspond to the following structures:

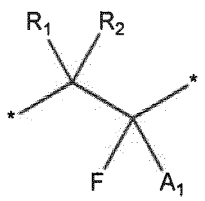

Formula 1

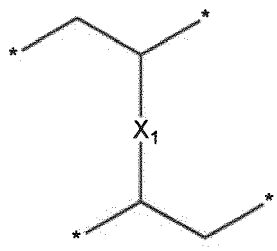

Formula 2

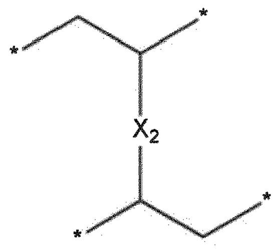

Formula 3

wherein

$R_1$ and $R_2$ are independently hydrogen, alkyl, cycloalkyl, or aryl;
$A_1$ is carboxylic, phosphonic, or phosphoric, in its salt or acid form;
$X_1$ is arylene;
$X_2$ is alkylene, an ether moiety or an amide moiety.

Embodiment 17 The polymer of Embodiment 16 wherein Formula 1, Formula 2, and Formula 3 correspond to the following structures:

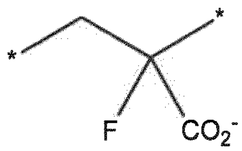
Formula 1A

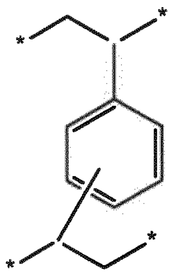
Formula 2A

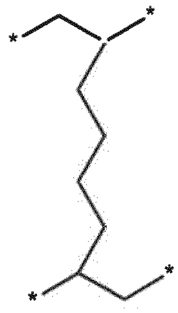
Formula 3A .

Embodiment 18 A crosslinked cation exchange polymer comprising a reaction product of a polymerization mixture comprising three or more monomers, the monomers corresponding to Formula 11, Formula 22, and Formula 33; wherein

(i) the monomers corresponding to Formula 11 constitute at least 85 wt.% based on the total weight of monomers of Formulae 11, 22, and 33 in the polymerization mixture, and the weight ratio of the monomer corresponding to Formula 22 to the monomer corresponding to Formula 33 is from 4:1 to 1:4, or
(ii) the mole fraction of the monomer of Formula 11 in the polymerization mixture is at least 0.87 based on the total number of moles of the monomers of Formulae 11, 22, and 33 and the mole ratio of the monomer of Formula 22 to the monomer of Formula 33 in the polymerization mixture is from 0.2:1 to 7: 1, and
Formula 11, Formula 22, and Formula 33 correspond to the following structures:

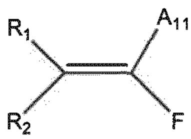
Formula 11

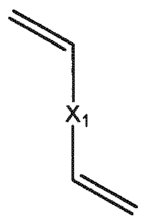
Formula 22

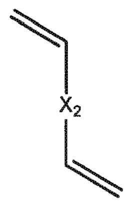
Formula 33

wherein

$R_1$ and $R_2$ are each independently hydrogen, alkyl, cycloalkyl, or aryl;
$A_{11}$ is an optionally protected carboxylic, phosphonic, or phosphoric;
$X_1$ is arylene; and
$X_2$ is alkylene, an ether moiety, or an amide moiety

Embodiment 19 The polymer of Embodiment 18 wherein Formula 11, Formula 22, and Formula 33 correspond to the following structures:

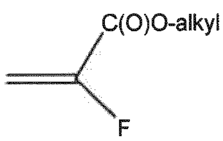
Formula 11A

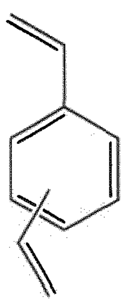
Formula 22A

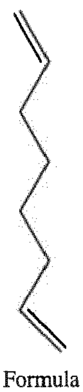
Formula 33A .

Embodiment 20. The polymer of Embodiment 18 wherein $A_{11}$ is protected carboxylic, phosphonic, or phosphoric.
Embodiment 21. The polymer of any one of Embodiments 18 to 20 wherein the polymerization mixture further comprises a polymerization initiator.
Embodiment 22. A crosslinked cation exchange polymer in an acid or salt form, the cation exchange polymer comprising a reaction product of the crosslinked polymer of any one of Embodiments 18 to 21 and a hydrolysis agent.
Embodiment 23. The polymer of any one of Embodiments 16, 18, and 20 to 22 wherein $X_2$ of Formulae 3 or 33 is either (a) an ether moiety selected from

either $-(CH_2)_d-O-(CH_2)_e-$ or $-(CH_2)_d-O-(CH_2)_e-O-(CH_2)_d-$ wherein d and e are independently an integer of 1 through 5, or (b) an amide moiety of the formula $-C(O)-NH-(CH_2)_p-NH-C(O)-$ wherein p is an integer of 1 through 8, or (c) Formulae 3 or 33 is a mixture of structural units having the ether moiety and the amide moiety.

Embodiment 24. The polymer of any one of Embodiments 16, 18, and 20 to 22 wherein $X_2$ is alkylene, optionally ethylene, propylene, butylene, pentylene, or hexylene.
Embodiment 25. The polymer of any one ofEmbodiments 16, 18, and 20 to 24 wherein $X_1$ is phenylene.
Embodiment 26. The polymer of any one of Embodiments 16, 18, and 20 to 25 wherein $R_1$ and $R_2$ are hydrogen.
Embodiment 27. The polymer of any one of Embodiments 16, 17, and 22 to 26 wherein the cation of the salt is calcium, sodium, or a combination thereof.
Embodiment 28. The polymer of Embodiment 27 wherein the cation of the salt is calcium.
Embodiment 29. A crosslinked cation exchange polymer or a pharmaceutical composition for removing potassium from the gastrointestinal tract wherein the therapy comprises administering a polymer of any one of Embodiments 16 to 28 to an animal subject in need thereof, whereby the pharmaceutical composition or the polymer passes through the gastrointestinal tract of the subject, and removes a therapeutically effective amount of potassium ion from the gastrointestinal tract of the subject.
Embodiment 30. The pharmaceutical composition of Embodiment 29 wherein the subject is experiencing hyperkalemia, suffering rom chronic kidney disease, suffering from congestive heart failure, or undergoing dialysis.
Embodiment 31. The pharmaceutical composition of Embodiment 29 or 30 wherein the subject is a human and the human is being treated with an agent that causes potassium retention, the agent that causes potassium retention being an angiotensin-converting enzyme inhibitor (optionally captopril, zofenopril, enalapril, ramipril, quinapril, perindopril, lisinopril, benazipril, fosinopril, or a combination thereof), an angiotensin receptor blocker (optionally candesartan, eprosartan, irbesartan, losartan, olrnesartan, telmisartan, valsartan, or a combination thereof), an aldosterone antagonist (optionally spironolactone, eplerenone, or a combination thereof). Embodiment 32. A composition comprising linear sugar alcohol stabilized crosslinked (calcium 2-fluoroacrylate)-divinylbenzene-1,7-octadiene terpolymer.
Embodiment 33. A composition according to Embodiment 32, obtainable by slurrying the crosslinked (calcium 2-fluoroacrylate)-divinylbenzene-1,7-octadiene terpolymer with an aqueous solution of the linear sugar alcohol.
Embodiment 34. A composition according to Embodiment 33 wherein the slurry contains an excess amount of the linear sugar alcohol based on polymer weight and wherein the slurring is maintained under standard conditions.
Embodiment 35. A composition according to Embodiment 34 wherein the slurrying is maintained for at least 3 hours at ambient temperature and pressure.
Embodiment 36. A composition according to Embodiment 32 to 35, comprising one or more excipients.
Embodiment 37. A composition according to Embodiment 32 to 36, wherein the linear sugar alcohol is sorbitol.
Embodiment 38. A process for the manufacture of the composition according to any of Embodiments 32 to 37, wherein the cross-linked cation exchange polymer is slurried with an aqueous solution of the linear sugar alcohol.
Embodiment 39. The composition according to any of Embodiments 32 to 37 for use in the treatment of hyperkalemia, chronic kidney disease, and/or congestive heart failure.

## Claims

1. A linear sugar alcohol stabilized crosslinked (calcium 2-fluoroacrylate)-divinylbenzene-1,7-octadiene terpolymer with an amount of calcium in the polymer in the range of from about 8 wt.% to about 25 wt.%, based on the total weight of the polymer.

2. A linear sugar alcohol stabilized crosslinked (calcium 2-fluoroacrylate)-divinylbenzene-1,7-octadiene terpolymer, which is in the form of substantially spherical particles having a mean diameter of from about 20 $\mu$m to about 200 $\mu$m.

3. A linear sugar alcohol stabilized crosslinked (calcium 2-fluoroacrylate)-divinylbenzene-1,7-octadiene terpolymer,

which is in the form of substantially spherical particles having a particle size distribution wherein not more than about 5 volume% of the particles have a diameter less than about 30 μm (i.e., D(0.05) < 30 μm), not more than about 5 volume% of the particles have a diameter greater than about 250 μm (i.e., D(0.05) > 250 μm), and at least about 50 volume% of the particles have a diameter in the range from about 70 to about 150 μm.

**4.** A linear sugar alcohol stabilized crosslinked (calcium 2-fluoroacrylate)-divinylbenzene-1,7-octadiene terpolymer having a swelling ratio in physiological isotonic buffer of less than about 10 grams of water per gram of polymer.

**5.** A linear sugar alcohol stabilized crosslinked (calcium 2-fluoroacrylate)-divinylbenzene-1,7-octadiene terpolymer being **characterized by** two or more of the parameters as defined in claims 1 to 4.

**6.** A linear sugar alcohol stabilized crosslinked (calcium 2-fluoroacrylate)-divinylbenzene-1,7-octadiene terpolymer obtainable by slurrying a crosslinked (calcium 2-fluoroacrylate)-divinylbenzene-1,7-octadiene terpolymer with an aqueous solution of a linear sugar alcohol, wherein the linear sugar alcohol stabilized crosslinked (calcium 2-fluoroacrylate)-divinylbenzene-1,7-octadiene terpolymer is **characterized by** one or more of the parameters as defined in claims 1 to 4.

**7.** The linear sugar alcohol stabilized crosslinked (calcium 2-fluoroacrylate)-divinylbenzene-1,7-octadiene terpolymer according to claim 6, wherein the slurry contains an excess amount of the linear sugar alcohol based on polymer weight,
and/or
wherein the slurrying is maintained for at least 3 hours at ambient temperature and pressure.

**8.** The linear sugar alcohol stabilized crosslinked (calcium 2-fluoroacrylate)-divinylbenzene-1,7-octadiene terpolymer according to any one of claims 1 to 7, wherein the amount of calcium in the polymer is in the range of from about 10 wt.% to about 20 wt.%, based on the total weight of the polymer.

**9.** The linear sugar alcohol stabilized crosslinked (calcium 2-fluoroacrylate)-divinylbenzene-1,7-octadiene terpolymer according to any one of claims 1 to 8, having a swelling ratio in physiological isotonic buffer from about 1 to about 7 grams of water per gram of polymer, preferably from about 1 to 5 grams of water per gram of polymer, more preferably from about 1 to 3 grams of water per gram of polymer.

**10.** The linear sugar alcohol stabilized crosslinked (calcium 2-fluoroacrylate)-divinylbenzene-1,7-octadiene terpolymer according to any one of claims 1 to 9, represented by the general structure of the Formula 40A:

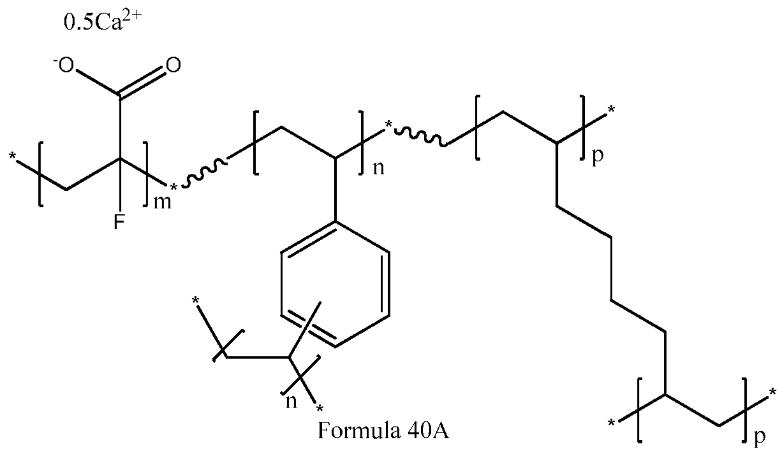

Formula 40A

wherein m is in the range of from about 85 to about 93 mol%, n is in the range of from about 1 to about 10 mol% and p is in the range of from about 1 to about 10 mol%, calculated based on the ratios of monomers added to the polymerization mixture, stabilized by 10wt% to 40 wt% of a linear sugar alcohol.

**11.** The linear sugar alcohol stabilized crosslinked (calcium 2-fluoroacrylate)-divinylbenzene-1,7-octadiene terpolymer according to any one of claims 1 to 10, wherein the linear sugar alcohol is selected from the group consisting of arabitol, erythritol, glycerol, maltitol, mannitol, ribitol, sorbitol, xylitol, threitol, galactitol, isomalt, iditol, lactitol and

combinations thereof; preferably sorbitol, xylitol, or a combination thereof; more preferably sorbitol.

**12.** A pharmaceutical composition, comprising the linear sugar alcohol stabilized crosslinked (calcium 2-fluoroacrylate)-divinylbenzene-1,7-octadiene terpolymer according to any one of claims 1 to 11 and one or more pharmaceutically acceptable excipients.

**13.** The linear sugar alcohol stabilized crosslinked (calcium 2-fluoroacrylate)-divinylbenzene-1,7-octadiene terpolymer according to any of claims 1 to 11 or the pharmaceutical composition according to claim 12 for use

in a method of removing potassium from the gastrointestinal tract of a subject in need thereof, whereby the polymer passes through the gastrointestinal tract of the subject, and removes a therapeutically effective amount of potassium ion from the gastrointestinal tract of the subject, and/or
in the treatment of hyperkalemia, chronic kidney disease, and/or congestive heart failure.

**14.** The linear sugar alcohol stabilized crosslinked (calcium 2-fluoroacrylate)-divinylbenzene-1,7-octadiene terpolymer according to any one of claims 1 to 11 or the pharmaceutical composition according to claim 12, for administration to a subject experiencing hyperkalemia, suffering from chronic kidney disease, suffering from congestive heart failure, and/or undergoing dialysis.

**15.** The linear sugar alcohol stabilized crosslinked (calcium 2-fluoroacrylate)-divinylbenzene-1,7-octadiene terpolymer or the pharmaceutical composition for the use according to any one of claims 13 or 14, for administration to a human subject being treated with an agent that causes potassium retention, the agent that causes potassium retention being an angiotensin-converting enzyme inhibitor (preferably captopril, zofenopril, enalapril, ramipril, quinapril, perindopril, lisinopril, benazepril, fosinopril, or a combination thereof), an angiotensin receptor blocker (preferably candesartan, eprosartan, irbesartan, losartan, olmesartan, telmisartan, valsartan, or a combination thereof), or an aldosterone antagonist (preferably spironolactone, eplerenone, or a combination thereof).

**Patentansprüche**

**1.** Mit linearem Zuckeralkohol stabilisiertes vernetztes (Calcium-2-Fluoracrylat)-Divinylbenzol-1,7-octadien-Terpolymer mit einem Calciumgehalt im Polymer im Bereich von etwa 8 Gew.-% bis etwa 25 Gew.-%, bezogen auf das Gesamtgewicht des Polymers.

**2.** Mit linearem Zuckeralkohol stabilisiertes vernetztes (Calcium-2-Fluoracrylat)-Divinylbenzol-1,7-octadien-Terpolymer welches in Form von im Wesentlichen sphärischen Partikeln mit einem mittleren Durchmesser von etwa 20 $\mu$m bis etwa 200 $\mu$m vorliegt.

**3.** Mit linearem Zuckeralkohol stabilisiertes vernetztes (Calcium-2-Fluoracrylat)-Divinylbenzol-1,7-octadien-Terpolymer welches in Form von im Wesentlichen sphärischen Partikeln vorliegt mit einer Partikelgrößenverteilung worin nicht mehr als etwa 5 Volumen-% der Partikel einen Durchmesser von weniger als etwa 30 $\mu$m (d.h. D(0.05) < 30 $\mu$m), nicht mehr als etwa 5 Volumen-% der Partikel einen Durchmesser von größer als etwa 250 $\mu$m (d.h., D(0.05) > 250 $\mu$m), und wenigstens etwa 50 Volumen-% der Partikel einen Durchmesser im Bereich von etwa 70 bis etwa 150 $\mu$m aufweisen.

**4.** Mit linearem Zuckeralkohol stabilisiertes vernetztes (Calcium-2-Fluoracrylat)-Divinylbenzol-1,7-octadien-Terpolymer welches eine Quellungsrate in physiologischem isotonischem Puffer von weniger als etwa 10 g Wasser pro Gramm Polymer aufweist.

**5.** Mit linearem Zuckeralkohol stabilisiertes vernetztes (Calcium-2-Fluoracrylat)-Divinylbenzol-1,7-octadien-Terpolymer welches durch zwei oder mehr der in den Ansprüchen 1 bis 4 definierten Parameter charakterisiert ist.

**6.** Mit linearem Zuckeralkohol stabilisiertes vernetztes (Calcium-2-Fluoracrylat)-Divinylbenzol-1,7-octadien-Terpolymer erhältlich durch Aufschlämmen eines vernetzten (Calcium-2-Fluoracrylat)-Divinylbenzol-1,7-octadien-Terpolymers mit einer wässrigen Lösung eines linearen Zuckeralkohols, worin das mit linearem Zuckeralkohol stabilisierte vernetzte (Calcium-2-Fluoracrylat)-Divinylbenzol-1,7-octadien-Terpolymer durch zwei oder mehr der in den Ansprüchen 1 bis 4 definierten Parameter charakterisiert ist.

**7.** Das mit linearem Zuckeralkohol stabilisierte vernetzte (Calcium-2-Fluoracrylat)-Divinylbenzol-1,7-octadien-Terpolymer gemäß Anspruch 6 worin die Aufschlämmung einen Überschuss an linearem Zuckeralkohol, bezogen auf das Polymergewicht, enthält
und/oder
worin die Aufschlämmung für mindestens 3 Stunden bei Umgebungstemperatur und - druck gehalten wird.

**8.** Das mit linearem Zuckeralkohol stabilisierte vernetzte (Calcium-2-Fluoracrylat)-Divinylbenzol-1,7-octadien-Terpolymer gemäß einem der Ansprüche 1 bis 7, worin der Calciumgehalt in dem Polymer im Bereich von etwa 10 Gew.-% bis etwa 20 Gew.-%, bezogen auf das Gesamtgewicht des Polymers, liegt.

**9.** Das mit linearem Zuckeralkohol stabilisierte vernetzte (Calcium-2-Fluoracrylat)-Divinylbenzol-1,7-octadien-Terpolymer gemäß einem der Ansprüche 1 bis 8, welches eine Quellungsrate in physiologischem isotonischem Puffer von etwa 1 bis etwa 7 g Wasser pro Gramm Polymer, bevorzugt von etwa 1 bis 5 g Wasser pro Gramm Polymer, bevorzugter von etwa 1 bis 3 g Wasser pro Gramm Polymer, aufweist.

**10.** Das mit linearem Zuckeralkohol stabilisierte vernetzte (Calcium-2-Fluoracrylat)-Divinylbenzol-1,7-octadien-Terpolymer gemäß einem der Ansprüche 1 bis 9, dargestellt durch die allgemeine Struktur der Formel 40A:

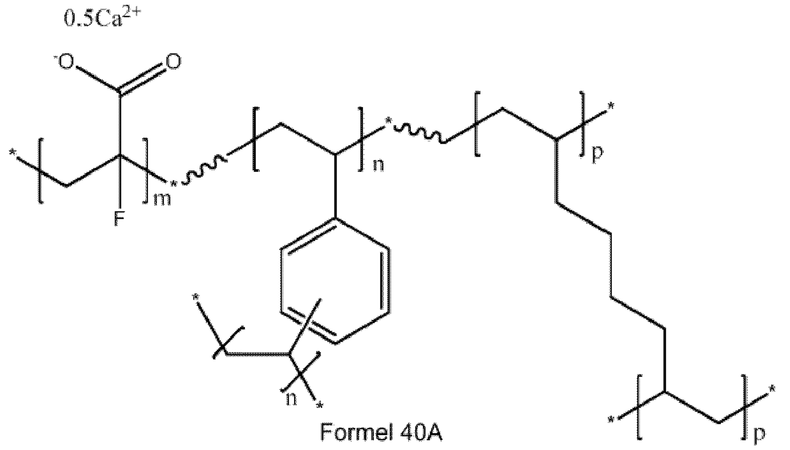

Formel 40A

worin m im Bereich von etwa 85 bis etwa 93 mol%, n im Bereich von etwa 1 bis etwa 10 mol% und p im Bereich von etwa 1 bis etwa 10 mol% liegt, berechnet auf Basis der Verhältnisse der zu der Polymerisationsmischung zugefügten Monomere, stabilisiert mit 10 Gew.-% bis 40 Gew.-% eines linearen Zuckeralkohols.

**11.** Das mit linearem Zuckeralkohol stabilisierte vernetzte (Calcium-2-Fluoracrylat)-Divinylbenzol-1,7-octadien-Terpolymer gemäß einem der Ansprüche 1 bis 10, worin der lineare Zuckeralkohol ausgewählt ist aus der Gruppe bestehend aus Arabitol, Erythritol, Glycerol, Maltitol, Mannitol, Ribitol, Sorbitol, Xylitol, Threitol, Galactitol, Isomalt, Iditol, Lactitol und Kombinationen davon; bevorzugt aus Sorbitol, Xylitol oder Kombinationen davon; bevorzugter ist Sorbitol.

**12.** Eine pharmazeutische Zusammensetzung, umfassend das mit linearem Zuckeralkohol stabilisierte vernetzte (Calcium-2-Fluoracrylat)-Divinylbenzol-1,7-octadien-Terpolymer gemäß einem der Ansprüche 1 bis 11 und einen oder mehrere pharmazeutisch akzeptable Hilfsstoffe.

**13.** Das mit linearem Zuckeralkohol stabilisierte vernetzte (Calcium-2-Fluoracrylat)-Divinylbenzol-1,7-octadien-Terpolymer gemäß einem der Ansprüche 1 bis 11 oder die pharmazeutische Zusammensetzung gemäß Anspruch 12 zur Verwendung

in einem Verfahren zur Entfernung von Kalium aus dem Gastrointestinaltrakt eines Subjekts, das dessen bedarf, wobei das Polymer den Gastrointestinaltrakt des Subjekts durchläuft und eine therapeutisch wirksame Menge von Kaliumionen aus dem Gastrointestinaltrakt des Subjekts entfernt, und/oder
in der Behandlung von Hyperkaliämie, chronischer Nierenerkrankung, und/oder kongestiver Herzinsuffizienz.

**14.** Das mit linearem Zuckeralkohol stabilisierte vernetzte (Calcium-2-Fluoracrylat)-Divinylbenzol-1,7-octadien-Terpolymer gemäß einem der Ansprüche 1 bis 11 oder die pharmazeutische Zusammensetzung gemäß Anspruch 12, zur

Verabreichung an ein Subjekt welches Hyperkaliämie durchmacht, unter chronischer Nierenerkrankung leidet, unter kongestiver Herzinsuffizienz leidet und/oder sich einer Dialyse unterzieht.

**15.** Das mit linearem Zuckeralkohol stabilisierte vernetzte (Calcium-2-Fluoracrylat)-Divinylbenzol-1,7-octadien-Terpolymer oder die pharmazeutische Zusammensetzung zur Verwendung gemäß einem der Ansprüche 13 oder 14, zur Verabreichung an ein humanes Subjekt welches mit einem Mittel behandelt wird, das Kaliumretention verursacht, wobei das Kaliumretention verursachende Mittel ein Angiotensin-Konvertierender-Enzym-Hemmer ist (bevorzugt Captopril, Zofenopril, Enalapril, Ramipril, Quinapril, Perindopril, Lisinopril, Benazipril, Fosinopril oder eine Kombination davon), ein Angiotensin-Rezeptorblocker ist (bevorzugt Candesartan, Eprosartan, Irbesartan, Losartan, Olmesartan, Telmisartan, Valsartan oder eine Kombination davon), oder ein Aldosteron-Antagonist ist (bevorzugt Spironolacton, Eplerenon oder eine Kombination davon).

## Revendications

**1.** Terpolymère réticulé de (2-fluoroacrylate de calcium)-divinylbenzène-1,7-octadiène stabilisé par un alcool de sucre linéaire, avec une quantité de calcium dans le polymère comprise entre environ 8 % en poids et environ 25 % en poids, par rapport au poids total du polymère.

**2.** Terpolymère réticulé de (2-fluoroacrylate de calcium)-divinylbenzène-1,7-octadiène stabilisé par un alcool de sucre linéaire, qui se présente sous la forme de particules sensiblement sphériques d'un diamètre moyen d'environ 20 $\mu$m à environ 200 $\mu$m.

**3.** Terpolymère réticulé de (2-fluoroacrylate de calcium)-divinylbenzène-1,7-octadiène stabilisé par un alcool de sucre linéaire, qui se présente sous la forme de particules sensiblement sphériques ayant une distribution de taille de particules dans laquelle pas plus de 5 % en volume environ des particules ont un diamètre inférieur à 30 $\mu$m environ (c'est-à-dire, D(0,05) < 30 $\mu$m), pas plus de 5 % en volume des particules ont un diamètre supérieur à environ 250 $\mu$m (c'est-à-dire D(0,05) > 250 $\mu$m), et au moins 50 % en volume des particules ont un diamètre compris entre environ 70 et environ 150 $\mu$m.

**4.** Terpolymère réticulé de (2-fluoroacrylate de calcium)-divinylbenzène-1,7-octadiène stabilisé par un alcool de sucre linéaire, ayant un ratio de gonflement dans un tampon physiologique isotonique inférieur à environ 10 grammes d'eau par gramme de polymère.

**5.** Terpolymère réticulé de (2-fluoroacrylate de calcium)-divinylbenzène-1,7-octadiène stabilisé par un alcool de sucre linéaire, **caractérisé par** au moins deux des paramètres définis dans les revendications 1 à 4.

**6.** Terpolymère réticulé de (2-fluoroacrylate de calcium)-divinylbenzène-1,7-octadiène stabilisé par un alcool de sucre linéaire, pouvant être obtenu en mettant en suspension un terpolymère réticulé de (2-fluoroacrylate de calcium)-divinylbenzène-1,7-octadiène avec une solution aqueuse d'un alcool de sucre linéaire, dans lequel le terpolymère réticulé de (2-fluoroacrylate de calcium)-divinylbenzène-1,7-octadiène stabilisé par un alcool de sucre linéaire est **caractérisé par** un ou plusieurs des paramètres définis dans les revendications 1 à 4.

**7.** Terpolymère réticulé de (2-fluoroacrylate de calcium)-divinylbenzène-1,7-octadiène stabilisé par un alcool de sucre linéaire selon la revendication 6, dans lequel la suspension contient une quantité excessive d'alcool de sucre linéaire par rapport au poids du polymère,
et/ou
dans lequel la suspension est maintenue pendant au moins 3 heures à température et pression ambiantes.

**8.** Terpolymère réticulé de (2-fluoroacrylate de calcium)-divinylbenzène-1,7-octadiène stabilisé par un alcool de sucre linéaire, selon l'une quelconque des revendications 1 à 7, dans lequel la quantité de calcium dans le polymère est comprise entre environ 10 % en poids et environ 20 % en poids, par rapport au poids total du polymère.

**9.** Terpolymère réticulé de (2-fluoroacrylate de calcium)-divinylbenzène-1,7-octadiène stabilisé par un alcool de sucre linéaire, selon l'une quelconque des revendications 1 à 8, ayant un ratio de gonflement dans un tampon physiologique isotonique d'environ 1 à environ 7 grammes d'eau par gramme de polymère, de préférence d'environ 1 à 5 grammes d'eau par gramme de polymère, plus préférentiellement d'environ 1 à 3 grammes d'eau par gramme de polymère.

**10.** Terpolymère réticulé de (2-fluoroacrylate de calcium)-divinylbenzène-1,7-octadiène stabilisé par un alcool de sucre linéaire, selon l'une quelconque des revendications 1 à 9, représenté par la structure générale de la formule 40A :

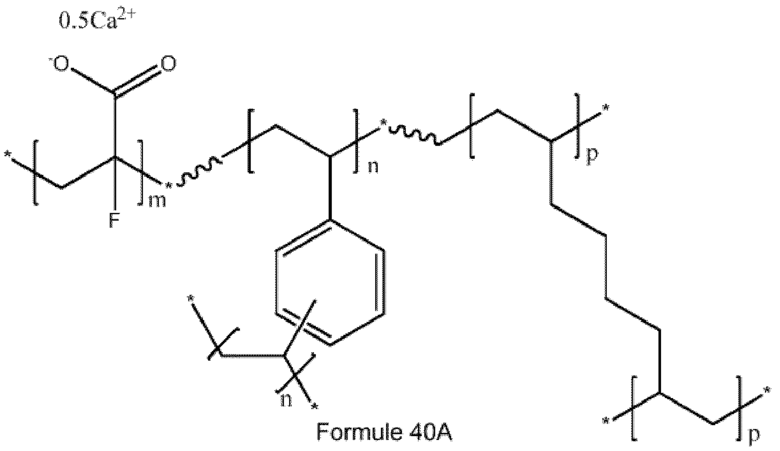

Formule 40A

où m est compris entre environ 85 et environ 93 % en moles, n est compris entre environ 1 et environ 10 % en moles et p est compris entre environ 1 et environ 10 % en moles, calculés sur la base des rapports des monomères ajoutés au mélange de polymérisation, stabilisé par 10 à 40 % en poids d'un alcool de sucre linéaire.

**11.** Terpolymère réticulé de (2-fluoroacrylate de calcium)-divinylbenzène-1,7-octadiène stabilisé par un alcool de sucre linéaire selon l'une quelconque des revendications 1 à 10, dans lequel l'alcool de sucre linéaire est choisi dans le groupe constitué par l'arabitol, l'érythritol, le glycérol, le maltitol, le mannitol, le ribitol, le sorbitol, le xylitol, le thréitol, le galactitol, l'isomalt, l'iditol, le lactitol et combinaisons de ces derniers; de préférence le sorbitol, le xylitol ou une combinaison de ceux-ci; et de manière encore plus préférée le sorbitol.

**12.** Composition pharmaceutique, comprenant le terpolymère réticulé de (2-fluoroacrylate de calcium)-divinylbenzène-1,7-octadiène stabilisé par un alcool de sucre linéaire selon l'une des revendications 1 à 11 et un ou plusieurs excipients pharmaceutiquement acceptables.

**13.** Terpolymère réticulé de (2-fluoroacrylate de calcium)-divinylbenzène-1,7-octadiène stabilisé par un alcool de sucre linéaire, selon l'une quelconque des revendications 1 à 11 ou composition pharmaceutique selon la revendication 12, pour utilisation

dans une méthode d'élimination du potassium du tractus gastro-intestinal d'un sujet qui en a besoin, le polymère traversant le tractus gastro-intestinal du sujet et éliminant une quantité thérapeutiquement efficace d'ions potassium du tractus gastro-intestinal du sujet, et/ou
dans le traitement de l'hyperkaliémie, de l'insuffisance rénale chronique et/ou de l'insuffisance cardiaque congestive.

**14.** Terpolymère réticulé de (2-fluoroacrylate de calcium)-divinylbenzène-1,7-octadiène, stabilisé par un alcool de sucre linéaire, selon l'une quelconque des revendications 1 à 11 ou composition pharmaceutique selon la revendication 12, pour administration à un sujet présentant une hyperkaliémie, souffrant d'une maladie rénale chronique, souffrant d'une insuffisance cardiaque congestive, et/ou sous dialyse.

**15.** Le terpolymère réticulé de (2-fluoroacrylate de calcium)-divinylbenzène-1,7-octadiène stabilisé par un alcool de sucre linéaire ou la composition pharmaceutique pour l'utilisation selon l'une quelconque des revendications 13 ou 14, pour l'administration à un sujet humain traité avec un agent qui provoque une rétention de potassium, l'agent qui provoque une rétention de potassium étant un inhibiteur de l'enzyme de conversion de l'angiotensine (de préférence le captopril, zofénopril, énalapril, ramipril, quinapril, périndopril, lisinopril, bénazépril, fosinopril, ou une combinaison de ceux-ci), un inhibiteur des récepteurs de l'angiotensine (de préférence candésartan, éprosartan, irbesartan, losartan, olmesartan, telmisartan, valsartan, ou une combinaison de ceux-ci), ou un antagoniste de l'aldostérone (de préférence spironolactone, éplérénone, ou une combinaison de ceux-ci).

FIG. 1A

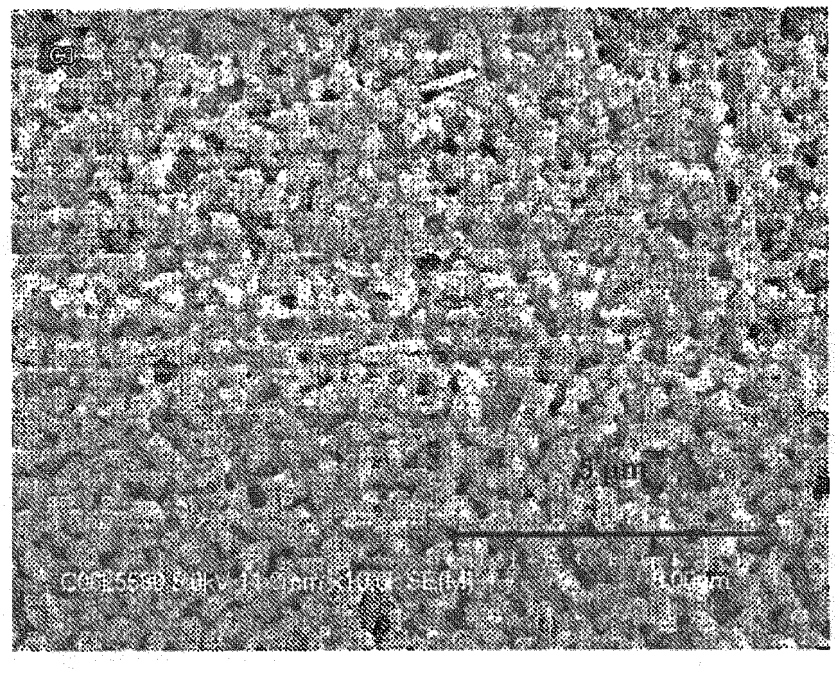


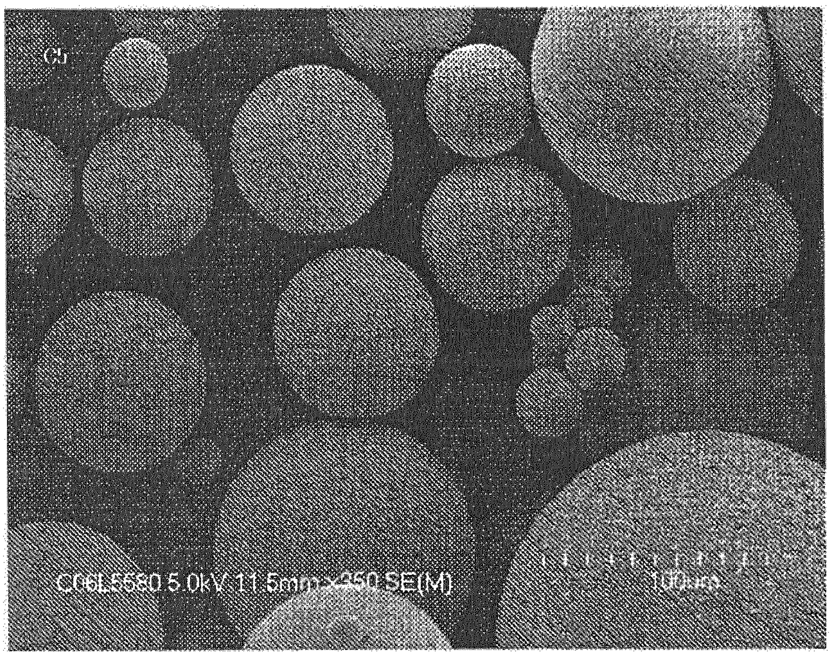

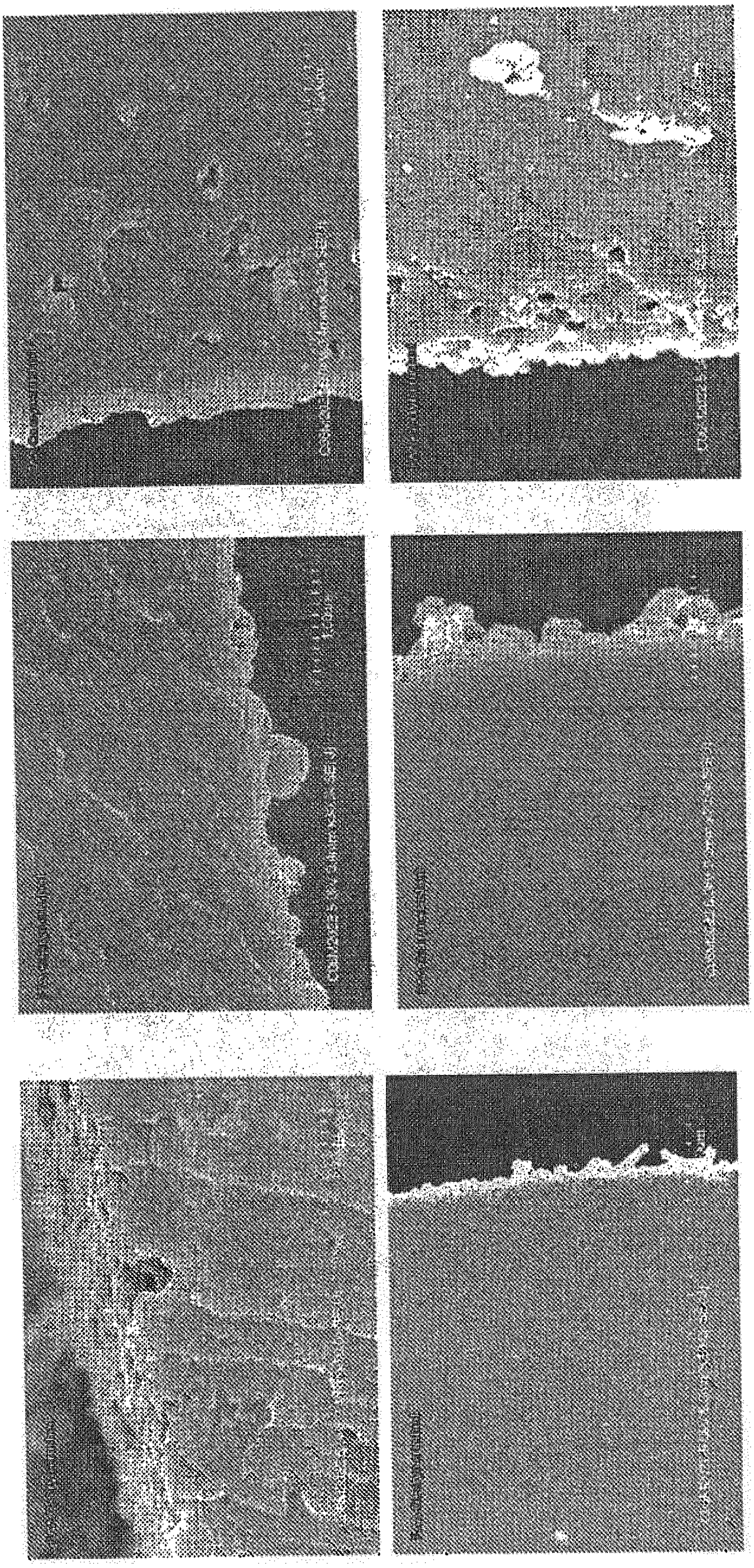

FIG. 1B

FIG. 2A

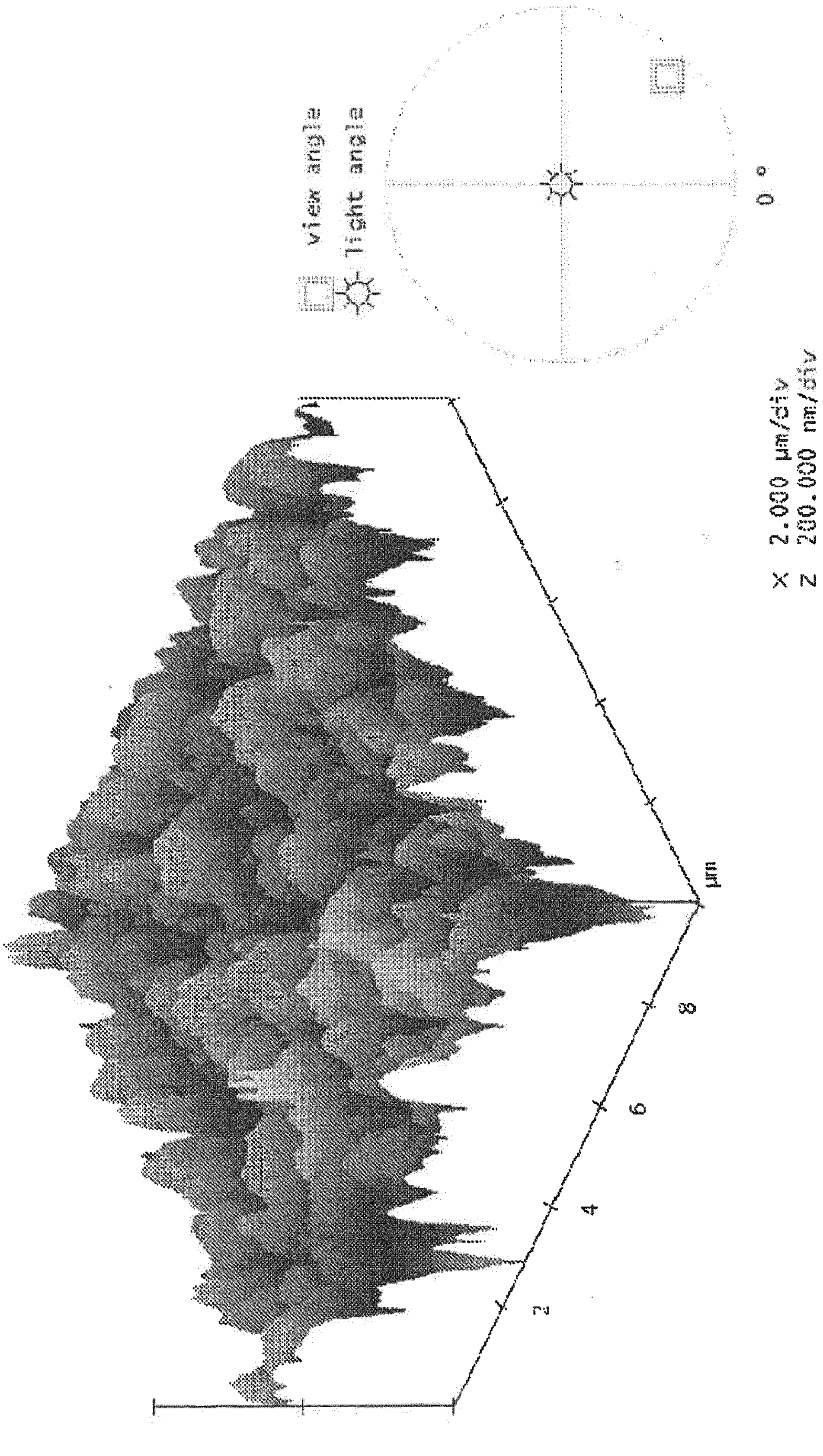

FIG. 2B

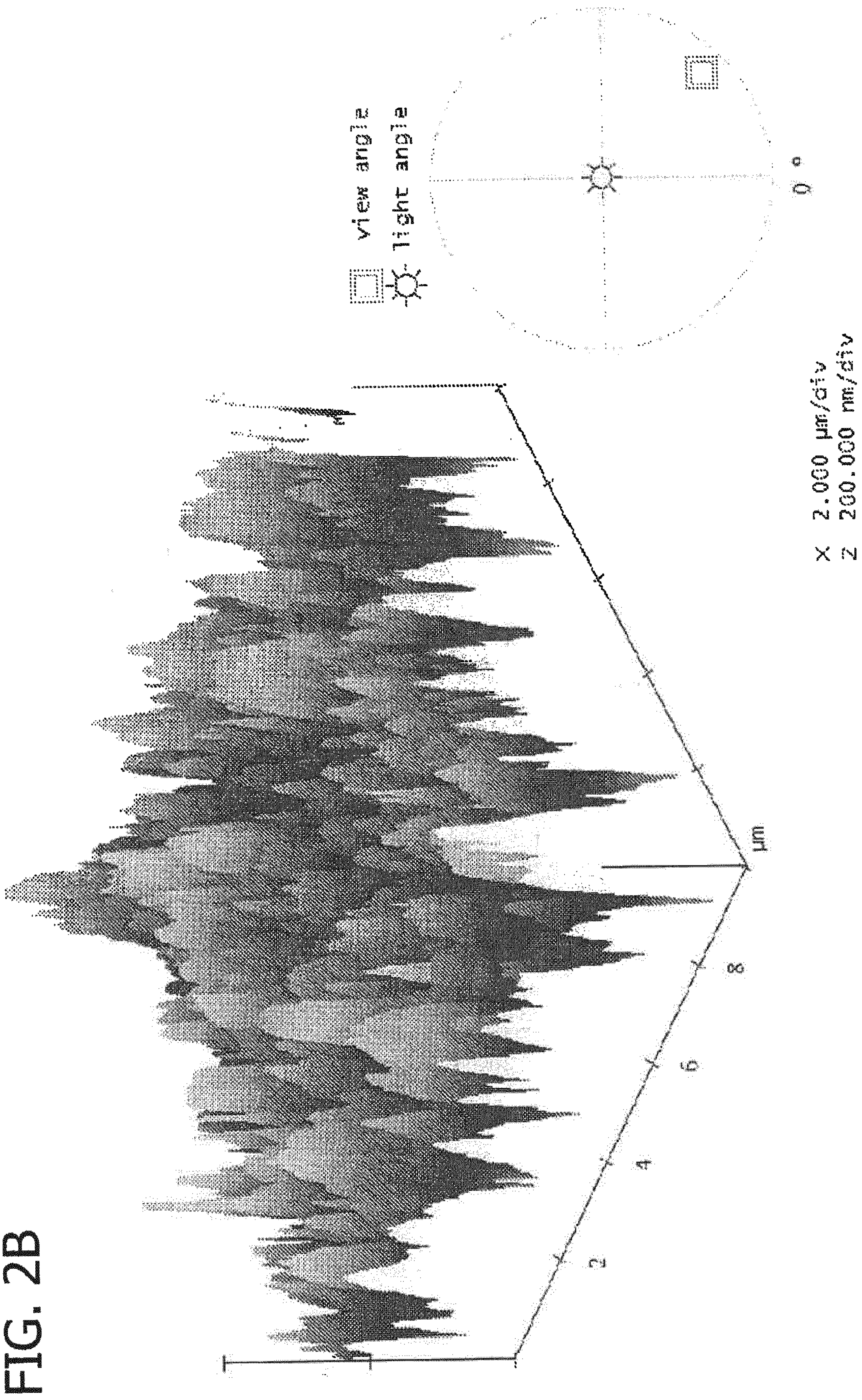

FIG. 3

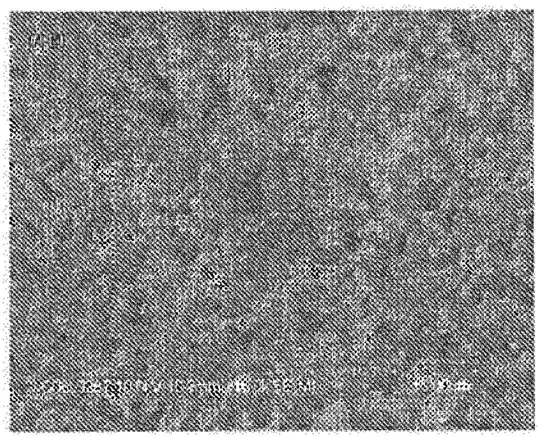

A1

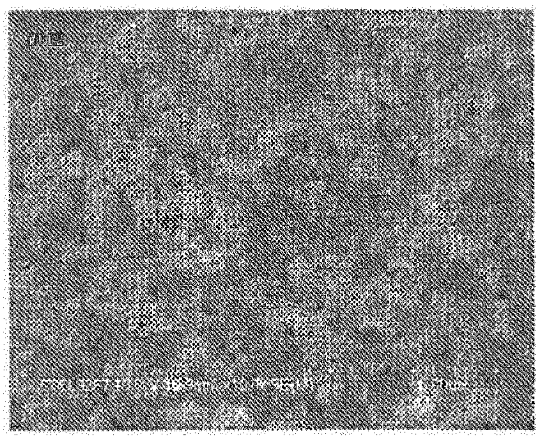

A2

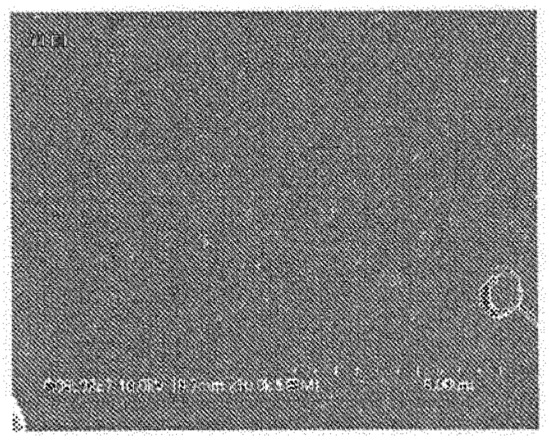

A3

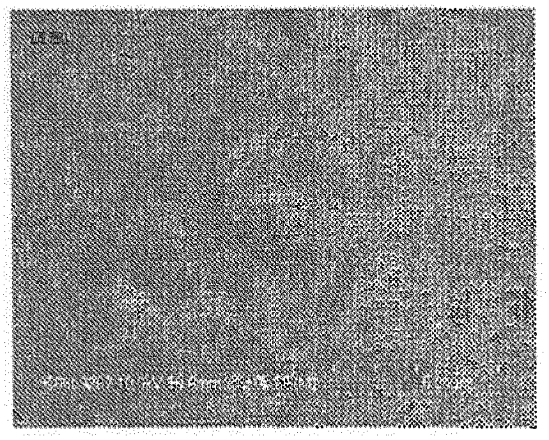

A4

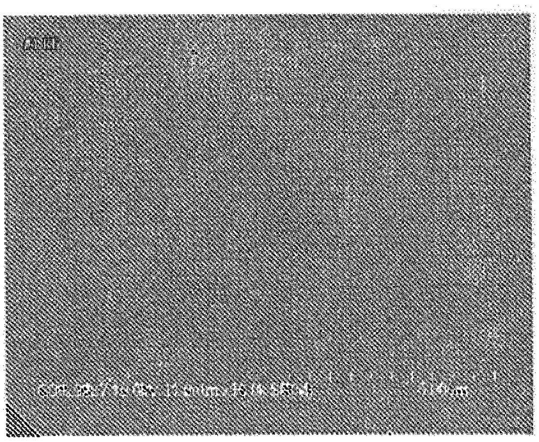

A5

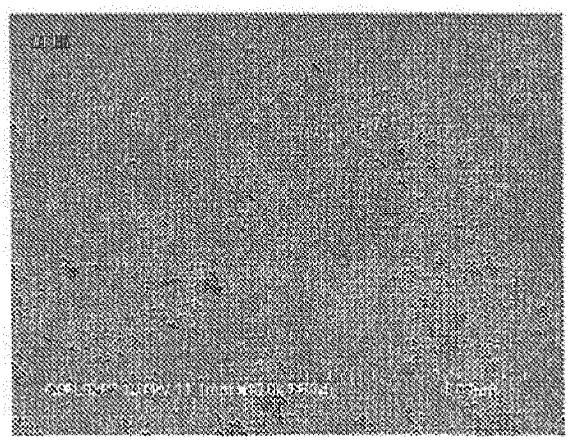

A6

FIG. 4

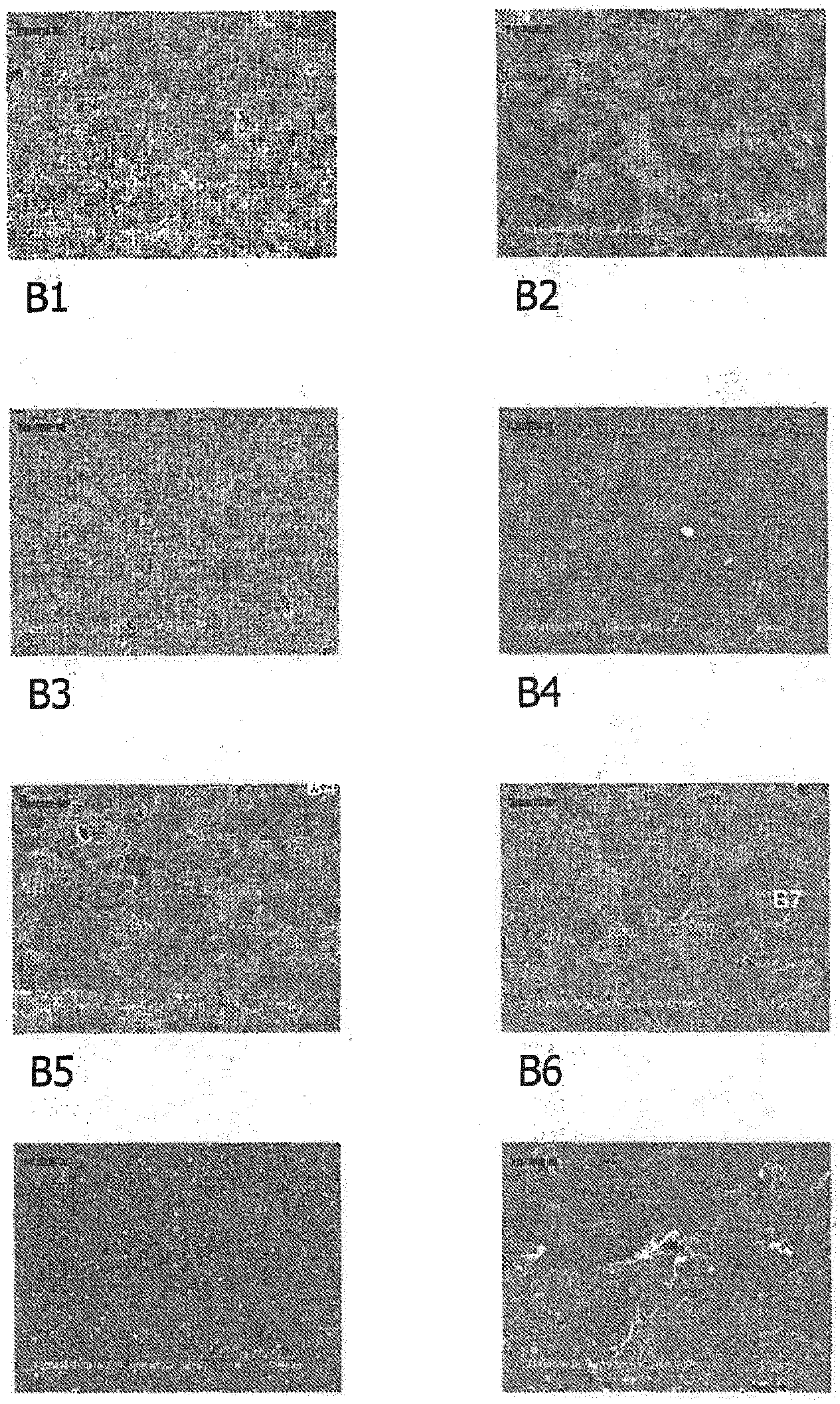

B1 B2

B3 B4

B5 B6

B7 B8

FIG. 5A

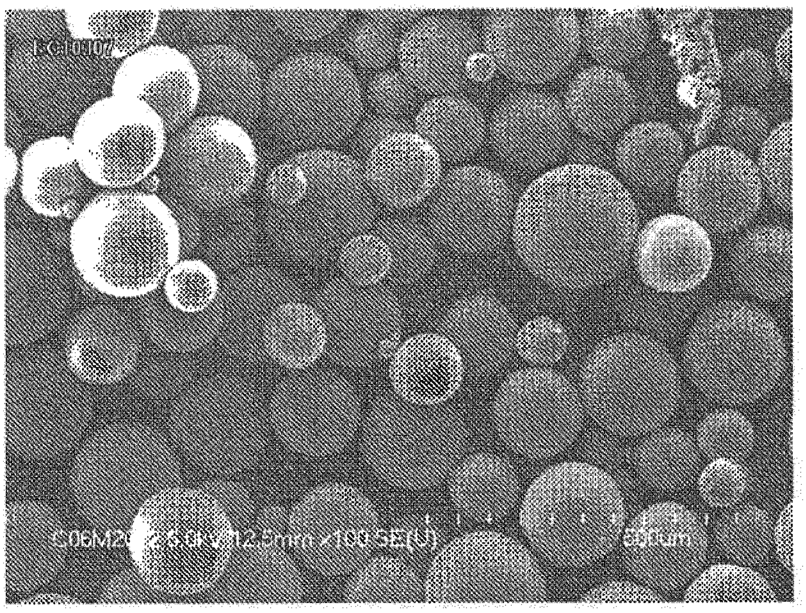

FIG. 5B

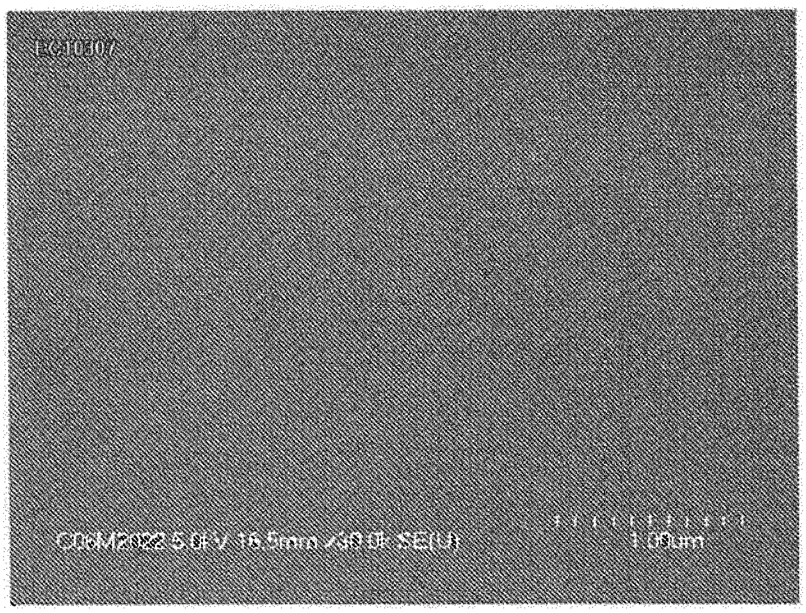

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005097081 A
- EP 1785141 A
- US 2005220752 A
- US 20060024336 A
- WO 2007038802 A
- US 5112993 A
- EP 415214 A
- US 20050220752
- US 6994827 B


### Non-patent literature cited in the description


- **GASSEN et al.** *J. Fluorine Chemistry*, 1991, vol. 55, 149-162
- **KF PITTMAN** ; **C. U., M. UEDA et al.** *Macromolecules*, 1980, vol. 13 (5), 1031-1036
- **BOGACHEV et al.** *Zhumal Organisheskoi Khimii*, 1986, vol. 22 (12), 2578-83
- McCutcheon's Emulsifiers and Detergents. 2007